(19) Europäisches Patentamt / European Patent Office / Office européen des brevets

(11) EP 3 247 769 B1

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(21) Anmeldenummer: **16702496.7**

(22) Anmeldetag: **20.01.2016**

(51) Int Cl.:
***C09K 11/06*** *(2006.01)* ***H01L 51/50*** *(2006.01)*
***C07C 255/58*** *(2006.01)* ***C07F 9/6561*** *(2006.01)*
***C07F 9/6568*** *(2006.01)* ***C07D 335/16*** *(2006.01)*
***C07D 409/04*** *(2006.01)* ***H01L 51/00*** *(2006.01)*
***C07F 9/6558*** *(2006.01)* ***C07F 9/53*** *(2006.01)*

(86) Internationale Anmeldenummer:
**PCT/EP2016/051161**

(87) Internationale Veröffentlichungsnummer:
**WO 2016/116520 (28.07.2016 Gazette 2016/30)**

(54) **PHENYLETHER-SUBSTITUIERTE ORGANISCHE MOLEKÜLE, INSBESONDERE ZUR VERWENDUNG IN OPTOELEKTRONISCHEN BAUELEMENTEN**

PHENYLETHER-SUBSTITUTED ORGANIC MOLECULES, IN PARTICULAR FOR USE IN OPTOELECTRONIC COMPONENTS

MOLÉCULES ORGANIQUES SUBSTITUÉES PAR UN ÉTHER PHÉNYLIQUE, EN PARTICULIER POUR UNE UTILISATION DANS DES COMPOSANTS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2015 EP 15151870**
**20.05.2015 DE 102015107893**

(43) Veröffentlichungstag der Anmeldung:
**29.11.2017 Patentblatt 2017/48**

(73) Patentinhaber: **cynora GmbH**
**76646 Bruchsal (DE)**

(72) Erfinder:
- **AMBROSEK, David**
**10437 Berlin (DE)**
- **DANZ, Michael**
**76344 Eggenstein-Leopoldshafen (DE)**
- **FLÜGGE, Harald**
**76135 Karlsruhe (DE)**
- **FRIEDRICHS, Jana**
**76137 Karlsruhe (DE)**
- **GRAB, Tobias**
**76133 Karlsruhe (DE)**
- **JACOB, Andreas**
**30449 Hannover (DE)**
- **SEIFERMANN, Stefan**
**77815 Bühl (DE)**
- **VOLZ, Daniel**
**76137 Karlsruhe (DE)**
- **MYDLAK, Mathias**
**76133 Karlsruhe (DE)**
- **GEORGIOS, Liaptsis**
**68161 Mannheim (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2013/161437 US-A1- 2012 075 171**
**US-A1- 2014 005 399**

- **MEGANATHAN NANDAKUMAR ET AL: "Diels-Alder Reaction of 1,3-Diarylbenzo[ c ]furans with Thiophene S,S -Dioxide/Indenone Derivatives: A Facile Preparation of Substituted Dibenzothiophene S,S -Dioxides and Fluorenones", ORGANIC LETTERS, Bd. 16, Nr. 11, 6. Juni 2014 (2014-06-06), Seiten 3068-3071, XP055266631, US ISSN: 1523-7060, DOI: 10.1021/ol501175q**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- BIGDELI M A ET AL: "Ab initio study of atropisomers of derivatives of 1,8-di-pyridine 9H-fluorene, dibenzo[b,d]furan, 9H-carbazole and dibenzo[b,d]thiophene", JOURNAL OF MOLECULAR STRUCTURE (THEOCHEM), ELSEVIER SCIENCE PUBLISHERS B.V., AMSTERDAM, NL, Bd. 860, Nr. 1-3, 15. Juli 2008 (2008-07-15), Seiten 64-79, XP022704999, ISSN: 0166-1280, DOI: 10.1016/J.THEOCHEM.2008.03.025[gefunden am 2008-04-01]

## Beschreibung

Die Erfindung betrifft rein organische Moleküle und deren Verwendung in organischen lichtemittierende Dioden (OLEDs) und in anderen optoelektronischen Bauelementen.

## Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermally activated delayed fluorescence oder auch singlet harvesting). Hierbei können beispielsweise Cu(I)-Komplexe verwendet werden, die aufgrund eines geringen Energieabstandes zwischen dem untersten Triplett-Zustand $T_1$ und dem darüberliegenden Singulett-Zustand $S_1$ ($\Delta E(S_1$-$T_1$) Triplett-Exitonen thermisch in einen Singulett-Zustand rückbesetzen können. Neben der Verwendung von Übergangsmetallkomplexen können auch rein organische Moleküle (ohne Metallion) diesen Effekt ausnutzen.

Ähnliche Moleküle sind aus WO 2013/161437 und US 2012/075171 A1 bekannt.

## Beschreibung

Die Erfindung betrifft in einem Aspekt rein organische Moleküle, die in optoelektronischen Bauelementen verwendet werden können.

Derartige organische Moleküle weisen eine Struktur der Formel 1 auf oder haben eine Struktur der Formel 1:

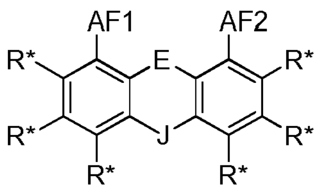

**Formel 1**

In Formel 1 bedeutet:

E = S, S(=O), O, C(=O), $CR^*_2$ $Si(R^4)2$, $Ge(R^4)_2$, $NR^2$, $PR^3$, $P(=O)R^7$, $P(=S)R^7$, $AsR^3$, $As(=O)R^7$, $As(=S)R^7$, $SbR^3$, $BR^3$;

J = keine Bindung, sodass an den zwei Phenylringen an dieser Position dann je ein Rest R* vorhanden ist, oder J ist eine Einfachbindung, so dass die beiden Phenylringe durch eine Einfachbindung miteinander verbunden sind.

R* ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder-S- ersetzt sein können und wobei ein oder mehrere H-Atome durch Deuterium, F, Cl, Br, I, CN, $Af1CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein mono- oder polycyclisches, aliphatisches, aromatisches und/oder benzoannelliertes Ringsystem bilden. In einer Ausführungsform können zwei oder mehrere dieser Substituenten R* miteinander auch ein Ringsystem mit insgesamt fünf oder sechs Ringgliedern bilden. In einer Ausführungsform ist das Ringsystem, dass gebildet werden kann auf ein monozyklisches aliphatisches Ringsystem mit insgesamt fünf oder sechs Ringgliedern beschränkt.

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden.

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden.

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden.

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert

sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden.

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden.

$R^7$ ist bei jedem Auftreten unabhängig voneinander ausgewählt aus der Gruppe bestehend aus Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden.

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $AS(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C=C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder-S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen

oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind insbesondere N, O, und S. Werden in der Beschreibung der vorliegenden Erfindung andere, davon abweichende Definitionen angegeben, beispielsweise bezüglich der Zahl der aromatischen Ringatome oder der enthaltenen Heteroatome, so gelten diese abweichenden Definitionen.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind insbesondere ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (insbesondere weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch System wie 9,9'-Diarylfluoren, Triarylamin, Diarylether, Stilben etc als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppen oder durch eine Silylgruppe verbunden sind. Weiterhin werden auch Systeme, in denen zwei oder mehr Aryl- oder Heteroyraylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen.

Es sind im organischen Molekül der Formel 1 zwei unterschiedliche chemische Einheiten AF1 und AF2 enthalten;

AF1: eine erste chemische Einheit, aufweisend ein konjugiertes System, insbesondere mindestens sechs in Konjugation stehende $\pi$-Elektronen (z. B. in Form mindestens eines aromatischen Systems);

AF2: eine zweite chemische Einheit, aufweisend ein konjugiertes System, insbesondere mindestens sechs in Konjugation stehende π-Elektronen (z. B. in Form mindestens eines aromatischen Systems);
Definitionsgemäß weist AF2 im Vergleich zu AF1 immer einen niedrigeren (mathematischen) HOMO-Zahlenwert auf (und damit auch entsprechend einen niedrigeren LUMO-Zahlenwert als AF1).
Dabei gelten für die organischen Moleküle insbesondere die folgenden Merkmale:

- Die Differenz der Energie des HOMO der zweiten chemischen Einheit AF2 und der Energie des HOMO der ersten chemischen Einheit AF1 > 0,8 eV ist (Δ HOMO = HOMO(AF2) - HOMO(AF1) > 0,8 eV);
- die Differenz der Energie des LUMO der zweiten chemischen Einheit AF2 und der Energie des LUMO der ersten chemischen Einheit AF1 > 0,8 eV ist (Δ LUMO = LUMO(AF2) - LUMO(AF1) > 0,8 eV); und
- die Differenz der Energie des LUMO der ersten chemischen Einheit AF1 und der Energie des HOMO der zweiten chemischen Einheit AF2 > 0,9 eV ist (Δ Gap = LUMO (AF1) - HOMO(AF2)> 0,9 eV).

In einer Ausführungsform sind die chemischen Einheiten AF1 und AF2 derart miteinander verknüpft, dass die elektronische Kommunikation zwischen ihnen unterbrochen wird. Diese Unterbrechung ist durch eine Lokalisierung der Grenzorbitale HOMO und LUMO auf separaten Molekülteilen gekennzeichnet, sodass ein Charge-Transfer Übergang ermöglicht wird.

In einer Ausführungsform sind die chemischen Einheiten AF1 und AF2 derart über einen Separator miteinander verbunden, dass die elektronische Kommunikation zwischen ihnen unterbrochen wird, was anhand der Lokalisierung der Grenzorbitale HOMO und LUMO auf separaten Molekülteilen gekennzeichnet ist. Der Separator kann dabei eine beliebige Struktur aufweisen, solange eine Unterbrechung der elektronischen Kommunikation gewährleistet wird.

Dabei werden die Energiewerte HOMO(AF1), HOMO(AF2), LUMO(AF1), LUMO(AF2) mithilfe der Dichtefunktionaltheorie (DFT) berechnet, wobei die Anknüpfungspositionen der chemischen Einheiten AF1 und AF2 und der Separatoren mit einem Wasserstoffatom entsprechend ihrer chemischen Valenzen abgesättigt werden. Die angegeben Grenzen beziehen sich auf Orbitalenergien in eV, die mit dem BP86-Funktional berechnet werden (Becke, A. D. Phys. Rev. A1988, 38, 3098-3100; Perdew, J. P. Phys. Rev. B1986, 33, 8822-8827).
Die elektronische Kommunikation zwischen den zwei chemischen Einheiten AF1 und AF2 über konjugierte Bindungen mit einem optionalen Separator ist unterbrochen, wenn die Grenzorbitale HOMO und LUMO auf separaten Molekülteilen lokalisiert sind, sodass ein Charge-Transfer Übergang ermöglicht wird. Die Lokalisierung der Grenzorbitale HOMO oder LUMO wird dabei mithilfe der Dichtefunktionaltheorie (DFT) mit dem BP86-Funktional (Becke, A. D. Phys. Rev. A1988, 38, 3098-3100; Perdew, J. P. Phys. Rev. B1986, 33, 8822-8827) visualisiert: Aus der Einelektronen-Wellenfunktion wird die Einelektronendichte durch Quadrieren berechnet und über den Raum integriert, den der untersuchte Molekülteil einnimmt. Dieser Raum kann aus den Atomkoordinaten und den van-der-Waals-Radien der Atome bestimmt werden. Die resultierende Zahl entspricht dem Anteil des Orbitals auf dem Molekülteil. Eine mehrheitliche Trennung der Grenzorbitale entspricht dabei einem Überlappungsparameter O im Bereich 0.1-20%, um einen Charge-Transfer Übergang zu ermöglichen. Der Überlappungsparameter O zwischen der HOMO-Wellenfunktion $\phi_a$ und der der LUMO-Wellenfunktion $\phi_b$ ergibt sich aus dem Integral über den gesamten Raum über den jeweils kleineren Wert der quadrierten Wellenfunktion:

$$O = \int \rho \, \mathrm{d}\tau \text{ mit } \rho = \begin{cases} \varphi_a^2, & \text{wenn } |\varphi_a| < |\varphi_b| \\ \varphi_b^2, & \text{wenn } |\varphi_a| \geq |\varphi_b| \end{cases}$$

In einer Ausführungsform ist in Formel 1 bei J gleich einer Einfachbindung E ungleich S.

In einer Ausführungsform weisen die organischen Moleküle eine Struktur der Formel 2a/2b auf oder haben eine Struktur der Formel 2a/2b, wobei die bei Formel 1 angegebenen Definitionen gelten und das organische Molekül zwei verschiedene AF (AF1 und AF2) aufweist.

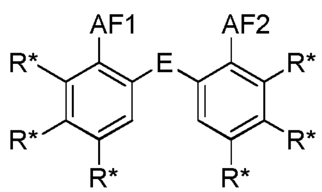

Formel 2a

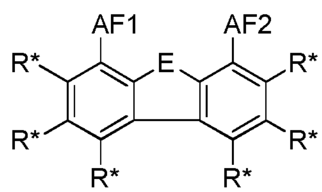

Formel 2b

In einer Ausführungsform sind die organischen Moleküle ausgewählt aus der im Folgenden dargestellten Gruppe:

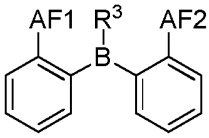
2-1

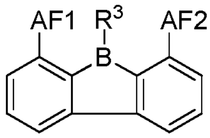
2-2

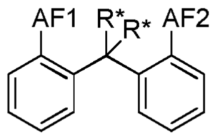
2-3

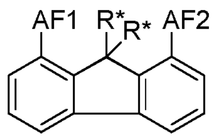
2-4

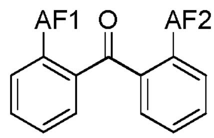
2-5

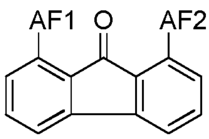
2-6

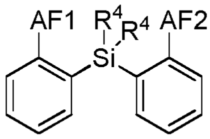
2-7

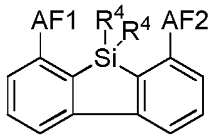
2-8

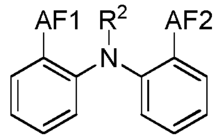
2-9

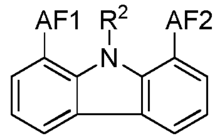
2-10

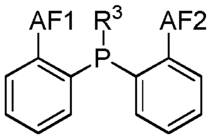
2-11

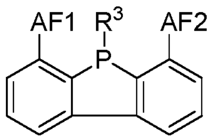
2-12

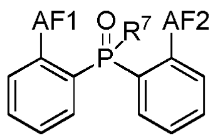
2-13

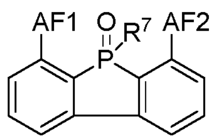
2-14

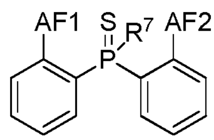
2-15

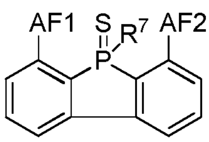
2-16

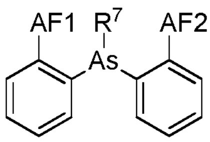
2-17

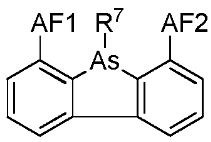
2-18

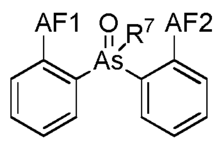
2-19

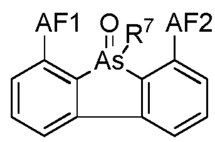
2-20

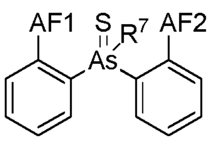
2-21

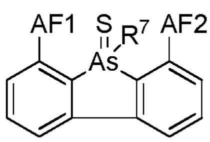
2-22

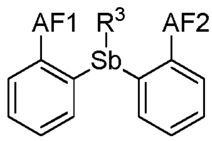
2-23

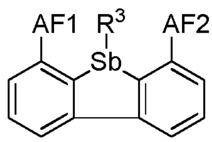
2-24

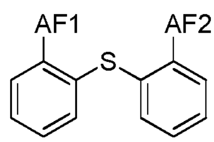
2-25

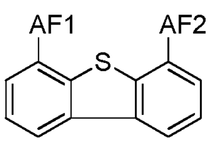
2-26

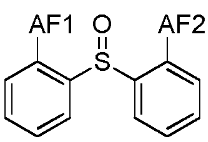
2-27

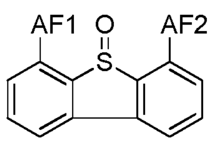
2-28

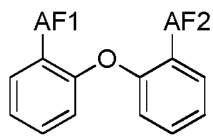
2-31

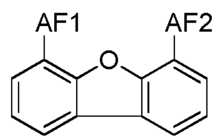
2-32

wobei alle Phenylringe mit R* substituiert sind.

Der Teil des organischen Moleküls gemäß Formel 1, Formel 2a oder Formel 2b, der nicht chemische Einheiten AF darstellt, wird auch als Separator S bezeichnet.

Streicht man in Formeln 1 und 2a und 2b die chemische Einheit AF, so verbleiben die Molekülfragmente, die hier als Separator S bezeichnet werden.

In einer Ausführungsform handelt es sich bei dem Separator um ein Molekülfragment mit einer der nachfolgenden Strukturen, wobei # die Stelle kennzeichnet, an denen die chemischen Einheiten AF an den Separator gebunden

werden:

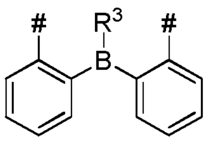 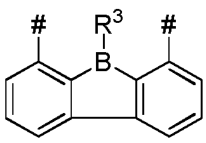 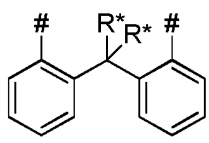 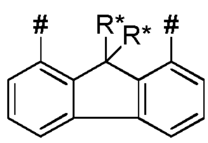 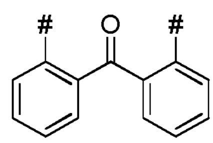

3-1 3-2 3-3 3-4 3-5

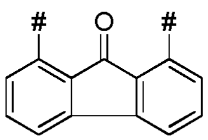 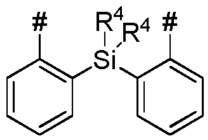 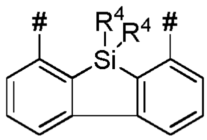 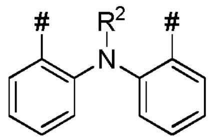 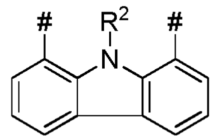

3-6 3-7 3-8 3-9 3-10

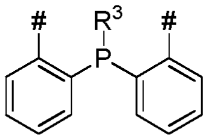 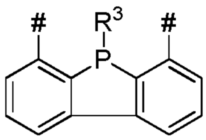 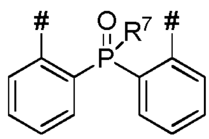 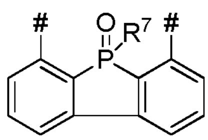 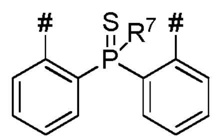

3-11 3-12 3-13 3-14 3-15

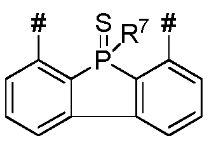 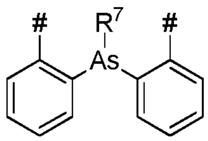 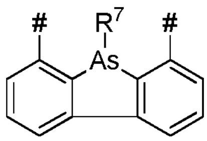 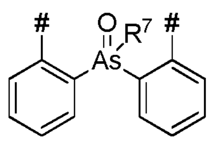 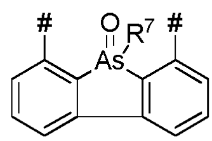

3-16 3-17 3-18 3-19 3-20

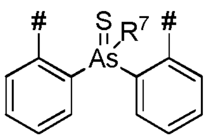 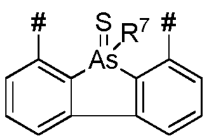 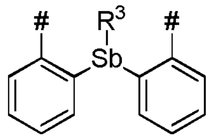 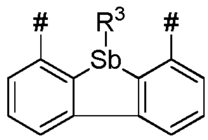 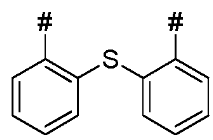

3-21 3-22 3-23 3-24 3-25

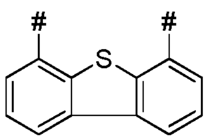 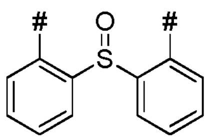 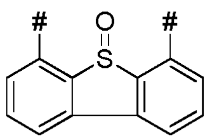 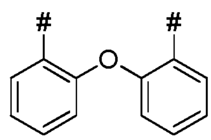 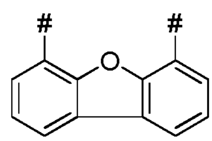

3-26 3-27 3-28 3-31 3-32

**Formel 3**

wobei alle Phenylringe mit R* substituiert sind.

In einer Ausführungsform unterscheidet insbesondere der Separator S die organischen Moleküle funktionell von Molekülen gemäß dem Stand der Technik, da die hier gezeigte Art der Trennung von AFs (oder Donoren und Akzeptoren) über den Separator bisher noch nicht bekannt ist. Separatoren dienen der Unterbrechung der elektronischen Kommunikation zwischen den chemischen Einheiten AF1 und AF2 durch eine derartige Verknüpfung der Einheiten, dass die Grenzorbitale HOMO und LUMO auf mehrheitlich separaten Molekülteilen liegen, was ohne den Separator nicht zwingend der Fall sein muss.

Separatoren im Sinne dieser Erfindung verändern die Lage des HOMO bzw. LUMO der AFs nicht signifikant. Als nicht signifikant gilt im Rahmen dieser Erfindung eine Veränderung von nicht mehr als +/- 0,4 eV. Die Berechnung

derartiger Energien ist bekannt und funktioniert nach der oben beschriebenen Weise per DFT-Rechnung.

Anhand spektroskopischer Auswahlregeln (symmetrische Moleküle) oder durch Messung des Extinktionskoeffizienten (UV/VIS-Spektroskopie) oder anhand quantenchemischer Berechnung der Oszillatorstärke kann vorhergesagt werden, ob ein quantenmechanischer Übergang erlaubt ist. Je größer die Oszillatorstärke, desto eher ist ein Übergang erlaubt und desto schneller ist der damit verbundene Prozess (Abklingdauer). Angestrebt sind Abklingdauern von < 300 $\mu$s, insbesondere < 100 $\mu$s, oder < 50 $\mu$s. Bei einer langen Abklingdauer des (organischen) Emitters kommt es bei hohen Stromstärken schnell zu Sättigungseffekten, was die Bauteillebensdauer negativ beeinflusst und die Erreichung hoher Helligkeiten verhindert.

Ein Maß für die Abklingdauer ist das quantenmechanischen Überlappungsintegral, welches näherungsweise durch den oben definierten Überlappungsparameter O dargestellt wird. Je kleiner das Überlappungsintegral, desto stärker sind die Grenzorbitale HOMO und LUMO getrennt, und umso wahrscheinlicher der Charge-Transfer-Übergang. Jedoch sinkt gleichermaßen die Wahrscheinlichkeit einer TADF-Emission aufgrund abnehmender Oszillatorstärken.

Bei einem Wert von 1 für den Überlappungsparameter O liegt nicht mehr verzögerte Fluoreszenz (TADF) aufgrund eines Charge-Transfer Übergangs vor.

Um eine effiziente TADF-Emission mit kurzen Abklingdauern zu erreichen, muss das Überlappungsintegral gezielt gesteuert werden. Die gewünschte Überlappung wird durch die geeignete Wahl eines erfindungsgemäßen Separators S erreicht.

Die erfindungsgemäßen Separatoren S erfüllen dabei insbesondere zwei wesentliche funktionelle Merkmale (s. Figur 2):

- die HOMO-Energie ist niedriger als die HOMO-Energie der als Donor fungierenden chemischen Einheit AF2 und
- die LUMO-Energie ist höher als die LUMO-Energie der als Akzeptor fungierenden chemischen Einheit AF1.

In einer Ausführungsform weist das organische Molekül einen Separator S auf, der ein Molekülfragment einer der in Formel 3 angegebenen Strukturen aufweist, wobei # die Stelle kennzeichnet, an denen die AF an den Separator gebunden werden und das organische Molekül zwei verschiedene AF (AF1 und AF2) aufweist.

In einer Ausführungsform weist die Einheit AF1 eine Struktur der Unterformel 1 auf oder hat eine Struktur der Unterformel 1

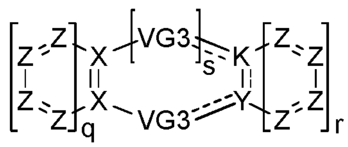

**Unterformel 1**

wobei gilt:

q ist 0 oder 1; r ist 0 oder 1; s ist 0 oder 1;

VG3 = verbrückende Gruppe ist bei jedem Auftreten unabhängig voneinander ausgewählt aus der Gruppe bestehend aus

N, O, S, CR**, C, eine Element-Element-Einfachbindung zwischen X und Y oder zwischen X und K, wobei nicht 2 Einheiten VG3 gleichzeitig eine Element-Element-Einfachbindung zwischen X und Y und zwischen X und K sind;
BR**, NR**, $GeR^{**}_2$, $AsR^{**}_2$, $SiR^{**}_2$, wobei nicht zwei Einheiten VG3 gleichzeitig gleich BR**, NR**, $GeR^{**}_2$, $AsR^{**}_2$, $SiR^{**}_2$ sind;

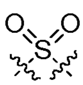, 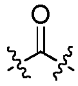, 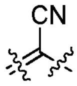, 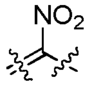, 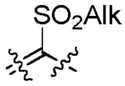, 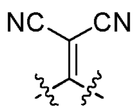, 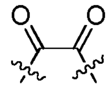, 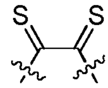, 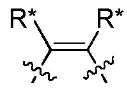,

und

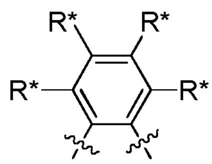,

wobei nicht zwei Einheiten VG3 gleichzeitig gleich

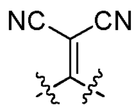,

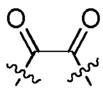, 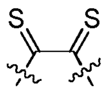, 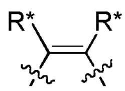, 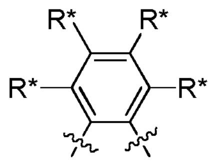

sind;

X ist bei jedem Auftreten unabhängig voneinander C; oder ist CR** oder N wenn q = 0;

Y ist C; oder ist CR**, $CR^{**}_2$, N, NR**, O oder S wenn r = 0;

K ist Y; oder ist R* wenn r = 0 und gleichzeitig s = 0;

Z ist bei jedem Auftreten unabhängig voneinander CR** oder N;

und wobei maximal 4 der Einheiten VG3, K, X, Y, Z gleichzeitig gleich N sind;
und wobei mindestens 1 der Einheiten VG3, K, X, Y, Z gleich einer von C-H verschiedenen Gruppe ist, die mindestens 1 Stickstoff- oder ein Sauerstoff- oder ein Schwefelatom enthält;

und wobei im Falle von r = 0 auch zwei benachbarte Gruppen Z miteinander oder eine Gruppe VG3 mit der ihr am nächsten gelegenen Gruppe Z oder X oder Y über die folgenden Einheiten verbrückt sein können:

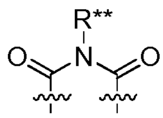, 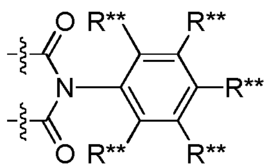;

wobei maximal drei dieser Einheiten enthalten sind;

R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform weist die Einheit AF1 eine Struktur der Unterformeln 1.1 bis 1.10 auf oder hat eine Struktur der Unterformeln 1.1 bis 1.10

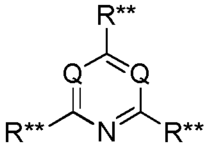

Unterformel 1.1

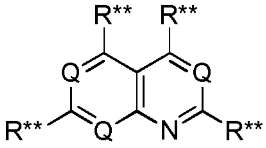

Unterformel 1.2

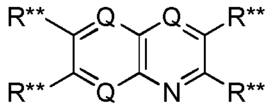

Unterformel 1.3

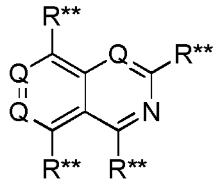

Unterformel 1.4

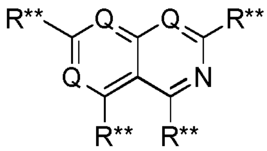

Unterformel 1.5

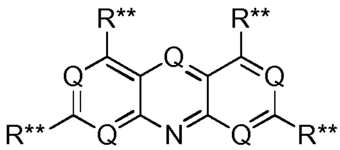

Unterformel 1.6

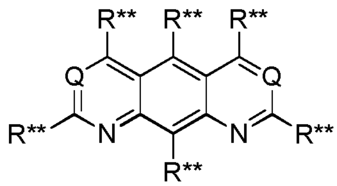

Unterformel 1.7

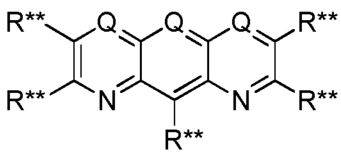

Unterformel 1.8

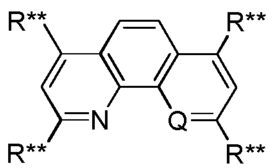

Unterformel 1.9

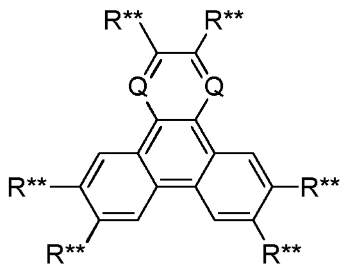

Unterformel 1.10

und wobei gilt:

Q ist N, CR**, wobei nicht zwei direkt benachbarte Einheiten Q gleichzeitig gleich N sind;

R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform weist die Einheit AF1 eine Struktur der Unterformeln 1.11 bis 1.13 auf oder hat eine Struktur der Unterformeln 1.11 bis 1.13

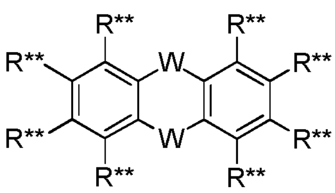

Unterformel 1.11

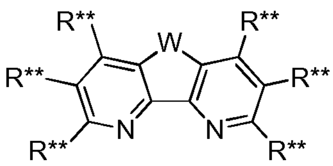

Unterformel 1.12

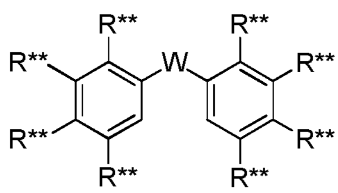

Unterformel 1.13

und wobei gilt:
W ist

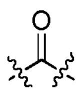 , 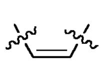 , 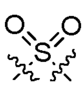 ,

eine Element-Element-Einfachbindung, wobei nicht zwei Einheiten W gleichzeitig eine Element-Element-Einfachbindung

sind, NR**, wobei nicht zwei Einheiten W gleichzeitig gleich NR** sind;
R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform weist die Einheit AF1 eine Struktur der Unterformel 1.14 auf oder hat eine Struktur der Unterformel 1.14

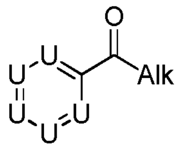

**Unterformel 1.14**

und wobei gilt:

U ist CR** oder N, wobei maximal 3 Einheiten U gleichzeitig gleich N sind und wobei keine benachbarten Einheiten U gleichzeitig gleich N sind;

Alk ist ausgewählt aus der Gruppe bestehend aus Methyl, Ethyl, Propyl *iso*-Propyl, Butyl, *tert*-Butyl, Pentyl, Hexyl, 2-Ethylhexyl, Cyclohexyl, Heptyl, Octyl, Nonyl, Decyl, Undecyl und Dodecyl;

R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform weist die Einheit AF1 eine Struktur der Unterformeln 1.15 bis 1.22 auf oder hat eine Struktur der Unterformeln 1.15 bis 1.22

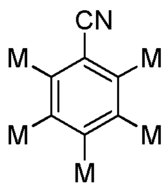

**Unterformel 1.15**

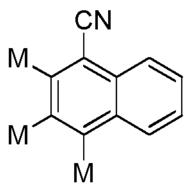

**Unterformel 1.16**

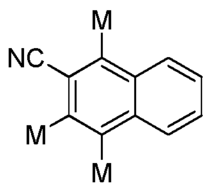

**Unterformel 1.17**

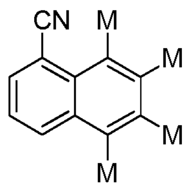

**Unterformel 1.18**

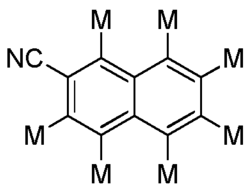

**Unterformel 1.19**

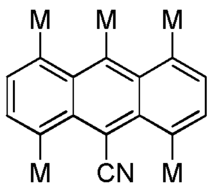

**Unterformel 1.20**

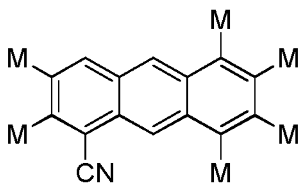

**Unterformel 1.21**

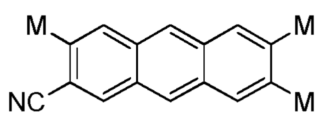

**Unterformel 1.22**

und wobei gilt:
M ist ausgewählt aus der Gruppe bestehend aus H, Deuterium, Alk, Phenyl, Pyridyl und CN, wobei maximal 4 Einheiten M gleichzeitig gleich CN sind, oder kennzeichnet eine chemische Bindung an einen Separator S, wobei genau ein M eine chemische Bindung an einen Separator S ist;
In einer weiteren Ausführungsform weist die Einheit AF1 eine Struktur der Unterformeln 1.23 bis 1.24 auf oder hat eine Struktur der Unterformeln 1.23 bis 1.24

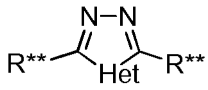
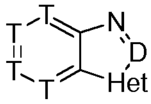

**Unterformel 1.23** **Unterformel 1.24**

und wobei gilt: Het ist NR**, O, S, $SO_2$;
D ist N oder CR**;
T ist N oder CR**, wobei maximal 2 Einheiten T gleich N sind, und wobei keine benachbarten Einheiten T gleichzeitig gleich N sind;
R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform weist die Einheit AF1 eine Struktur der Unterformeln 1.25 bis 1.36 auf oder hat eine Struktur der Unterformeln 1.25 bis 1.36

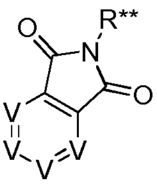
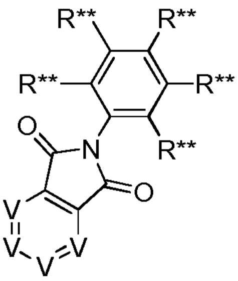
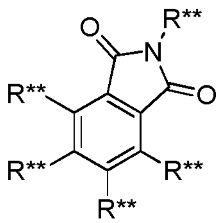
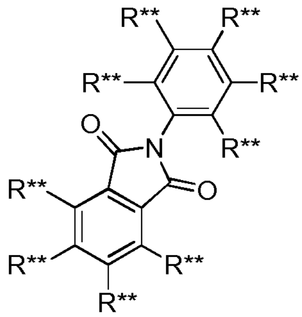

**Unterformel 1.25** **Unterformel 1.26** **Unterformel 1.27** **Unterformel 1.28**

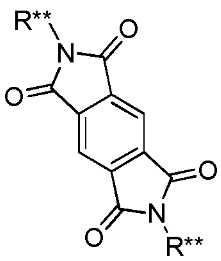
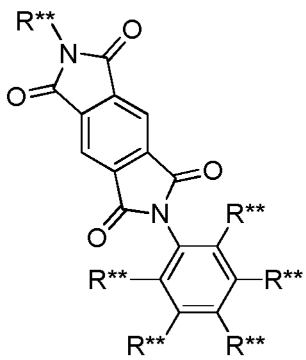
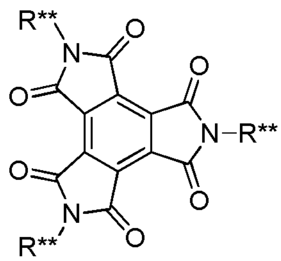

**Unterformel 1.29** **Unterformel 1.30** **Unterformel 1.31**

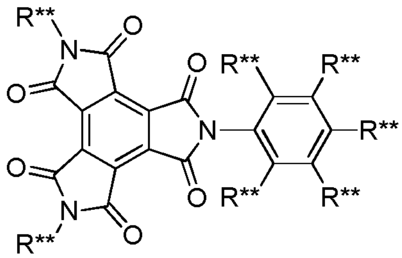
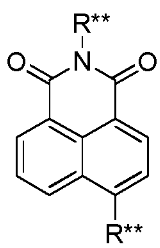
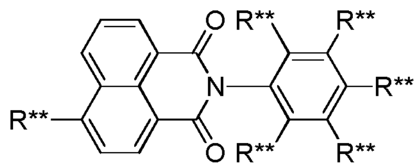

**Unterformel 1.32** **Unterformel 1.33** **Unterformel 1.34**

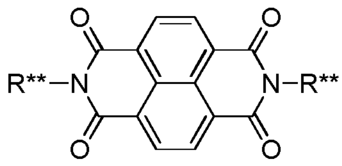
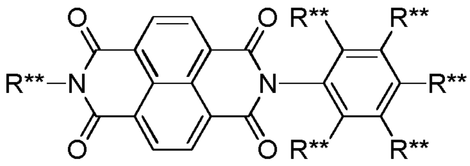

**Unterformel 1.35** **Unterformel 1.36**

und wobei gilt: V ist bei jedem Auftreten CR**, N, wobei mindestens eine Einheit V gleich N ist; und wobei maximal zwei Einheiten V gleichzeitig gleich N sind; und wobei nicht zwei einander benachbarte Einheiten V gleichzeitig gleich N sind; R** ist bei jedem Auftreten unabhängig voneinander entweder ein Rest R*, oder eine chemische Bindung an einen Separator S, wobei genau ein R** eine chemische Bindung an einen Separator S ist.

R* ist wie bei Formel 1 definiert.

In einer weiteren Ausführungsform der organischen Moleküle weist die Einheit AF2 eine Struktur der Unterformel 2 auf oder hat eine Struktur der Unterformel 2

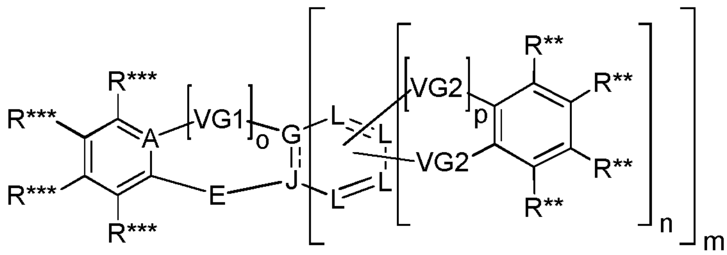

**Unterformel 2**

wobei gilt:

m ist 0 oder 1;
n ist 0 oder 1, wobei bei m = 0 immer auch n = 0 ist;
o ist 0 oder 1;
p ist 0 oder 1;
A ist CR*** wenn o = 0, ansonsten C;
VG1 = verbrückende Gruppe, ist ausgewählt aus der Gruppe bestehend aus

- NR**, CR**$_2$, O, S und einer C-C-Einfachbindung; oder
- NR**, CR**$_2$, O, S, einer C-C-Einfachbindung, BR**, AsR**, SiR**$_2$, GeR**$_2$,

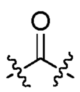, 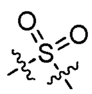, 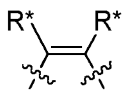, 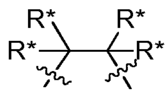, 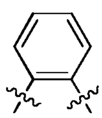, 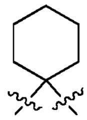, 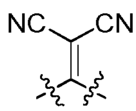, 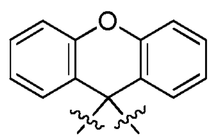,

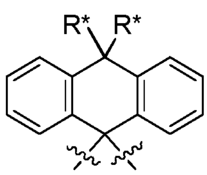, 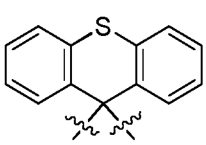, und 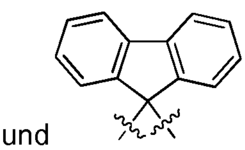,

wenn m = 1 und gleichzeitig n = 0; VG2 = verbrückende Gruppe ist bei jedem Auftreten unabhängig voneinander ausgewählt aus der Gruppe bestehend aus CR**$_2$, NR**, O, S und einer C-C-Einfachbindung, wobei nicht zwei Einheiten VG2 gleichzeitig gleich einer C-C-Einfachbindung sind;

E ist ausgewählt aus der Gruppe bestehend aus NR**,

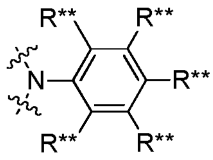
,

O und S;

G ist C wenn o = 1 und gleichzeitig m = 1; ist CR** wenn o = 0 und gleichzeitig m = 1; ist CR**, CR**$_2$, wenn o = 1 und gleichzeitig m = 0; ist R* wenn o = 0 und gleichzeitig m = 0; ist CR**, CR**$_2$, N oder NR* wenn m = 0 und gleichzeitig VG1 eine C-C-Einfachbindung ist;

J ist C wenn m = 1; ist CR**, CR**$_2$, NR** wenn m = 0;

L ist CR*** wenn n = 0; ist CR**, C (bei kovalenter Bindung zu VG2) wenn n = 1;

R*** ist R** oder ist ausgewählt aus den folgenden Einheiten, wobei maximal zwei der Reste R*** gleichzeitig gleich einer der folgenden Einheiten sind:

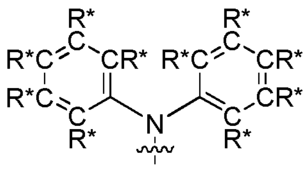
,
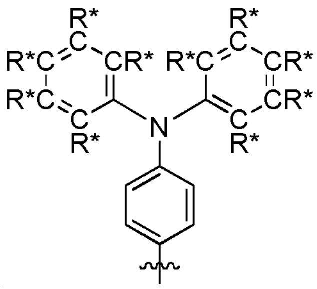
,
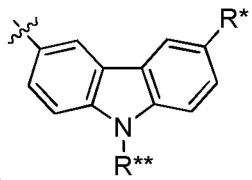
,
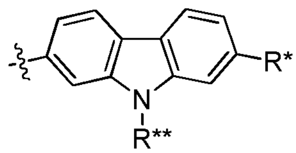
,

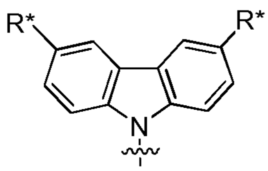
,
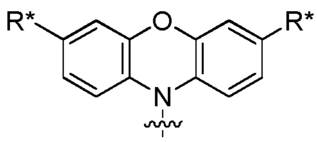
,
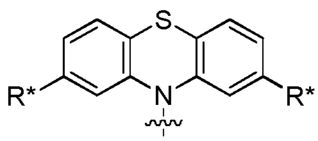
,

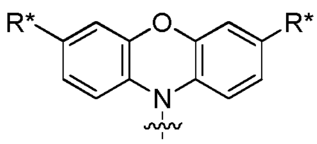
,
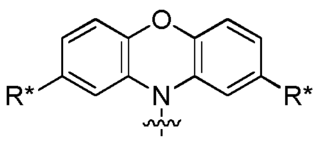
;

R** ist bei jedem Auftreten unabhängig voneinander ein Rest R* und/oder markiert eine Anknüpfungsstelle an einen Separator S, wobei genau ein R** eine Anknüpfungsstelle an einen Separator S ist.

In einer weiteren Ausführungsform weist die Einheit AF2 eine Struktur der Unterformel 3 auf oder hat eine Struktur der Unterformel 3

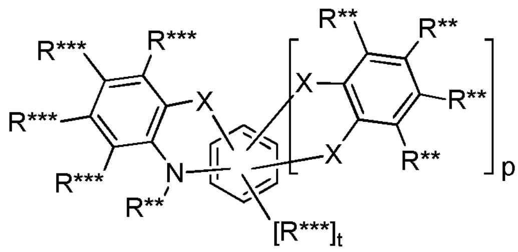

**Unterformel 3**

wobei in Unterformel 3 bedeutet:

p ist 0 oder 1;

t = 4 - 2p;

X ist CR**$_2$, NR**, Sauerstoff, Schwefel, eine direkte Bindung, wobei maximal zwei Platzhalter X gleichzeitig eine direkte Bindung sind, wobei diese nicht Bestandteil desselben Rings sind;

und im Übrigen die für Unterformel 2 gegebenen Definitionen gelten.

In einer weiteren Ausführungsform weist die Einheit AF2 des organischen Moleküls eine Struktur der Formel 4A1-4A7 auf oder hat eine Struktur der Formel 4A1-4A7

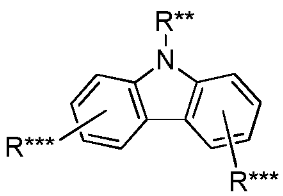

**Formel 4A1**

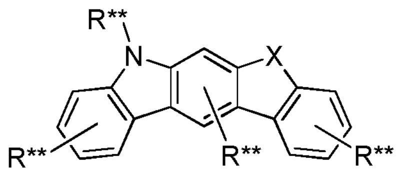

**Formel 4A2**

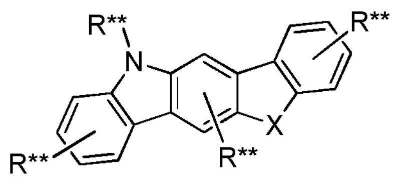

**Formel 4A3**

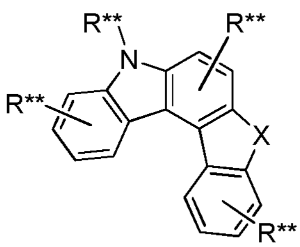

**Formel 4A4**

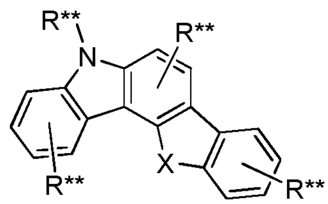

**Formel 4A5**

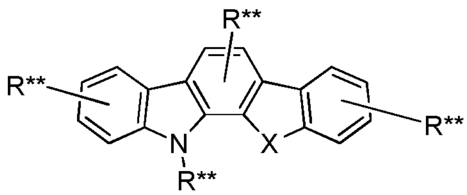

**Formel 4A6**

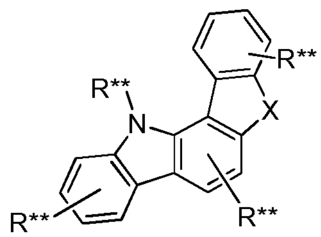

**Formel 4A7**

wobei in Formel 4A1-4A7 gilt:

X ist $C(R^{**})_2$, $NR^{**}$, Sauerstoff, Schwefel;

und im Übrigen die für Unterformel 2 angegebenen Definitionen gelten.

In einer weiteren Ausführungsform weisen die organischen Moleküle eine Struktur der Formel 5a/5b auf oder haben eine Struktur der Formel 5a/5b

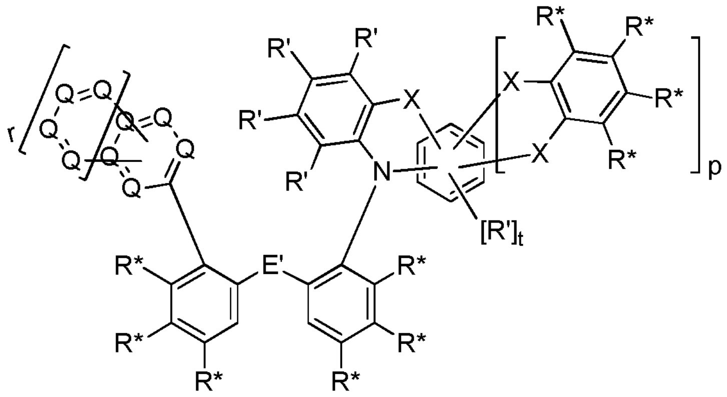

**Formel 5a**

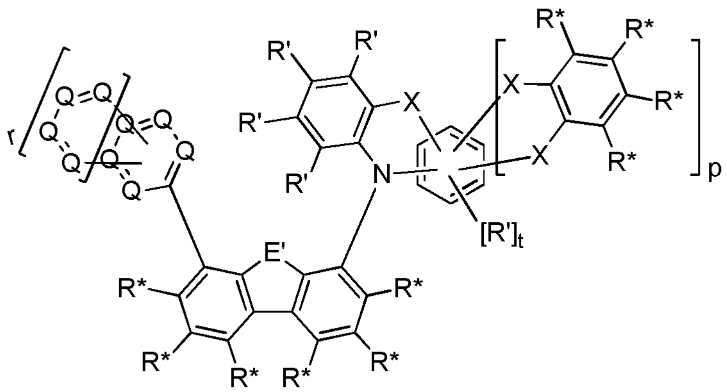

**Formel 5b**

wobei in Formel 5a/5b bedeutet:

p ist 0 oder 1;

t = 4 - 2p;

X ist $CR^*_2$, $NR^*$, Sauerstoff, Schwefel, eine direkte Bindung, wobei maximal zwei Platzhalter X gleichzeitig eine direkte Bindung sind, wobei diese nicht Bestandteil desselben Rings sind;

Q ist N, $CR^*$ oder für r = 1 die entsprechenden Anellierungspositionen C, wobei nicht zwei direkt benachbarte Einheiten Q gleichzeitig gleich N sind;

r = 0 oder 1,
E' ist S, O, C(=O), $CR^*_2$, $Si(R^4)_2$, $Ge(R^4)_2$, $NR^2$, $BR^3$;

R' ist R* oder ist ausgewählt aus den folgenden Einheiten, wobei maximal zwei der Reste R' gleichzeitig gleich einer der folgenden Einheiten sind:

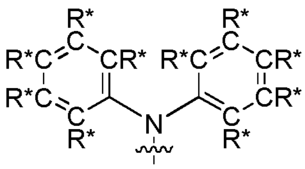, 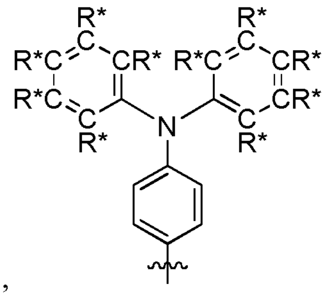, 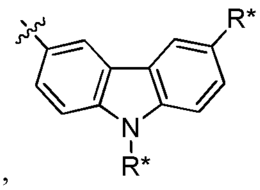, 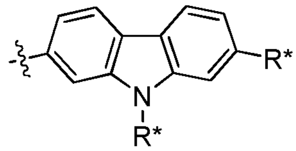,

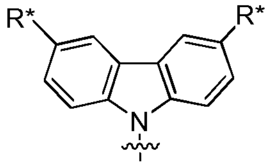, 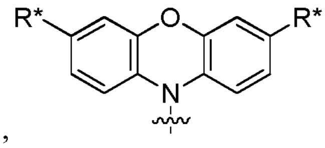, 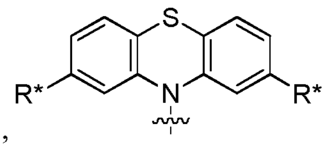,

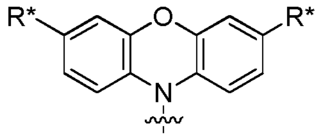, 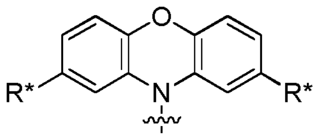;

und R* wie bei Formel 1 definiert ist.

In einer weiteren Ausführungsform weisen die organischen Moleküle eine Struktur der Formel 6a/6b auf oder haben eine Struktur der Formel 6a/6b

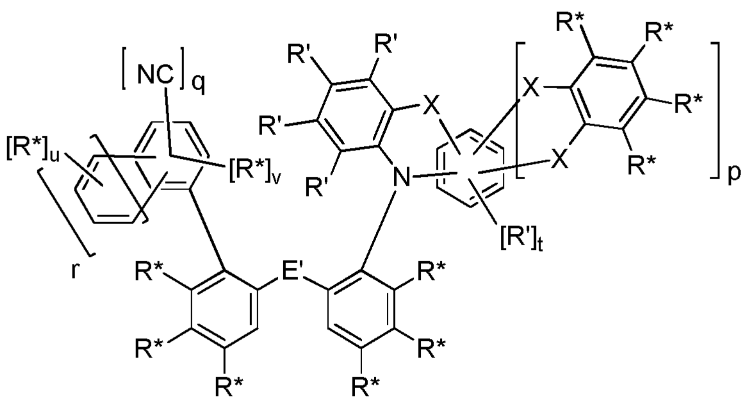

**Formel 6a**

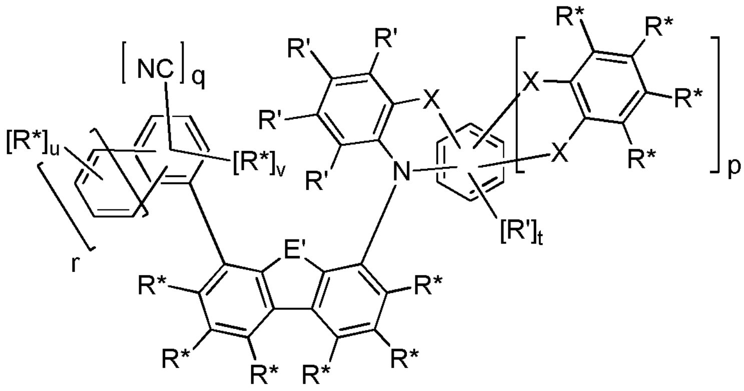

**Formel 6b**

wobei in Formel 6a/6b bedeutet:

p ist 0 oder 1;

t = 4 - 2p;

X ist $CR^*_2$, NR*, Sauerstoff, Schwefel, eine direkte Bindung, wobei maximal zwei Platzhalter X gleichzeitig eine direkte Bindung sind, wobei diese nicht Bestandteil desselben Rings sind;

r = 0 oder 1,

q = 1 bis 5, für r = 1 ist q = 1 bis 3,

u ist 1, 2, 3 oder 4

v ist 1, 2 oder 3; wobei gilt: u + v = 3 + 4r - q

E' ist S, O, C(=O), $CR^*_2$, $Si(R^4)2$, $Ge(R^4)_2$, $NR^2$, $BR^3$;

R' ist R* oder ist ausgewählt aus den folgenden Einheiten, wobei maximal zwei der Reste R' gleichzeitig gleich einer der folgenden Einheiten sind:

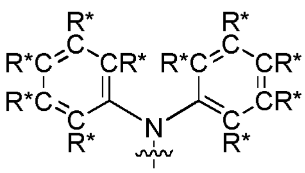 , 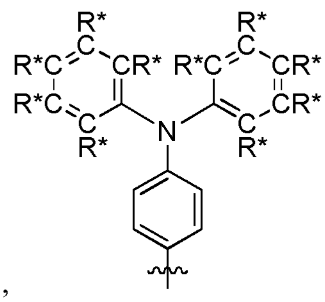 , 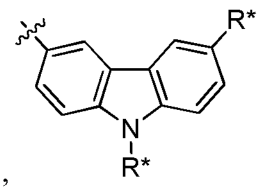 , 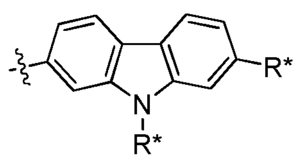 ,

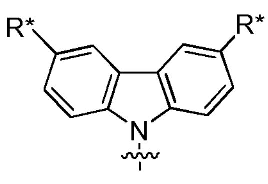 , 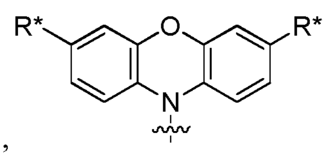 , 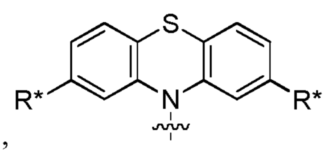 ,

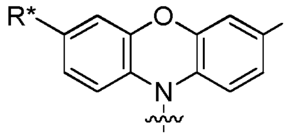 , 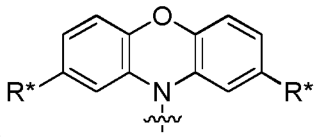 ;

und R* wie bei Formel 1 definiert ist.

In einer weiteren Ausführungsform weisen die organischen Moleküle eine Struktur der Formel 7a/7b auf oder haben eine Struktur der Formel 7a/7b

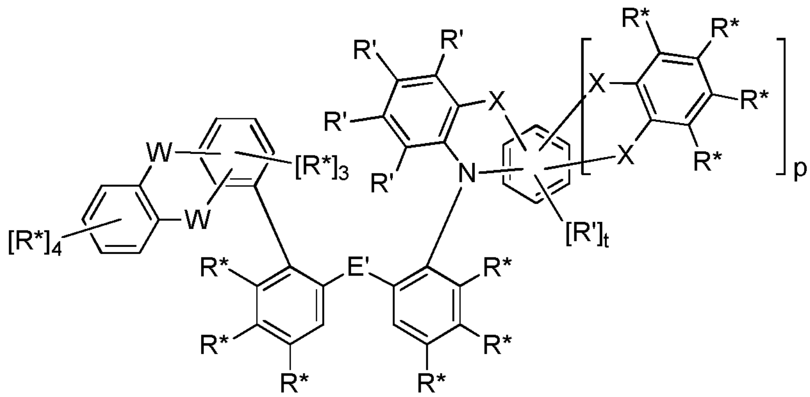

**Formel 7a**

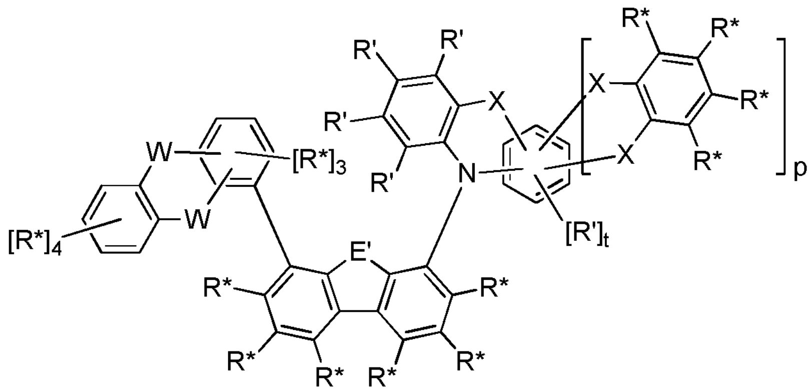

**Formel 7b**

wobei in Formel 7a/7b bedeutet:

p ist 0 oder 1;

t = 4 - 2p;

X ist $CR^*_2$, NR*, Sauerstoff, Schwefel, eine direkte Bindung, wobei maximal zwei Platzhalter X gleichzeitig eine direkte Bindung sind, wobei diese nicht Bestandteil desselben Rings sind;

W ist ausgewählt aus der Gruppe bestehend aus

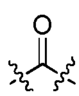 , 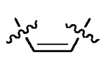 , 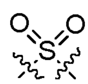 ,

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;

E' ist ausgewählt aus der Gruppe bestehend aus S, O, C(=O), $CR^*_2$, $Si(R^4)_2$, $Ge(R^4)_2$, $NR^2$ und $BR^3$;

R' ist R* oder ist ausgewählt aus der Gruppe der folgenden Einheiten, wobei maximal zwei der Reste R' gleichzeitig gleich einer der folgenden Einheiten sind:

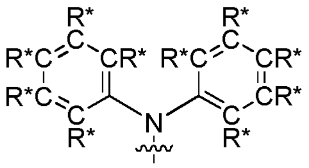 , 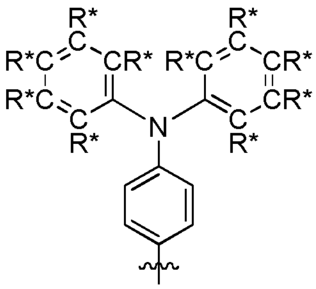 , 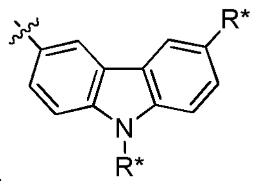 , 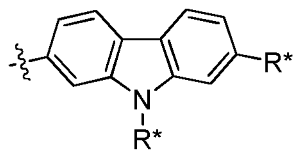 ,

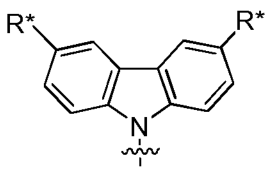 , 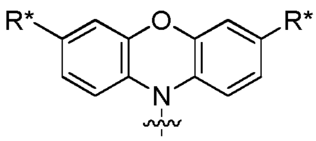 , 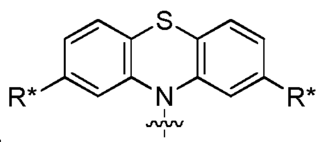 ,

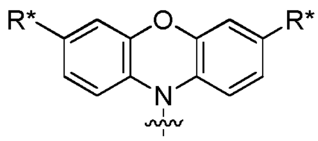 , 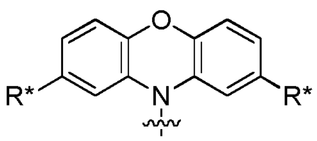 ;

und R* wie bei Formel 1 definiert ist.

In einer Ausführungsform ist bei den organischen Molekülen der Formeln 5b, 6b und 7b E' ausgewählt aus der Gruppe bestehend aus O, C(=O), $CR^*_2$, $Si(R^4)_2$, $Ge(R^4)_2$, $NR^2$, $BR^3$;

In einer Ausführungsform sind die chemischen Einheiten AF1 und AF2 ausgewählt aus den in Tabelle 1a und Tabelle 1b aufgeführten Verbindungen, wobei AF1 und AF2 nicht identisch sind.

**Tabelle 1a:** Auflistung der ersten chemischen Einheiten AF1Mögliche Anknüpfungspunkte der chemischen Einheit AF an einen Separator S sind mit Kleinbuchstaben bezeichnet.

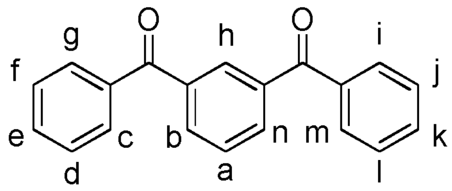

27

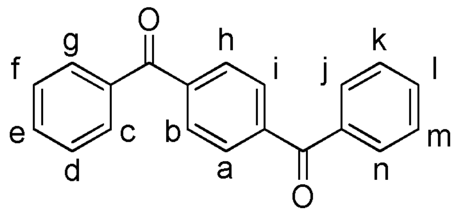

28

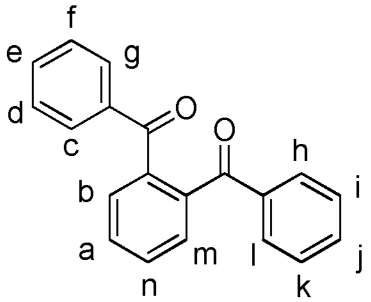

29

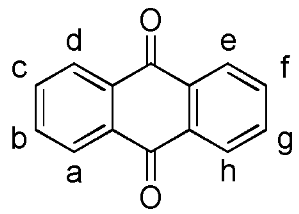

36

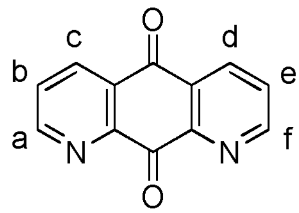

37

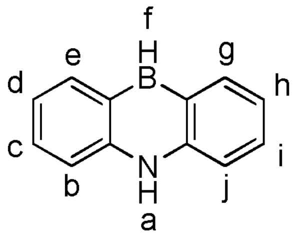

38

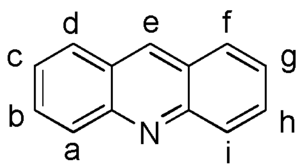

40

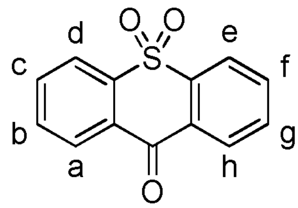

43

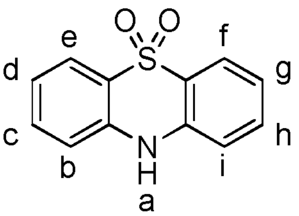

44

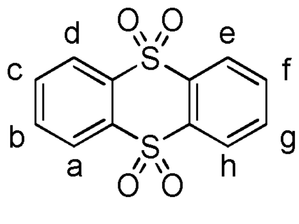

46

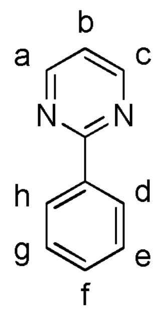

56

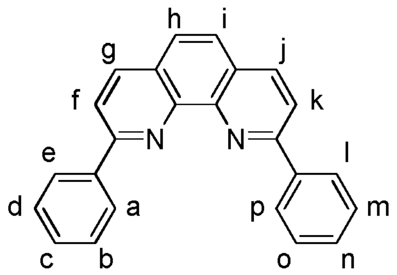

68

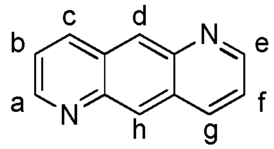

62

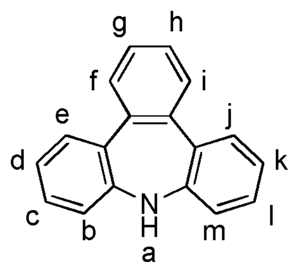

71

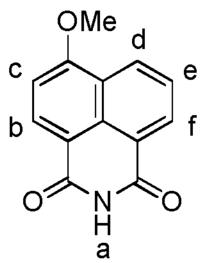

79

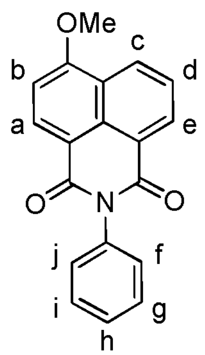

80

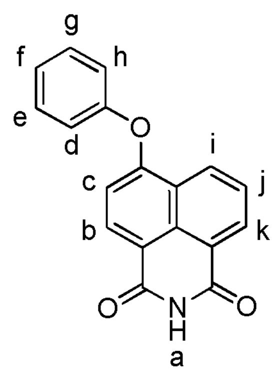

81

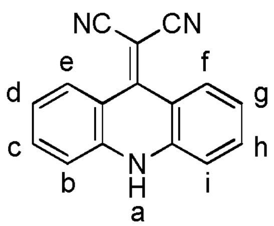

82

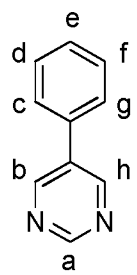

83

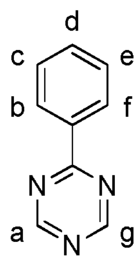

85

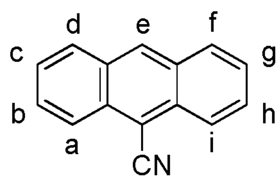

86

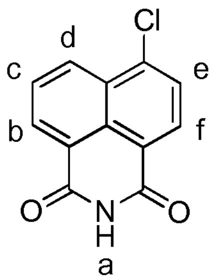

89

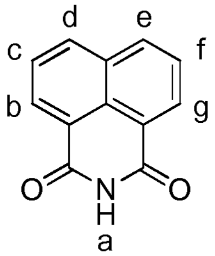

90

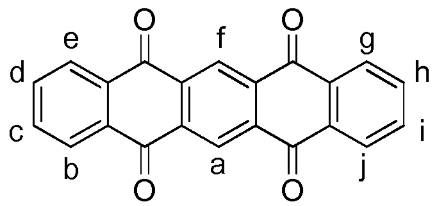

93

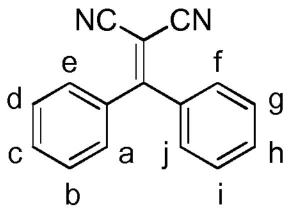

109

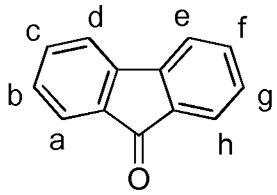

110

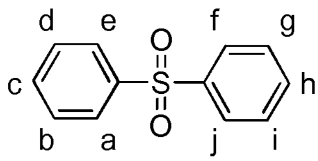

111

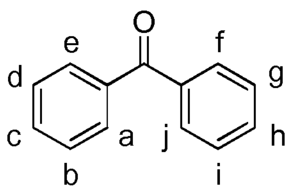

112

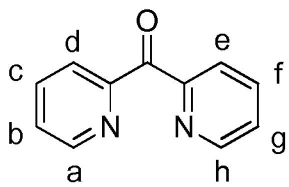

113

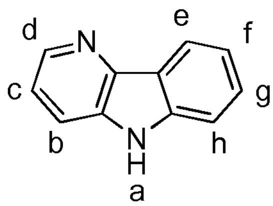

114

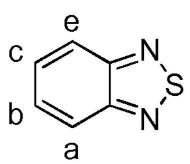

115

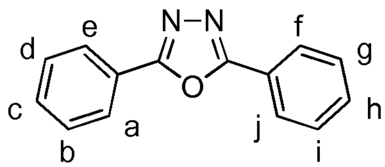

118

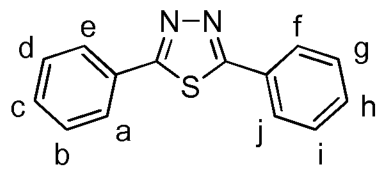

119

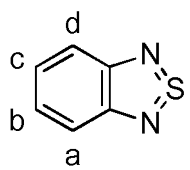

121

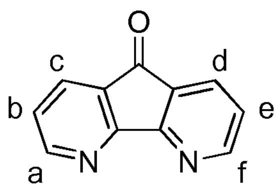

123

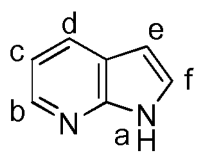

127

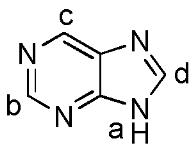
131

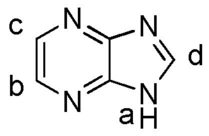
132

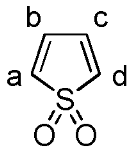
133

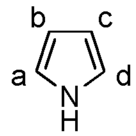
134

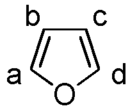
135

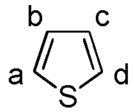
136

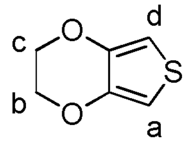
140

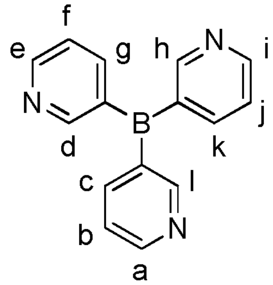
144

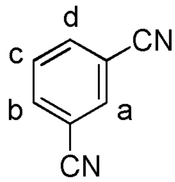
150

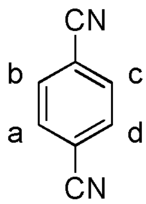
151

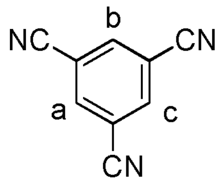
152

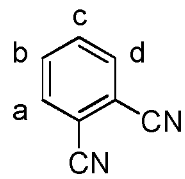
153

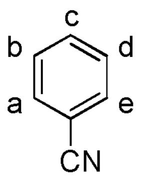
154

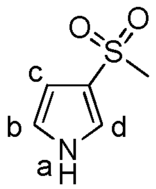
157

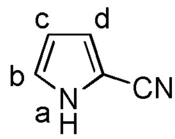
158

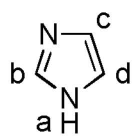
159

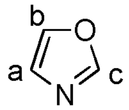
160

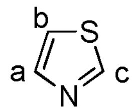
161

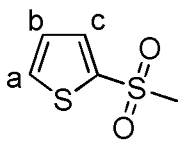
166

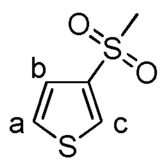
167

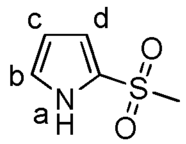

168

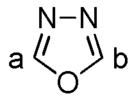

170

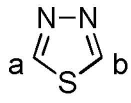

171

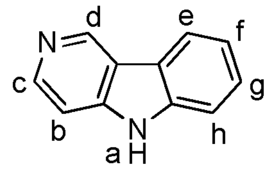

175

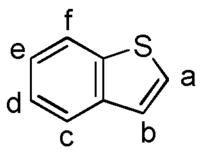

176

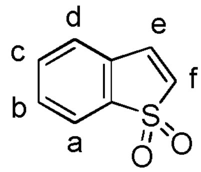

177

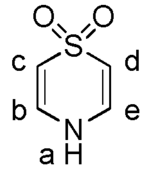

182

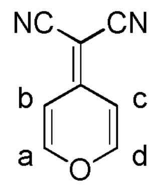

183

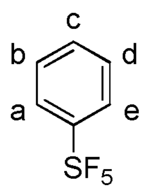

184

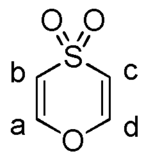

187

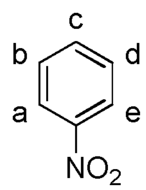

192

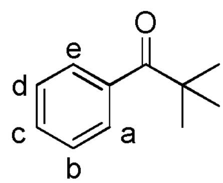

193

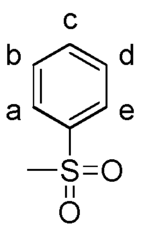

194

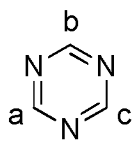

195

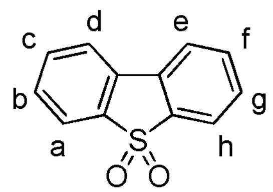

198

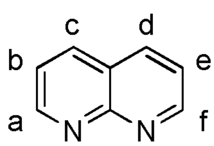

205

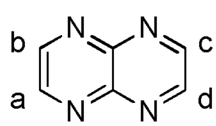

206

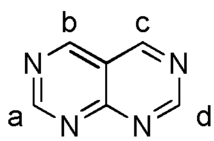

207

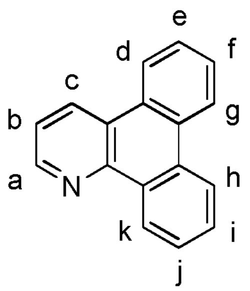
208

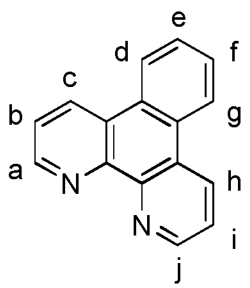
209

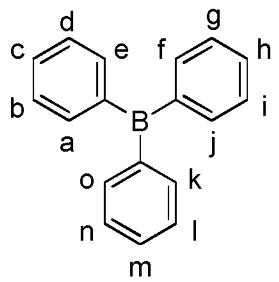
213

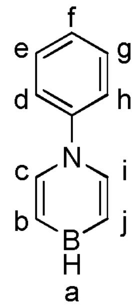
214

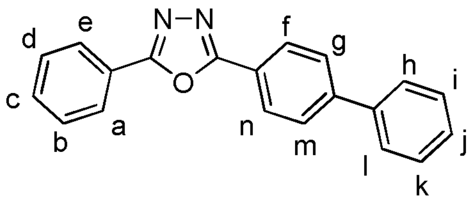
217

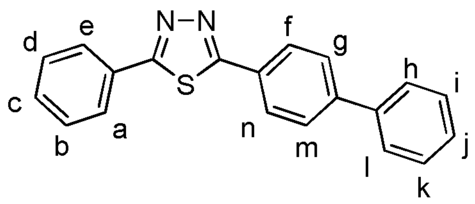
218

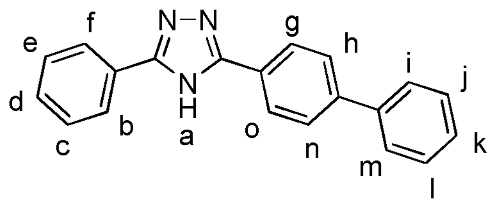
219

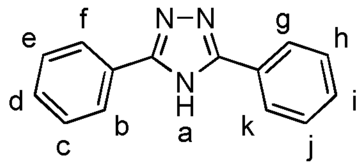
222

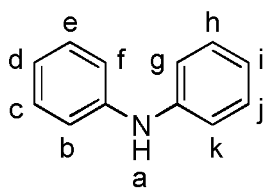
223

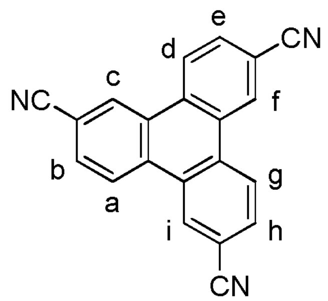
231

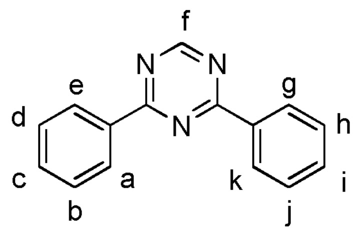
233

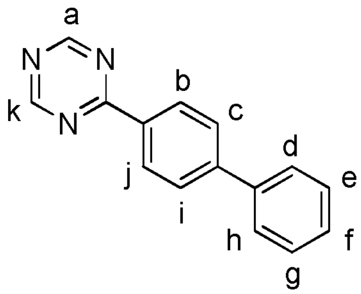

234

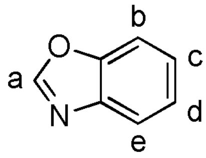

236

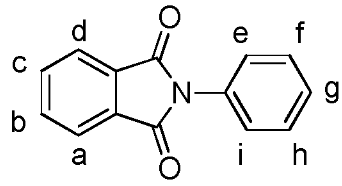

237

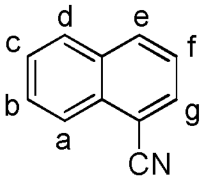

244

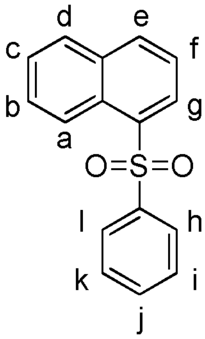

245

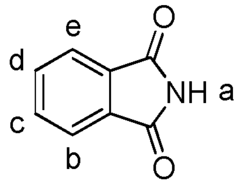

246

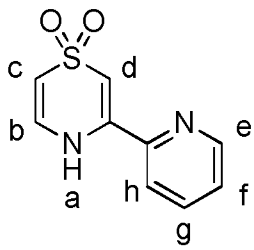

275

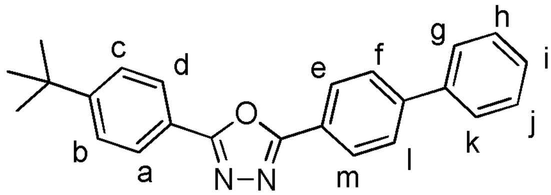

285

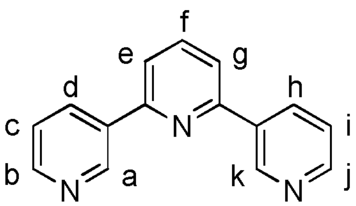

286

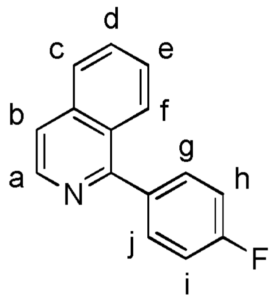

287

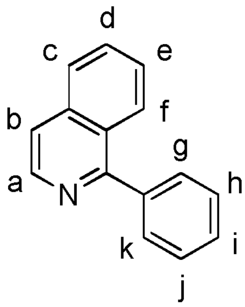

288

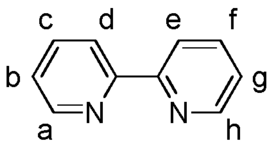

297

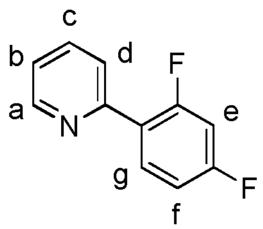

300

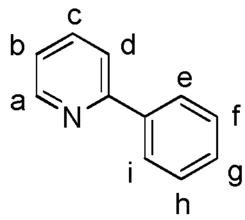

303

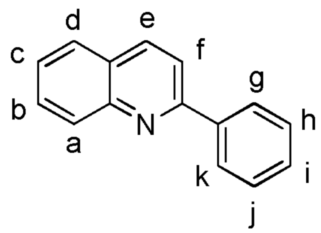

304

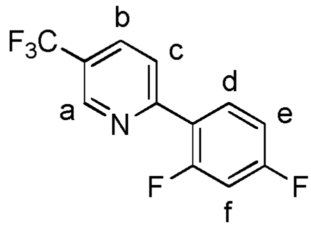

305

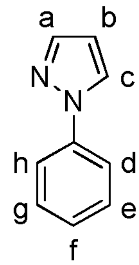

308

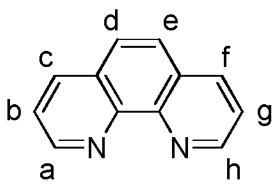

311

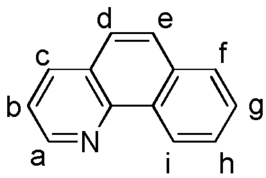

312

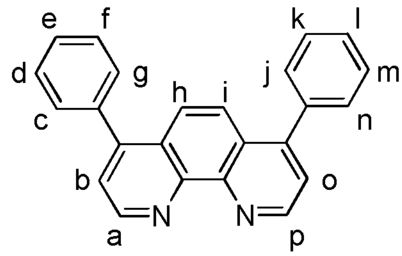

313

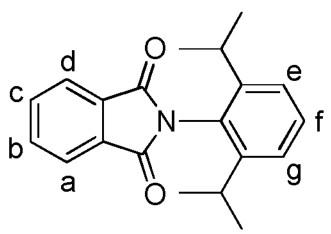

327

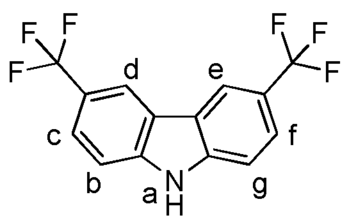

331

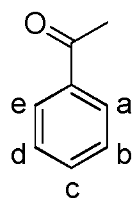

332

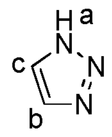

338

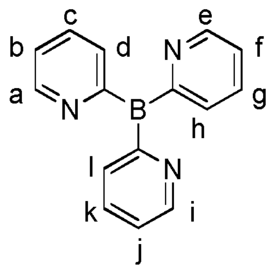

341

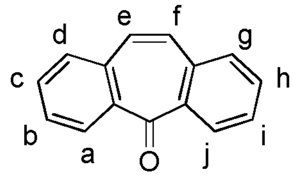

358

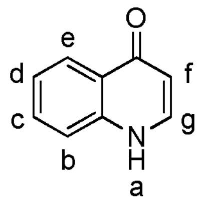

360

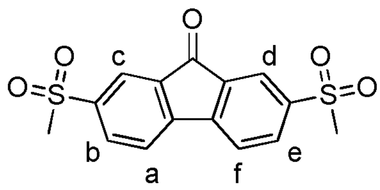

364

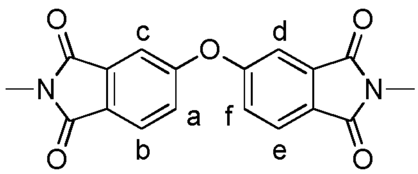

367

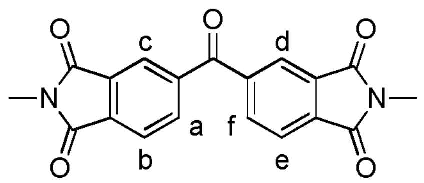

368

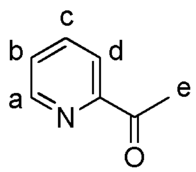

370

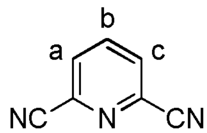

371

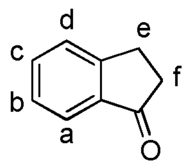

391

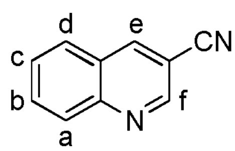

395

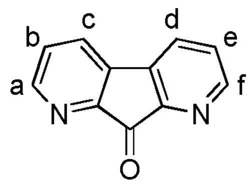

420

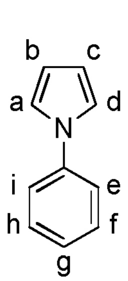

421

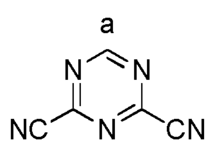

429

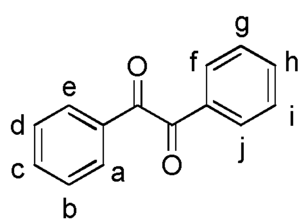

430

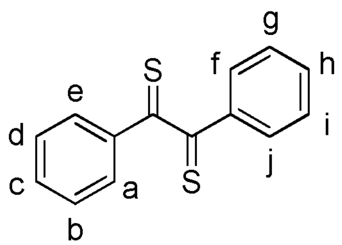

431

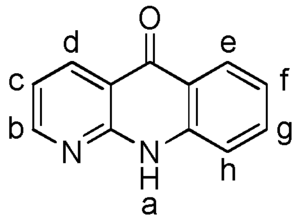

434

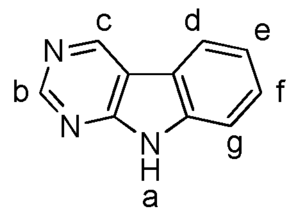

437

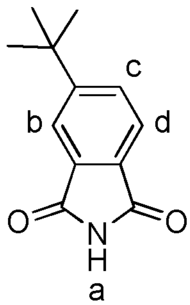

440

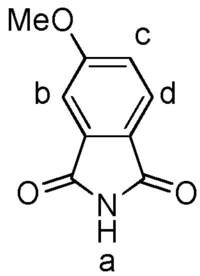

441

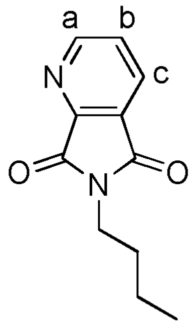

473

**Tabelle 1b:** Auflistung der zweiten chemischen Einheiten AF2.

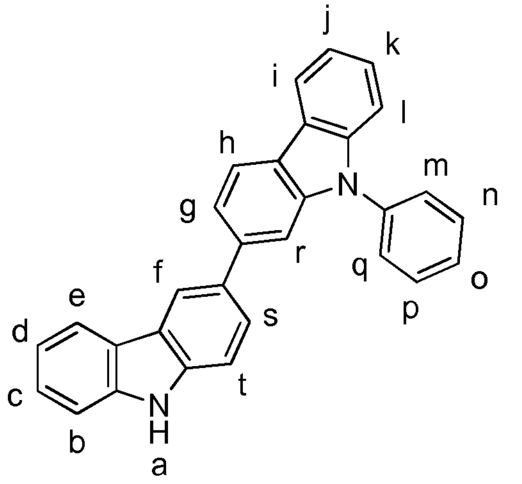

1

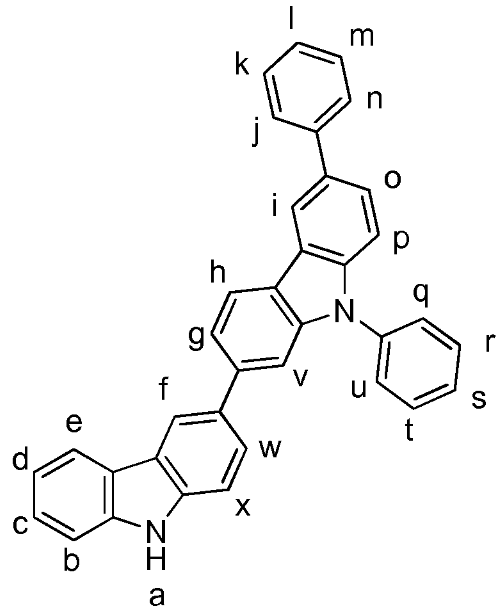

2

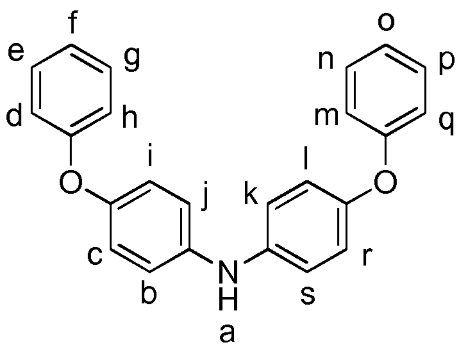

3

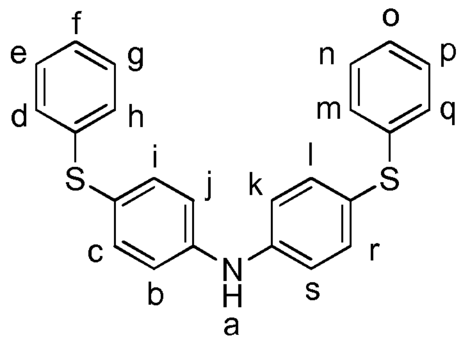

4

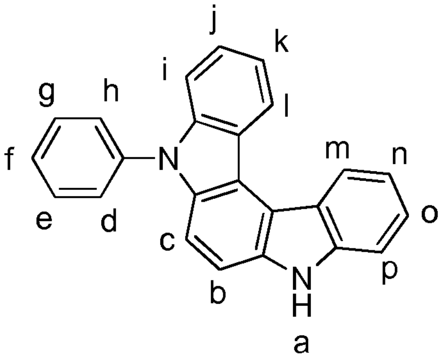

6

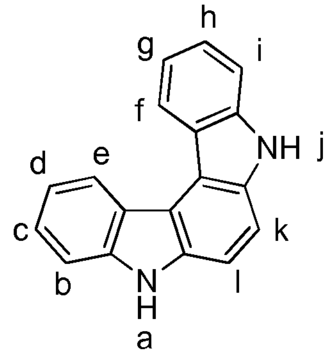

7

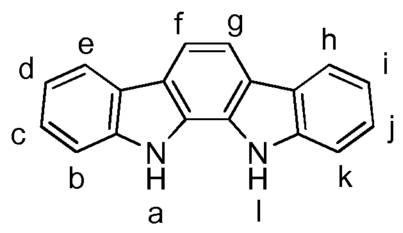

8

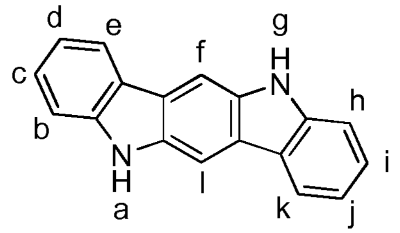

10

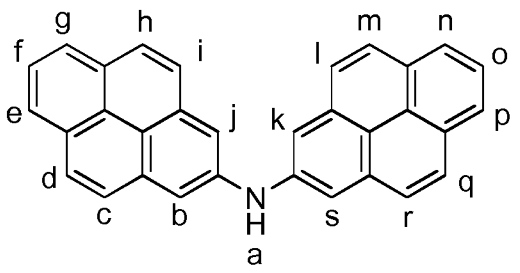

11

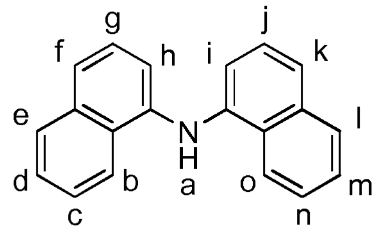

12

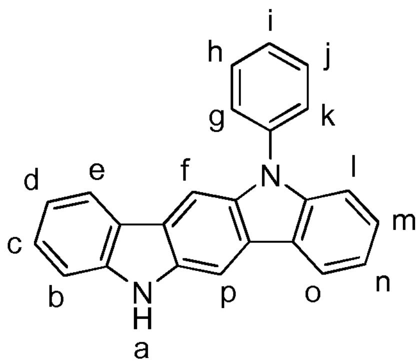

24

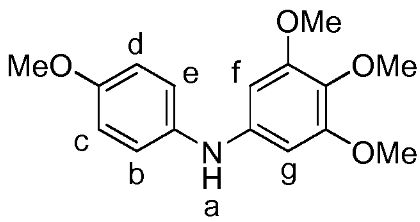

26

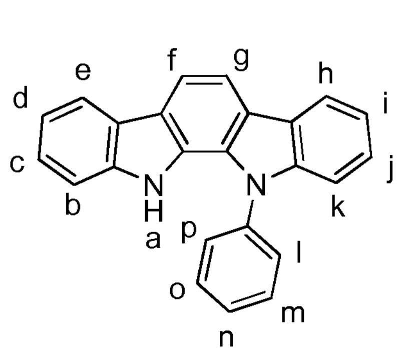

21

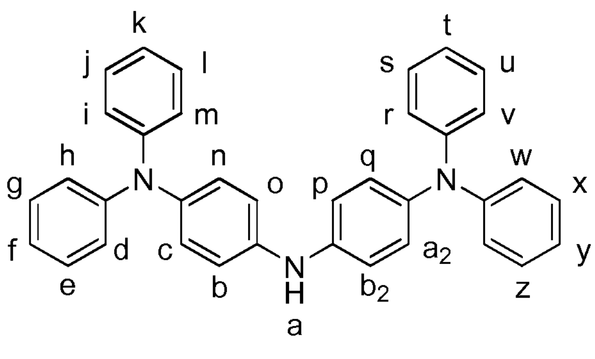

22

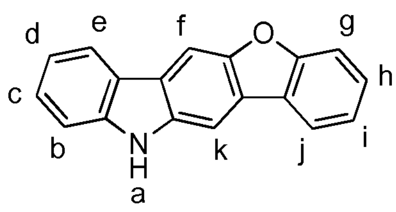
30

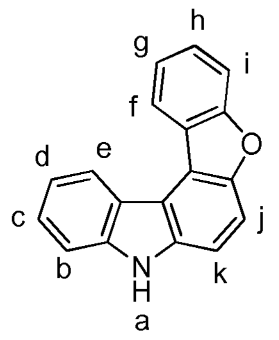
31

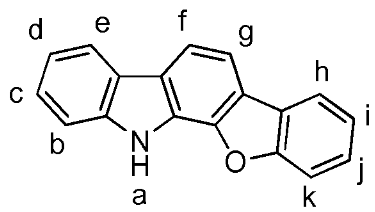
32

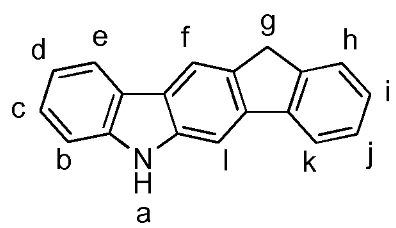
33

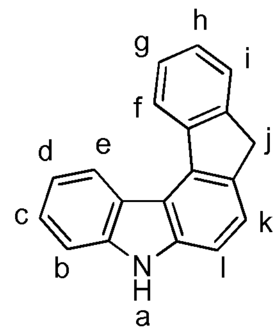
34

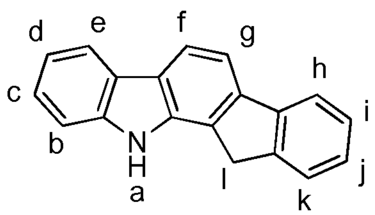
35

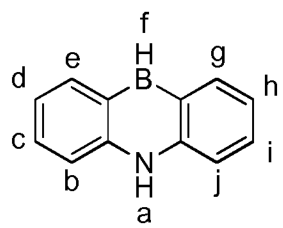
38

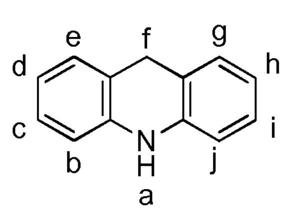
39

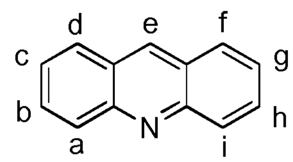
40

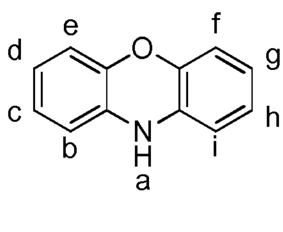
41

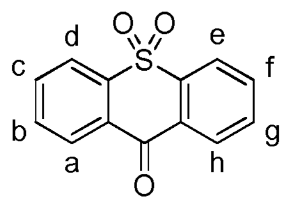
43

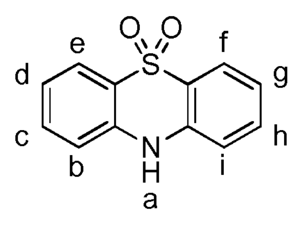
44

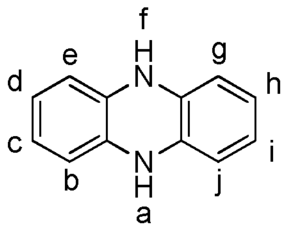

49

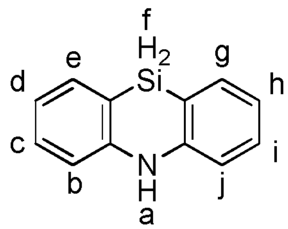

53

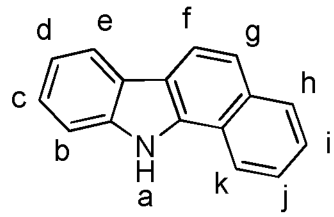

54

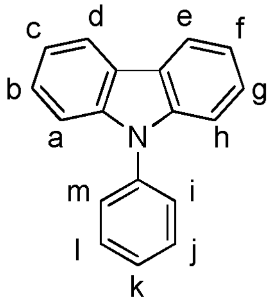

55

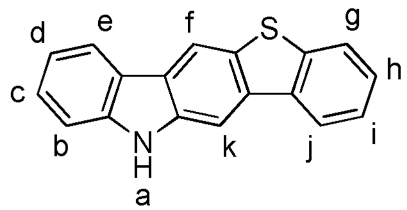

58

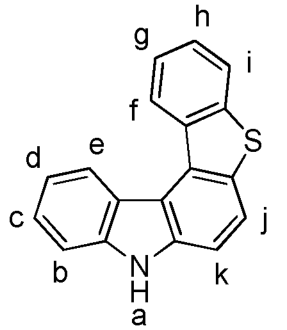

59

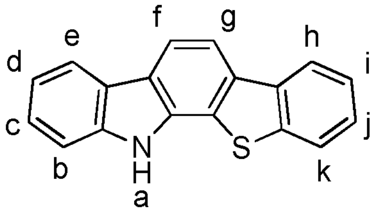

60

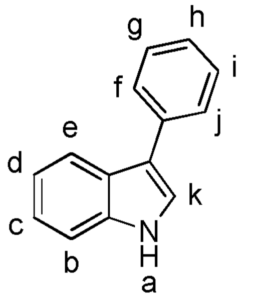

63

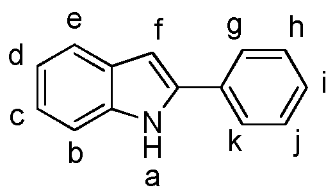

64

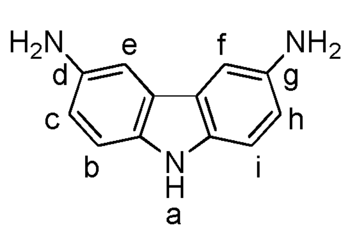

66

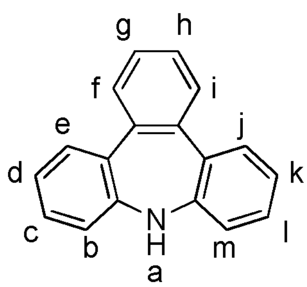

71

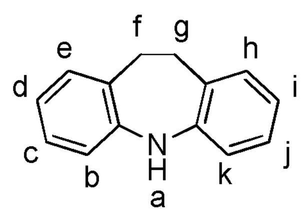

72

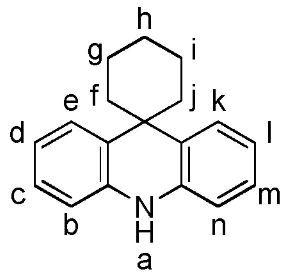

73

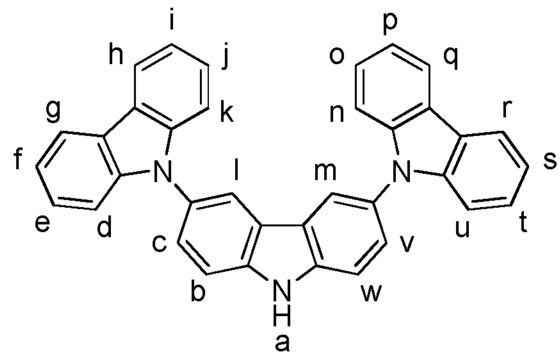

74

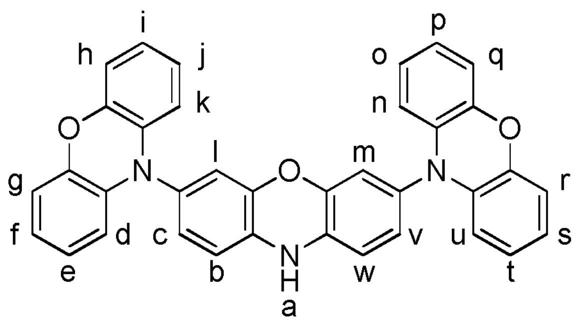

75

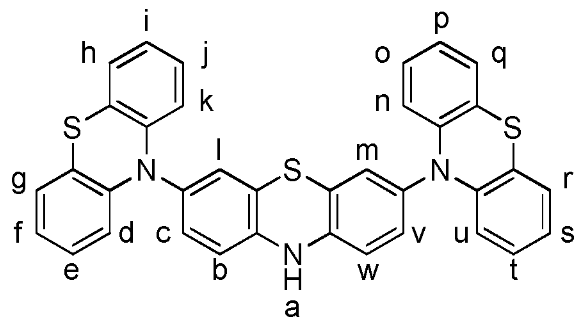

76

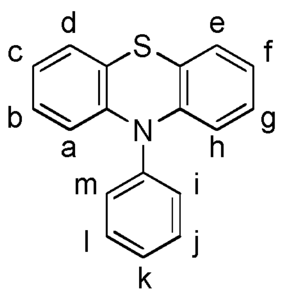

77

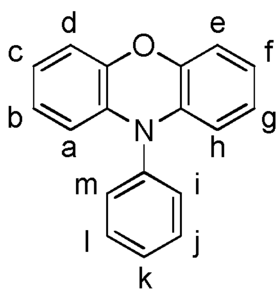

78

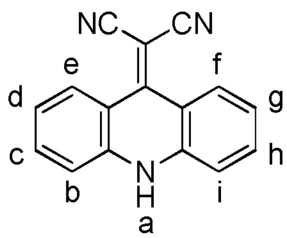

82

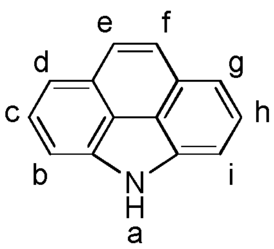

88

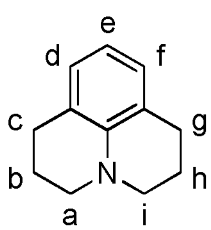

91

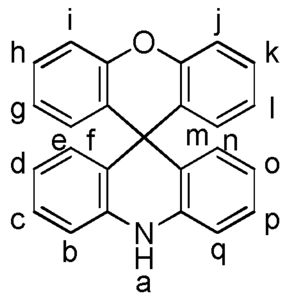

94

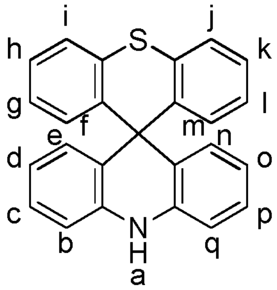

95

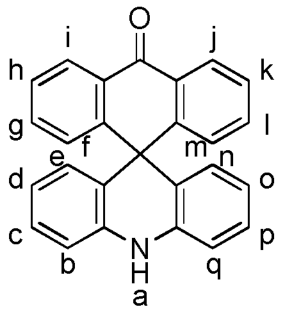

96

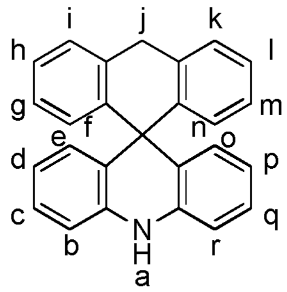

97

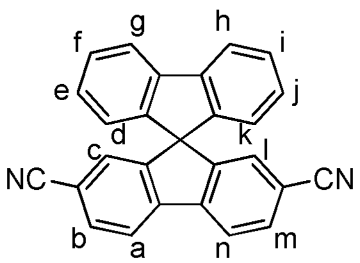

101

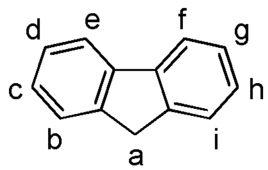

106

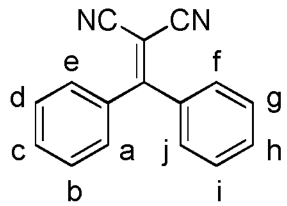

109

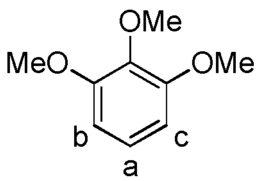

120

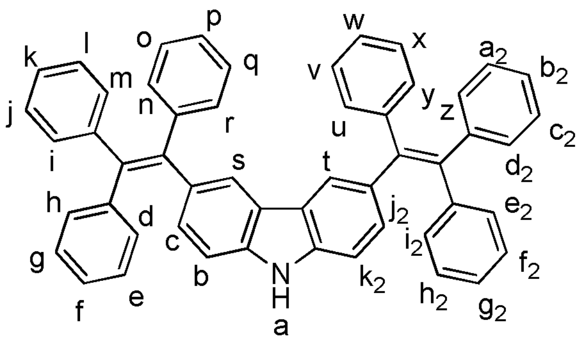

124

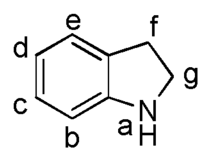

128

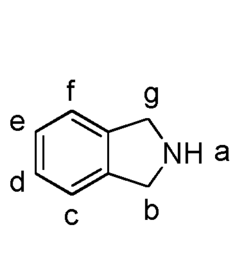

129

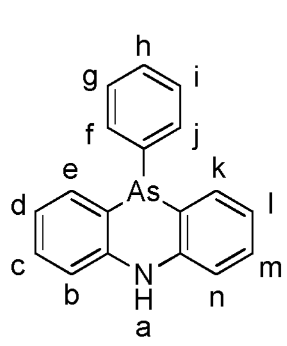

149

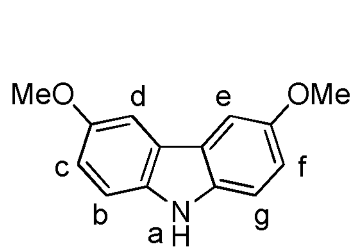

199

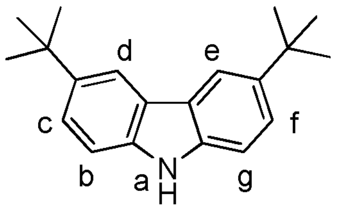

200

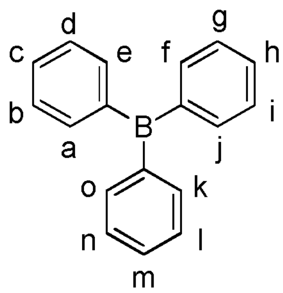

213

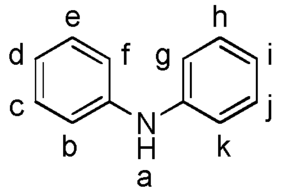

223

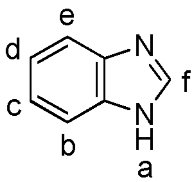

239

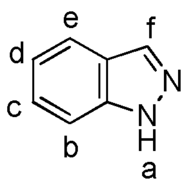

240

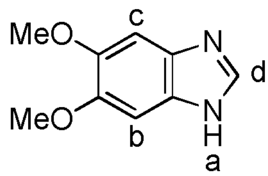

242

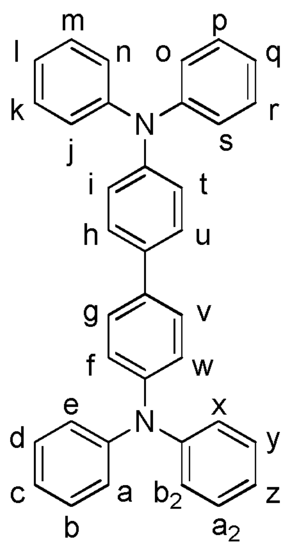

278

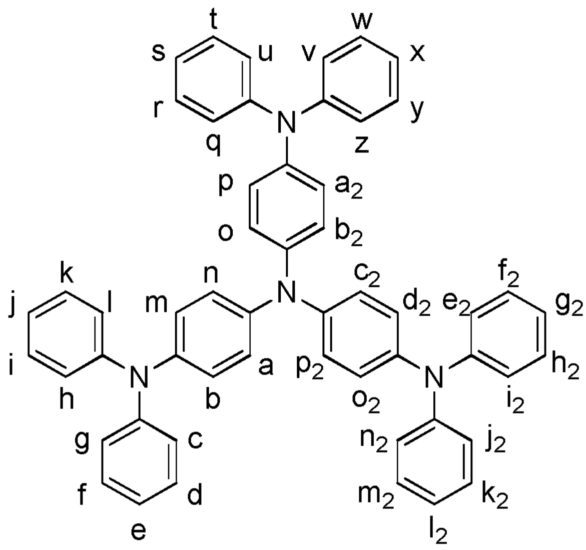

281

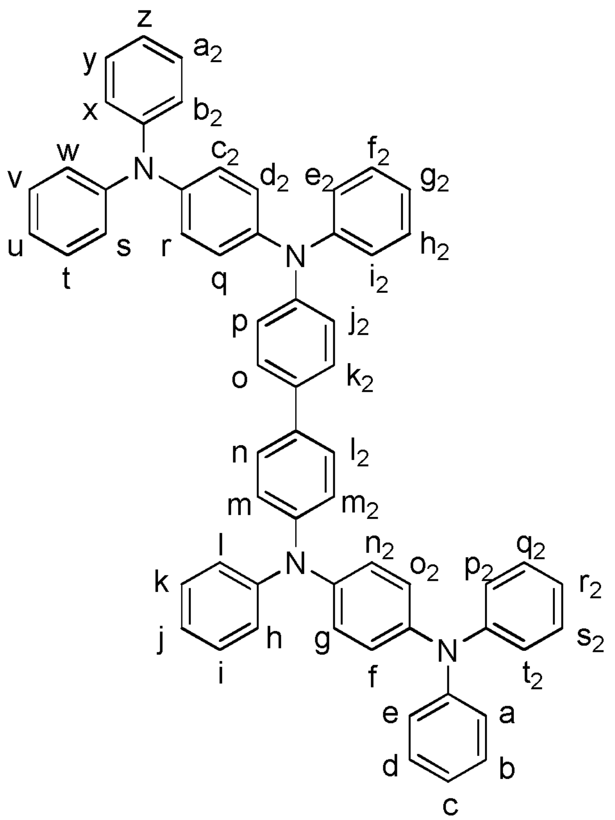

283

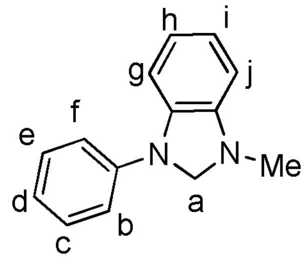

314

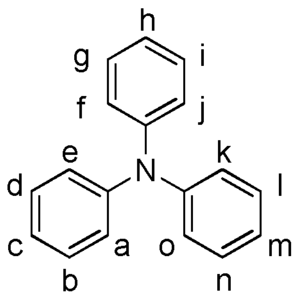

317

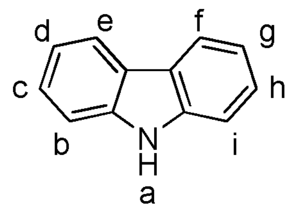

328

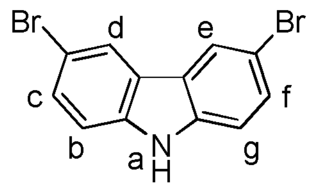

330

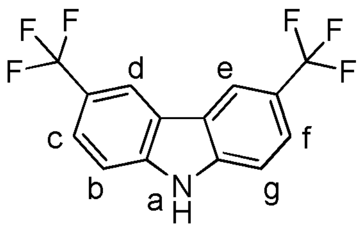

331

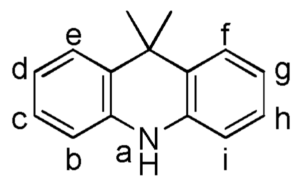

337

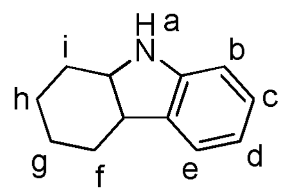

339

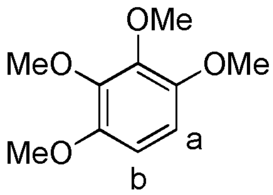

344

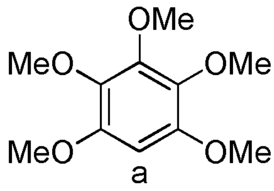

345

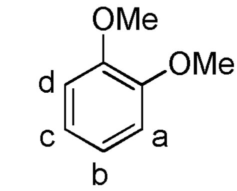

346

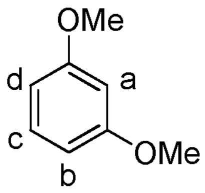

347

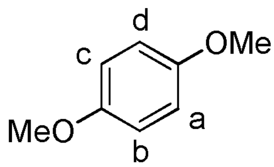

348

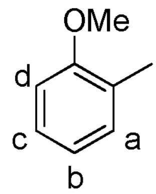

349

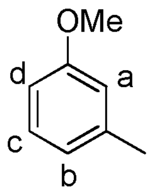

350

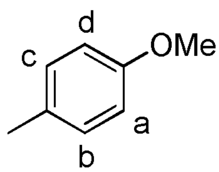

351

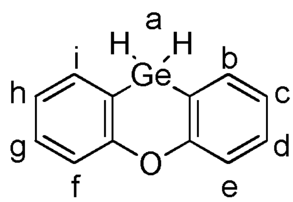

352

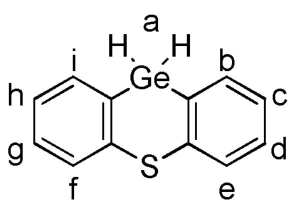

353

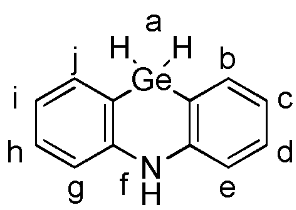

354

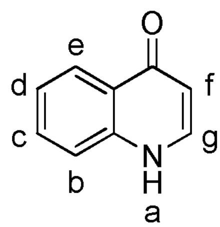

360

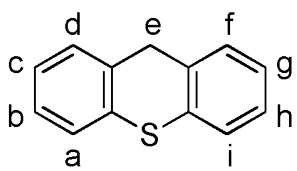

366

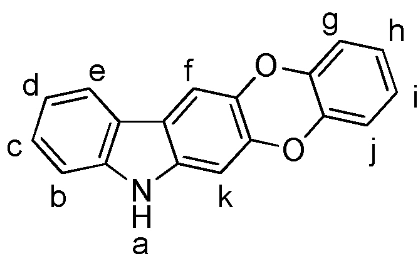

377

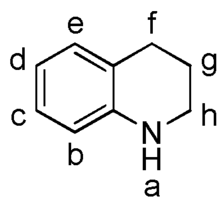

383

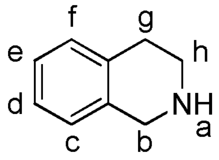

384

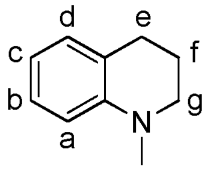

387

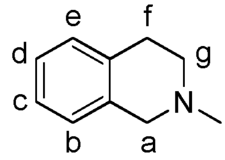

388

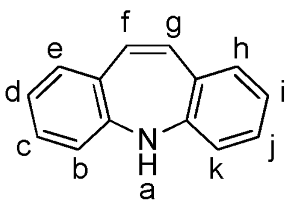

400

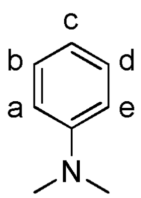

404

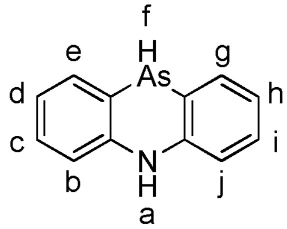

405

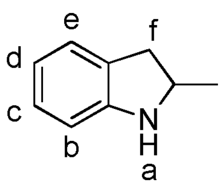

406

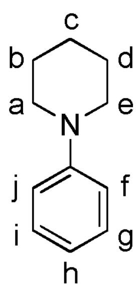

407

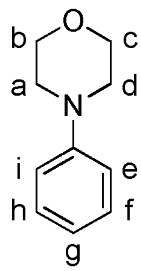

408

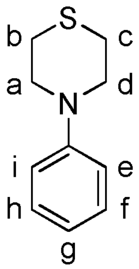

409

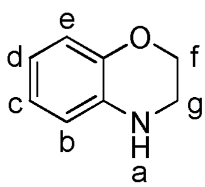

410

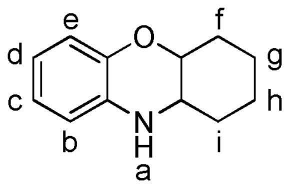

411

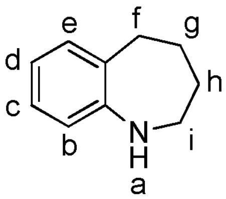

412

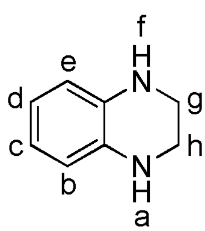

413

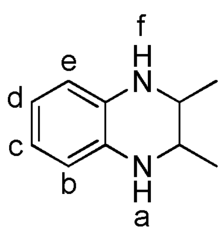

414

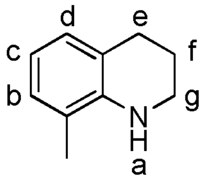

417

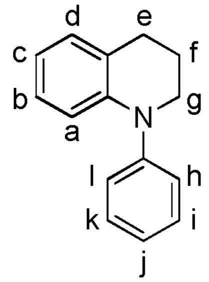

418

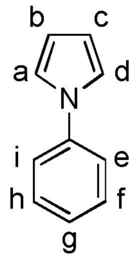

421

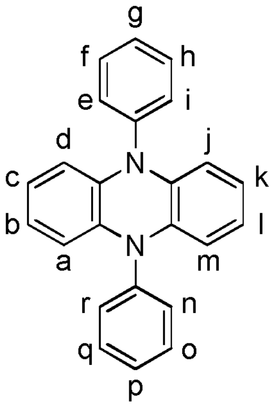

425

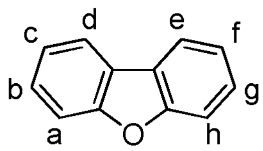

432

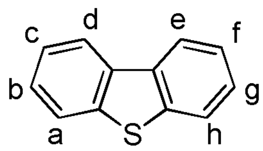

433

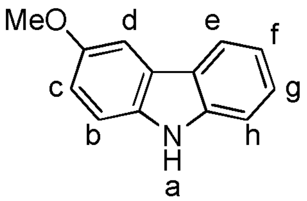

436

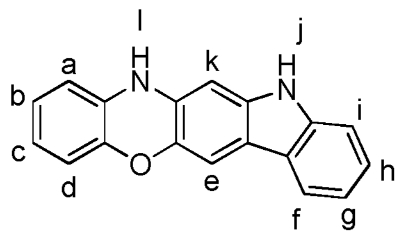

477

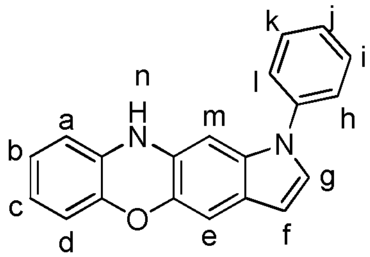

478

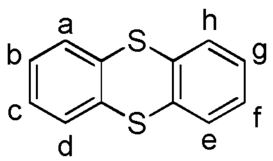

480

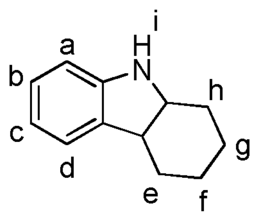

481

In Tabelle 1a und 1b sind mögliche Anknüpfungspositionen der chemischen Einheit AF zur Verknüpfung mit einem Separator S des erfindungsgemäßen Moleküls mit Kleinbuchstaben a bis z bezeichnet. Jede aromatische C-H und N-H Bindung ist optional mit einem löslichkeitsvermittelnden und/oder einem die Polymerisierbarkeit ermöglichenden Rest R substituiert.

**Tabelle 2:** Beispiele für erfindungsgemäße organische Moleküle Die Benennung von AF1 und AF2 entspricht der in Tabelle 1a und 1b, die Benennung des Separators S, der in dieser Tabelle als Spacer bezeichnet wird, entspricht der in Formel 3.

**Beispiele**

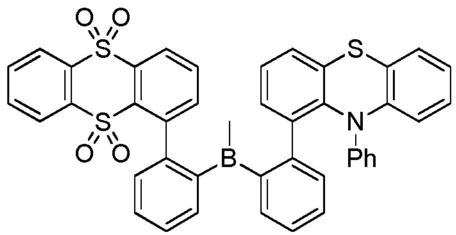

Spacer 3-1 AF1/AF2=77/46

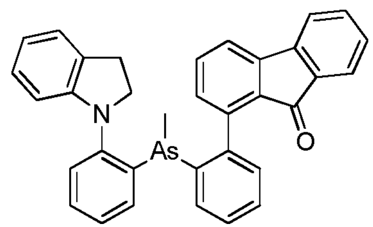

Spacer 3-17 AF1/AF2=128/110

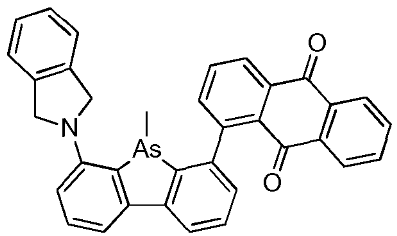

Spacer 3-18 AF1/AF2=129/36

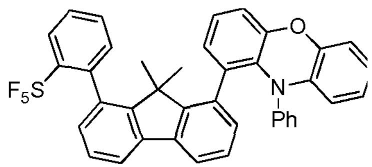

Spacer 3-4 AF1/AF2=78/184

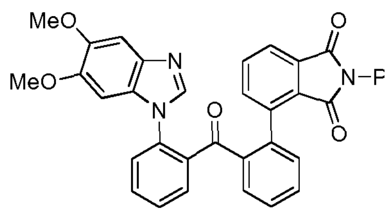

Spacer 3-5 AF1/AF2=242/237

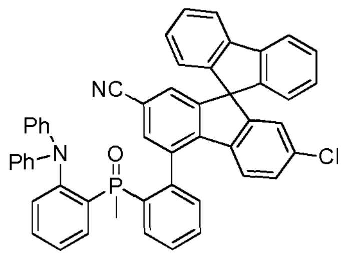

Spacer 3-13 AF1/AF2=223/101

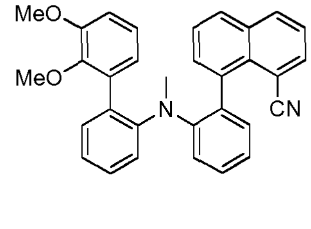

Spacer 3-9 AF1/AF2=346/244

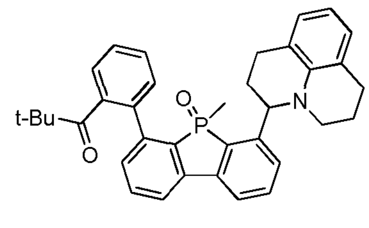

Spacer 3-14 AF1/AF2=91/193

(fortgesetzt)

**Beispiele**

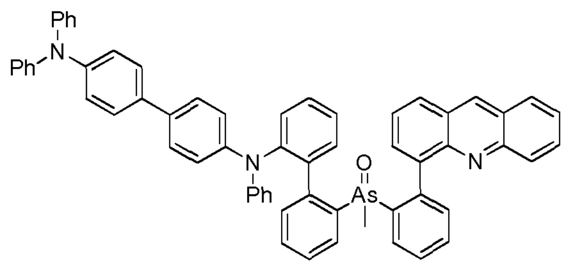

Spacer 3-19 AF1/AF2=278/40

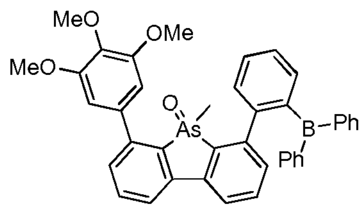

Spacer 3-20 AF1/AF2=120/213

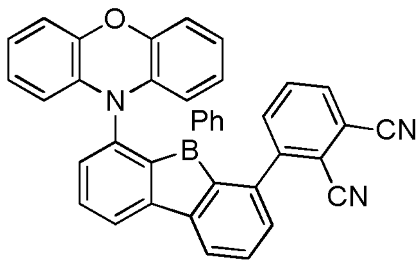

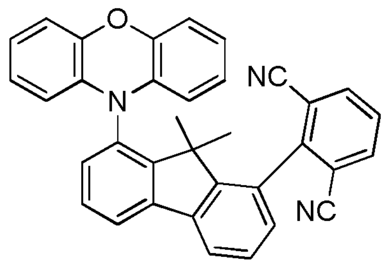

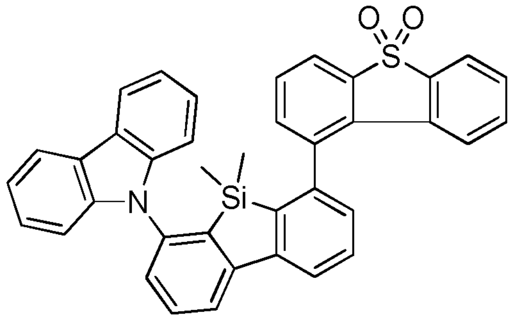

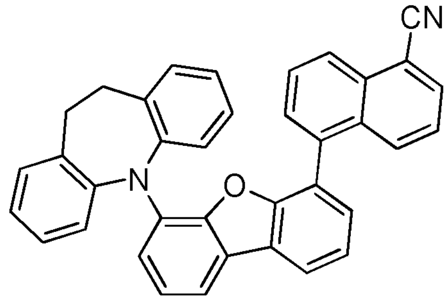

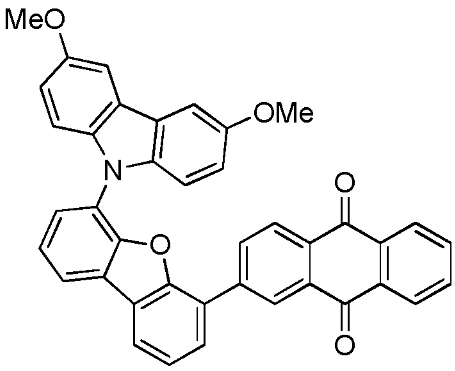

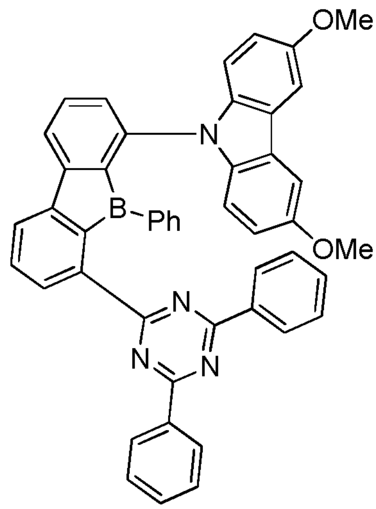

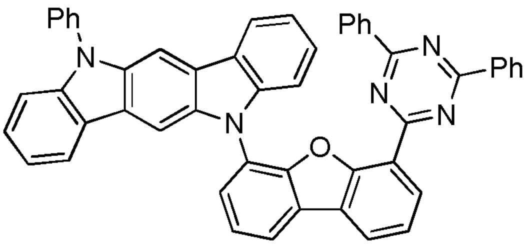

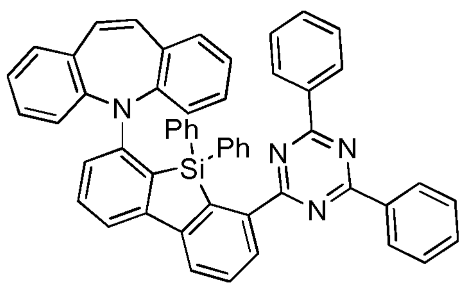

Durch Kombination der oben definierten Paare aus chemischen Einheiten AF1 und AF2 und der Festlegung der Verknüpfung durch einen Separator S, der insbesondere von einer Struktur der Formel 3 ist, ergeben sich entsprechend die beispielhaften erfindungsgemäßen Moleküle der Tabelle 2. Weitere organische Moleküle können durch Kombination der genannten Moleküleinheiten analog erhalten werden.

Hierdurch werden Verbindungen erhalten, die sich für den Einsatz in OLEDs eignen, beispielsweise als Emitter, Host- und/oder Matrixmaterial oder als Lochtransport-/Elektronenblockiermaterial. oder als Elektronentransport- bzw. Lochblockiermaterial Die Verwendung dieser Verbindungen führen zu guten Eigenschaften der organischen Elektrolumineszenzvorrichtung, insbesondere hinsichtlich der Lebensdauer, der Effizienz und der Betriebsspannung. Entsprechende Verbindungen, sowie organische Elektrolumineszenzvorrichtungen, welche derartige Verbindungen enthalten, sind daher der Gegenstand der vorliegenden Erfindung. Die überraschenden Effekte werden durch eine spezielle Anordnung ("face-to-face", d.h. einander gegenüberliegende Anordnung von Gruppen) der Donoren und Akzeptoren über den Separator in Verbindungen der oben aufgeführten Formeln erreicht. Durch diese relativ wohldefinierte (hochgeordnete) Parallelausrichtung der Donor- und Akzeptoreinheiten (face-to-face Anordnung), in der eine gewisse Nahordnung der Einheiten vorliegt, aber dennoch eine Trennung der Grenzorbitale gewährleistet ist, kann das Überlappungsintegral gezielt gesteuert werden. Bedingt durch die geringen Abstände der Gruppen zueinander könnten zwischenmolekulare Wechselwirkungen, wie beispielsweise $\pi$-$\pi$-Wechselwirkung über den Raum für einen schnellen Charge-Transfer-Übergang mit ursächlich sein.

Beispiele erfindungsgemäßer organischer Moleküle sind in Tabelle 3 dargestellt, die sich durch Kombination der chemischen Einheiten AF1 und AF2, dem Separator S und der Festlegung der Verknüpfung ergeben. Weitere organische Moleküle können durch Kombination der genannten Moleküleinheiten erhalten werden. Die Benennung der Moleküle erfolgt nach dem Schema AF-S-AF, wobei hinsichtlich der Benennung der chemischen Einheit AF auf Tabelle 1a und 1b verweisen wird, da die in der Tabelle 1a und 1b verwendeten Nummern auch in Tabelle 3 verwendet werden.

In einer Ausführungsform werden an die chemisch substituierbaren Positionen der so erhaltenen organischen Moleküle weitere Reste R angefügt, um die Löslichkeit der Emitter zu steigern und/oder die Polymerisierbarkeit zu ermöglichen ohne dabei die elektronischen Eigenschaften des Moleküls signifikant zu verändern, sodass auch bei Verwendung von R ein Emitter vorliegt, wobei

jedes R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$,$-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2-$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch ein mono- oder polycyclisches, aliphatisches, aromatisches und/oder benzoannelliertes Ringsystem bilden. In einer Ausführungsform können zwei oder mehrere dieser Substituenten R miteinander ein Ringsystem mit insgesamt fünf oder sechs Ringgliedern bilden. In einer Ausführungsform ist das Ringsystem, das gebildet werden kann auf ein monozyklisches aliphatisches Ringsystem mit insgesamt fünf oder sechs Ringgliedern beschränkt.

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden.

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden.

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden.

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden.

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden.

$R^7$ ist bei jedem Auftreten unabhängig voneinander ausgewählt aus der Gruppe bestehend aus Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, bzw. eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils

durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden.

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden.

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C=C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder-S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 8 und 9 erhalten werden, die als Polymere in der lichtemittierenden Schicht des optoelektronischen Bauelements Verwendung finden können.

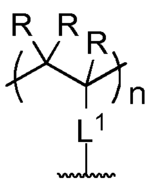

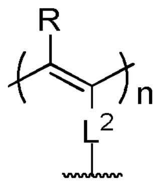

**Formel 8** **Formel 9**

In Formel 8 und 9 stehen L1 und L2 für gleiche oder verschiedene Linkergruppen, die 0 bis 20, insbesondere 1 bis 15, oder 2 bis 10 Kohlenstoffatome aufweisen, und wobei die gewellte Linie die Position kennzeichnet, über die die Linkergruppe an das organische Molekül der Formel 1 angebunden ist. R ist wie oben definiert. In einer Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, insbesondere eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form -C(=O)O- auf.

Vorteilhafte Ausführungsformen der Wiederholungseinheiten sind Strukturen der Formeln 10 bis 15:

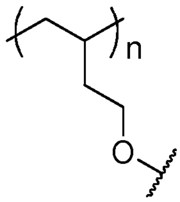
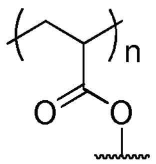
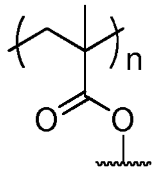
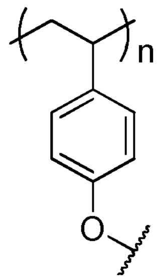
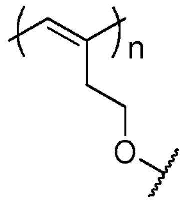
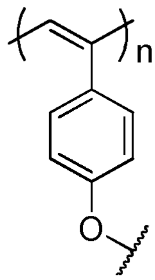

**Formel 10** **Formel 11** **Formel 12** **Formel 13** **Formel 14** **Formel 15**

Zur Herstellung der Polymere, die die Wiederholungseinheiten gemäß Formel 10 bis 15 aufweisen, werden die polymerisierbaren funktionellen Einheiten über eine Linkergruppe der Formeln 16 bis 21, die eine Hydroxyleinheit aufweisen, an das organische Molekül der Formel 1 angebunden und die daraus resultierenden Verbindungen mit sich selbst homopolymerisiert oder mit anderen geeigneten Monomeren copolymerisiert.

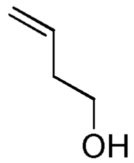
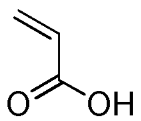
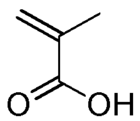
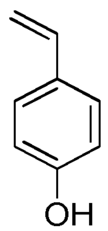
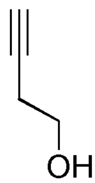
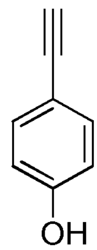

**Formel 16** **Formel 17** **Formel 18** **Formel 19** **Formel 20** **Formel 21**

Polymere, die eine Einheit gemäß Formel 8 oder Formel 9 aufweisen, können entweder ausschließlich Wiederholungseinheiten mit einer Struktur der allgemeinen Formel 8 oder 9, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche

- einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweisen und/oder
- eine Emissionslebensdauer von höchstens 50 $\mu$s aufweisen.

Eine weitere Ausführungsform der Erfindung betrifft die Verwendung eines Separators S in Form einer neutralen chemischen Einheit zur Bereitstellung eins erfindungsgemäßen organischen Moleküls, wobei eine erste chemische Einheit AF1, aufweisend ein konjugiertes System, insbesondere mindestens sechs in Konjugation stehende $\pi$-Elektronen und eine zweite chemische Einheit AF2, aufweisend ein konjugiertes System, insbesondere mindestens sechs in Konjugation stehende $\pi$-Elektronen, kovalent über den Separator S derart verknüpft wird, dass der Separator S eine direkte elektronische Wechselwirkung über konjugierte Bindungen zwischen dem konjugierten System der ersten chemischen Einheit AF1 und dem konjugierten System der zweiten chemischen Einheit AF2 unterbindet.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:

- organischen lichtemittierenden Dioden (OLEDs),
- lichtemittierende elektrochemischen Zellen,
- OLED-Sensoren, insbesondere in nicht hermetisch nach außen abgeschirmten Gas- und Dampf-Sensoren,
- organischen Dioden,

- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischen lichtemittierenden Dioden (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des Bauelements wird das organische Molekül als Emissionsmaterial in einer Emissionsschicht eingesetzt wird, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül mindestens ein Hostmaterial auf, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform weist das optoelektronische Bauelement mindestens ein Hostmaterial aus einem Material gemäß Formel 1 auf.

In einer weiteren Ausführungsform des optoelektronischen Bauelements beinhaltet die lichtemittierende Schicht fluoreszente oder phosphoreszente Materialien, welche ausgewählt sein können aus Formel 1 oder Formel 2a oder Formel 2b.

In einer weiteren Ausführungsform des optoelektronischen Bauelements bilden ein organisches Molekül gemäß

Formel 1 und ein funktionelles Material, beispielsweise in Form eines weiteren Emitter-Materials, eines Host-Materials, oder eines weiteren organisches Moleküls, welches zur Bildung eines Exciplex mit dem Molekül gemäß Formel 1 befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

In einer weiteren Ausführungsform des optoelektronischen Bauelements ist die Emission durch thermisch aktivierte verzögerte Fluoreszenz (TADF) charakterisiert.

In einer weiteren Ausführungsform des optoelektronischen Bauelements werden organische Moleküle gemäß Formel 1 oder Formel 2a oder Formel 2b als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett ($T_1$)- und Singulett ($S_1$)-Energieniveaus des Hostmaterials energetisch höher liegen als die Triplett ($T_1$)- und Singulett ($S_1$)-Energieniveaus des organischen Moleküls, und wobei das organische Molekül Fluoreszenz oder thermisch aktivierte verzögerte Fluoreszenz (TADF) emittiert, und einen $\Delta E(S_1\text{-}T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform weist das Verfahren die Verarbeitung des organischen Moleküls mittels eines Vakuumverdampfungsverfahrens oder aus einer Lösung auf.

In einer Ausführungsform weist das Verfahren das Aufbringen des organischen Moleküls auf einen Träger auf, wobei das Aufbringen insbesondere nass-chemisch, mittels kolloidaler Suspension oder mittels Sublimation erfolgt.

In einer weiteren Ausführungsform des Verfahrens wird mindestens eine Schicht

- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.

Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (Down-Konversion), insbesondere in einem optoelektronischen Bauelement der hier beschriebenen Art.

In einem weiteren Aspekt betrifft die Erfindung eine Anwendung, in der mindestens ein Material aus einem organischen Molekül der Formel 1 oder Formel 2a oder Formel 2b, durch äußere energetische Anregung zum Leuchten angeregt wird. Die äußere Anregung kann elektronisch oder optisch oder radioaktiv sein.

## Beispiele

### Beispiel 1

Die Synthese von 2-Bromo-9-H-Thioxanthen-9-on ist von Wang in Adv. Mater. 2014, 26, 5198-5204 beschrieben worden.

Dibenzofuron wird zweifach ortholithiiert und mit Brom abgefangen (Schwartz, J. Am. Chem. Soc., 1992, 114 (27), 10775-10784). Es schließt sich eine Kupfer-katalysierte Kupplungsreaktion an (in Anlehnung an WO002011057706A2), um die Triphenylamineinheit aufzubauen. Die im Molekül verbliebene Bromfunktionalität wird in das Boronsäurederivat überführt ((Schwartz, J. Am. Chem. Soc., 1992, 114 (27), 10775-10784), um im letzten Schritt mit 2-Bromo-9-H-Thioxanthen-9-on zum gewünschten Produkt gekuppelt zu werden (in Anlehnung nach (Schwartz, J. Am. Chem. Soc., 1992, 114 (27), 10775-10784).

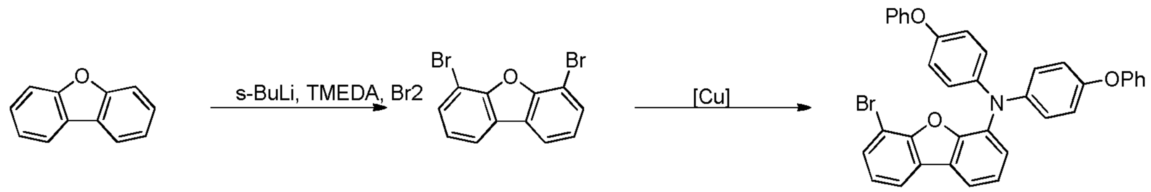


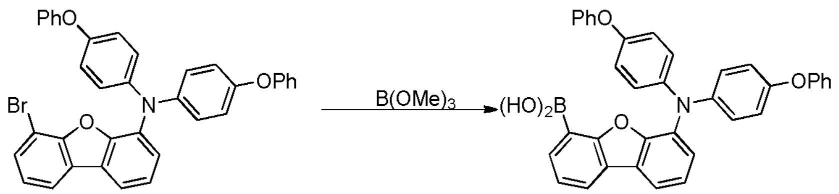


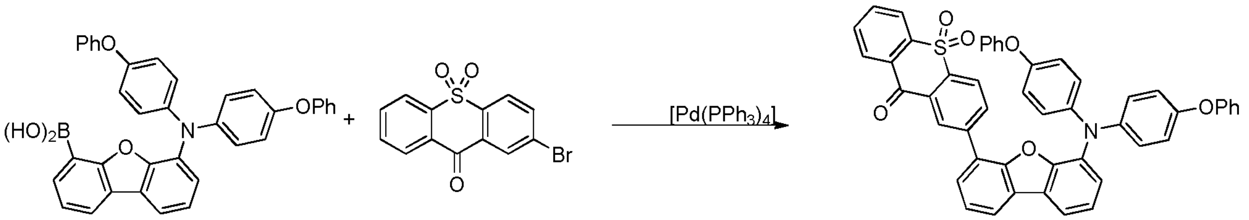


2,2-Dibromotriphenylamin wird aus Anilin und Dibromenzol unter Kupfervermittlung dargestellt (in Anlehnung an WO002011057706A2). Im nächsten Schritt erfolgt die Einführung des Bis(4-phenoxyphenyl)amin-Teils (in Anlehnung an WO002011057706A2). Die im Molekül verbliebene Bromfunktionalität wird in das Boronsäurederivat überführt ((Schwartz, J. Am. Chem. Soc., 1992, 114 (27), 10775-10784), um im letzten Schritt mit 2-Bromo-9-H-Thioxanthen-9-on zum gewünschten Produkt gekuppelt zu werden (in Anlehnung nach (Schwartz, J. Am. Chem. Soc., 1992, 114 (27), 10775-10784).

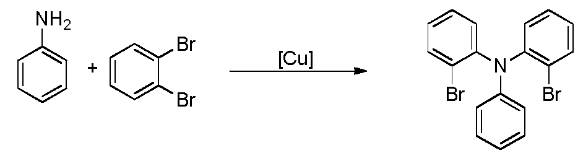


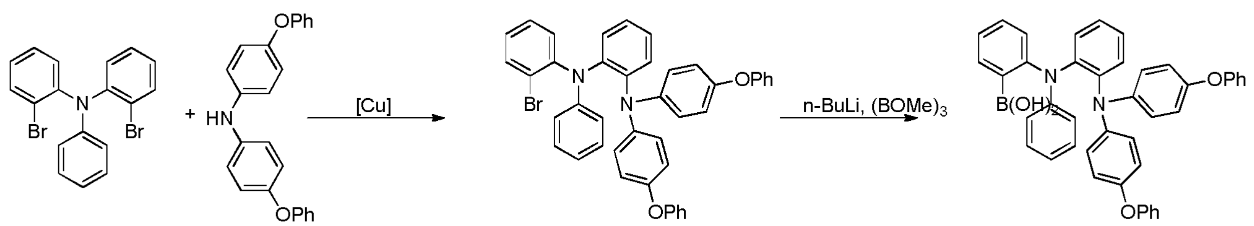


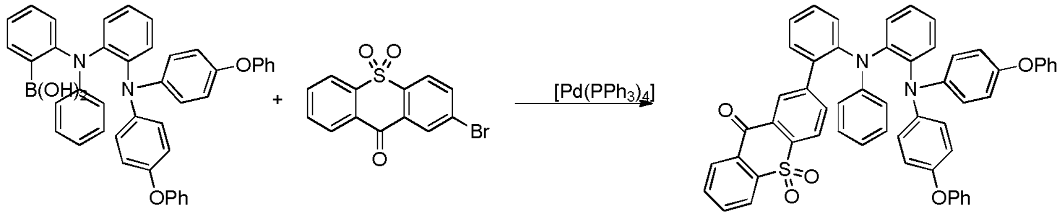

**Berechnungen nach der Dichtefuntionaltheorie**

**Variante 1 (BP86)**

Für die DFT-Rechnungen (Dichtefunktionaltheorie) wurde das BP86-Funktional (Becke, A. D. Phys. Rev. A1988, 38, 3098-3100; Perdew, J. P. Phys. Rev. B1986, 33, 8822-8827) und def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) verwendet. Zur numerischen Integration kam das m4-Grid zum Einsatz und die resolution-of-identity-Näherung (RI) (Häser, M.; Ahlrichs, R. J. Comput. Chem. 1989, 10, 104-111; Weigend, F.; Häser, M. Theor. Chem. Acc. 1997, 97, 331-340; Sierka, M.; Hogekamp, A.; Ahlrichs, R. J. Chem. Phys. 2003, 118, 9136-9148) wurde in allen Rechnungen verwendet. Die DFT-Rechnungen wurden mit dem Turbomole-Programmpaket (Version 6.5) (TURBOMOLE V6.4 2012, University of Karlsruhe/Forschungszentrum Karlsruhe GmbH, 1989-2007, TURBOMOLE GmbH, 2007; http://www.turbomole.com) durchgeführt.

**Variante 2 (TD-B3LYP)**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D. , J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D. , J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F. ; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, University of Karlsruhe und Forschungszentrum Karlsruhe GmbH, 1989-2007, TURBOMOLE GmbH, 2007; http://www.turbomole.com) durchgeführt.

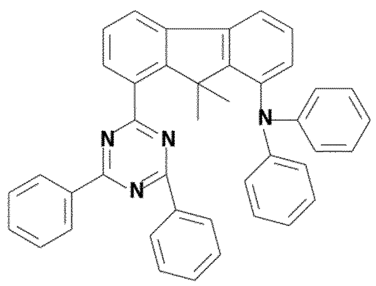

(0,000 eV)

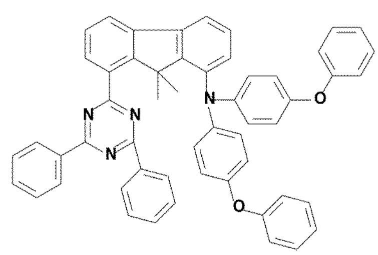

(0,020 eV)

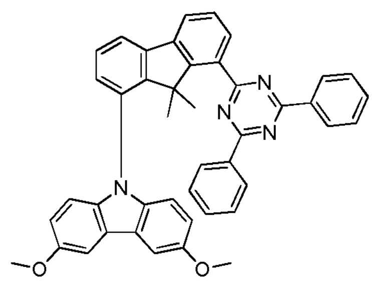

(0,000 eV)

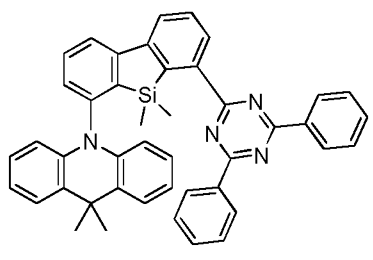

(0,256 eV)

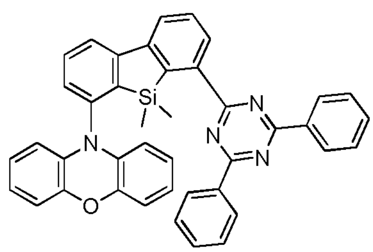

(0,001 eV)

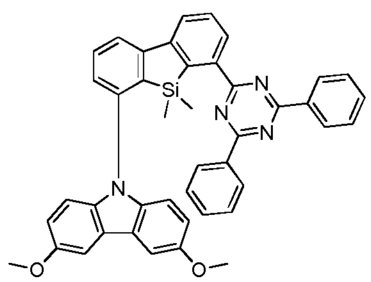

(0,000 eV)

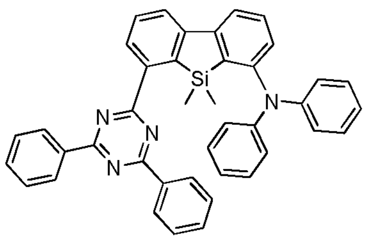

(0,069 eV)

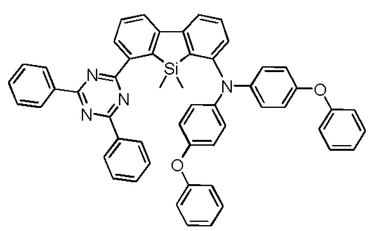

(0,036 eV)

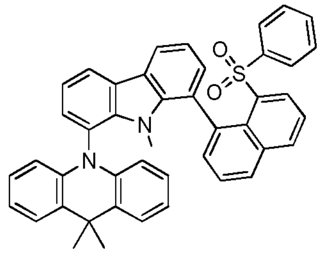

(0,030 eV)

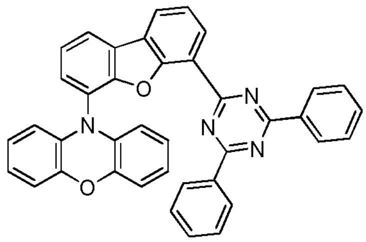

(0,005 eV)

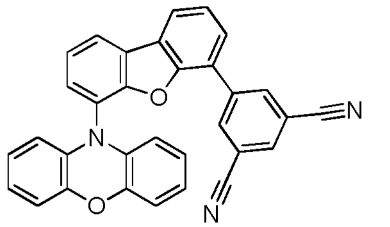

(0,003 eV)

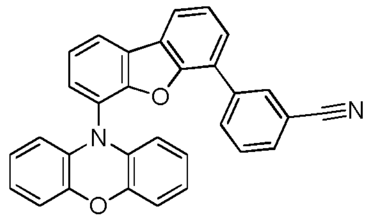

(0,023 eV)

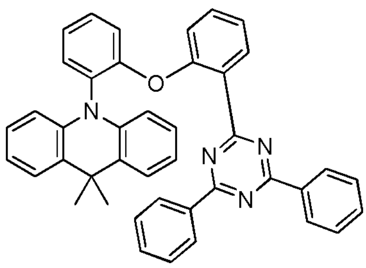

(0,003 eV)

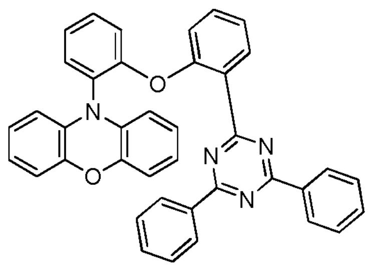

(0,009 eV)

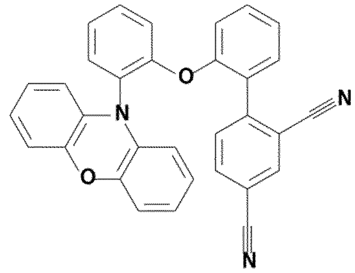

(0,003 eV)

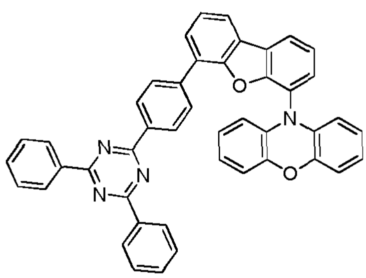

(0,004 eV)

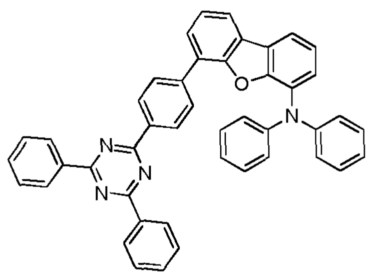

(0,093 eV)

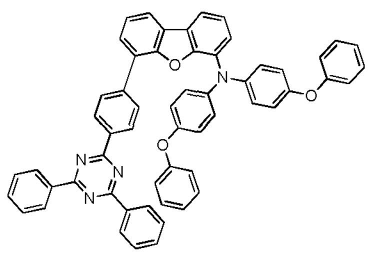

(0,072 eV)

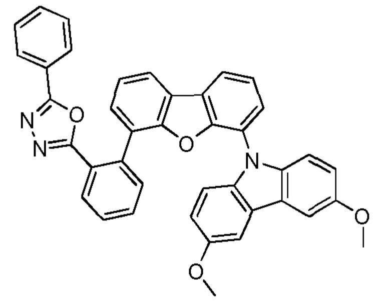

(0,002 eV)

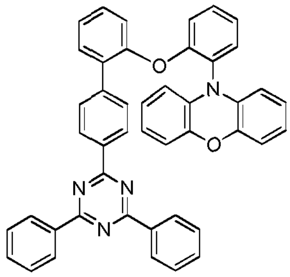

(0,001 eV)

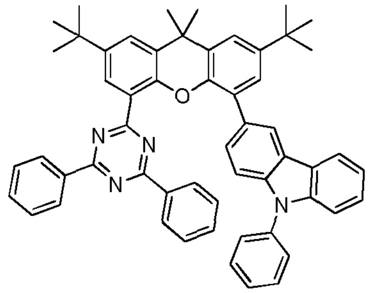

(0,149 eV)

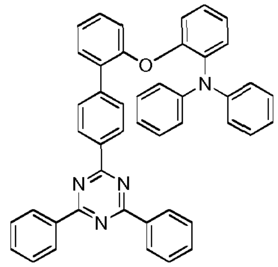

(0,010 eV)

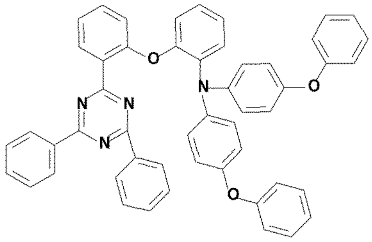

(0,009 eV)

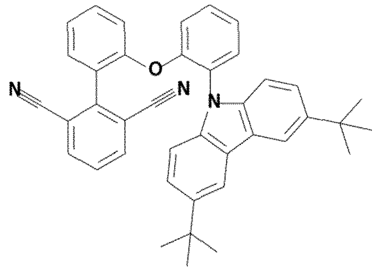

(0,002 eV)

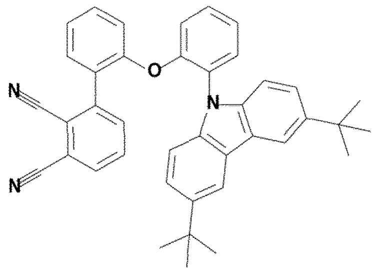

(0,001 eV)

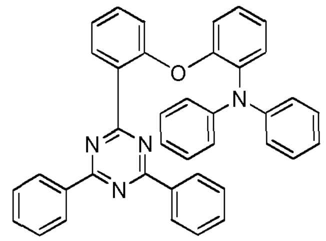

(0,016 eV)

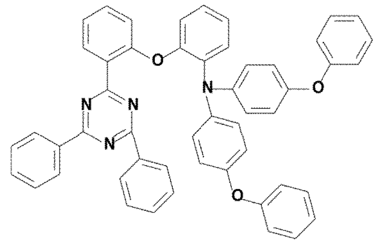

(0,008 eV)

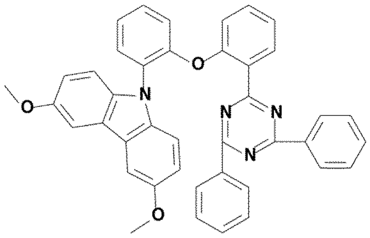

(0,005 eV)

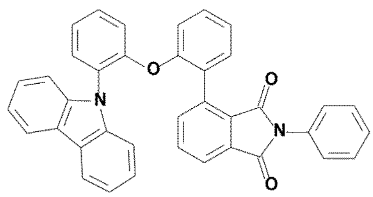

(0,003 eV)

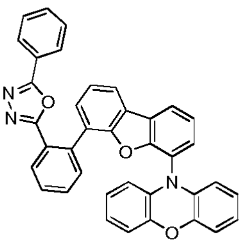

(0,001 eV)

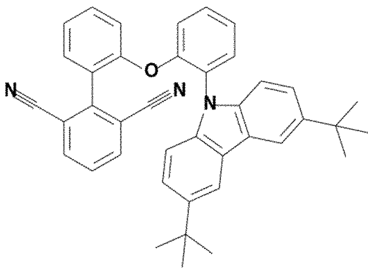

(0,002 eV)

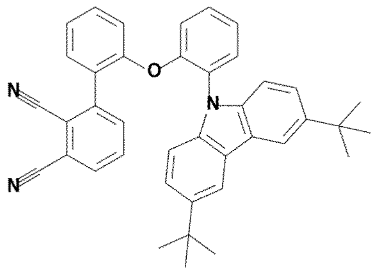

(0,018 eV)

**Figuren**

Es zeigen

Figur 1: Schematische Darstellung des Aufbaus einer organischen lichtemittierenden Diode (OLED).
Figur 2: Schematische Darstellung des Energieniveaudiagrammes (relative Energie in eV) eines erfindungsgemäßen Emitters (Lichtemission resultiert aus dem Übergang LUMO AF2 hin zu HOMO AF1).

**Patentansprüche**

**1.** Organisches Molekül, aufweisend eine Struktur der Formel 1

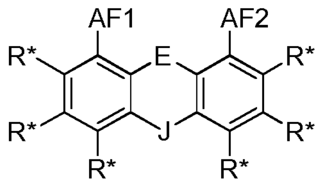

**Formel 1**

wobei

E = ausgewählt ist aus der Gruppe bestehend aus S, S(=O), O, C(=O), $CR^*_2$ $Si(R^4)2$, $Ge(R^4)_2$, $NR^2$, $PR^3$, $P(=O)R^7$, $P(=S)R^7$, $AsR^3$, $As(=O)R^7$, $As(=S)R^7$, $SbR^3$ und $BR^3$;
J = keine Bindung, sodass an den zwei Phenylringen an dieser Position dann je ein Rest R* vorhanden ist, oder eine Einfachbindung, die die beiden gezeigten Phenylringe miteinander verbindet;
R* ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, oder eine benachbarte $CH_2$-Gruppe durch $-Si(R^4)_2-$, $-Ge(R^4)_2-$, $-Sn(R^4)_2-$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, oder-S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R* auch miteinander ein Ringsystem mit insgesamt fünf oder sechs Ring-

gliedern bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren

Resten $R^9$ substituiert sein kann, wobei eine oder mehrere benachbarte $CH_2$-Gruppen durch -$R^9C=CR^9$-, -C=C-, oder eine benachbarte $CH_2$-Gruppe durch-Si($R^4$)$_2$-, -Ge($R^4$)$_2$-, -Sn($R^4$)$_2$, -C(=O)-,-C(=S)-, -C(=Se)-, -C=N-, -C(=O)O-, -C(=O)N($R^3$)-, -P(=O)($R^7$)-, -As(=O)($R^7$)-, -P(=S)($R^7$)-,-As(=S)($R^7$)-, -S(=O)-, -S(=O)$_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus Phenyl, Naphthyl, N($R^2$)$_2$, CN, $CF_3$, C(=O)O$R^3$, C(=O)N($R^3$)$_2$, Si($R^4$)$_3$, C(=O)$R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C=C-, oder eine benachbarte $CH_2$-Gruppe durch -Si($R^4$)$_2$-,-Ge($R^4$)$_2$-, -Sn($R^4$)$_2$, -C(=O)-, -C(=S)-, -C(=Se)-, -C=N-, -C(=O)O-, -C(=O)N($R^3$)-, -P(=O)($R^7$)-,-As(=O)($R^7$)-, -P(=S)($R^7$)-, -As(=S)($R^7$)-, -S(=O)-, -S(=O)$_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, N($R^2$)$_2$, CN, $CF_3$, $NO_2$, OH, COOH, C(=O)O$R^3$, C(=O)N($R^3$)$_2$, Si($R^4$)$_3$, B(O$R^5$)$_2$, C(=O)$R^3$, P(=O)($R^7$)$_2$, P(=S)($R^7$)$_2$, As(=O)($R^7$)$_2$, P(=S)($R^7$)$_2$, S(=O)$R^3$, S(=O)$_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch -$R^3C=CR^3$-, -C=C-, oder eine benachbarte $CH_2$-Gruppe durch -Si($R^4$)$_2$-, -Ge($R^4$)$_2$-, -Sn($R^4$)$_2$, -C(=O)-, -C(=S)-, -C(=Se)-,-C=N-, -C(=O)O-, -C(=O)N($R^3$)-, -P(=O)($R^7$)-, -As(=O)($R^7$)-, -P(=S)($R^7$)-, -As(=S)($R^7$)-, -S(=O)-, -S(=O)$_2$-, -$NR^2$-, -O-, oder-S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

AF1 und AF2 sind organische chemische Einheiten, wobei AF1 und AF2 unterschiedlich voneinander sind und wobei AF1 eine Struktur der Unterformel 1 aufweist

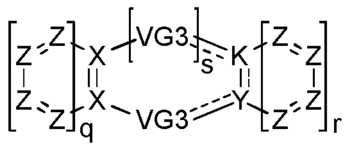

**Unterformel 1**

wobei gilt:

q ist 0, 1; r ist 0, 1; s ist 0, 1;
VG3 = verbrückende Gruppe, ist bei jedem Auftreten unabhängig voneinander ausgewählt aus den Gruppen bestehend aus N, O, S, CR**, C, eine Element-Element-Einfachbindung zwischen X und Y oder zwischen X und K, wobei nicht 2 Einheiten VG3 gleichzeitig eine Element-Element-Einfachbindung zwischen X und Y und zwischen X und K sind;
BR**, NR**, $GeR^{**}_2$, $AsR^{**}_2$, $SiR^{**}_2$, wobei nicht zwei Einheiten VG3 gleichzeitig gleich BR**, NR**, $GeR^{**}_2$, $AsR^{**}_2$, $SiR^{**}_2$ sind;

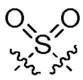, 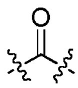, 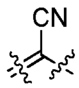, 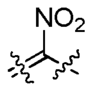, 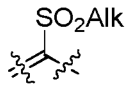, 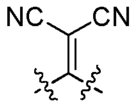, 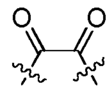, 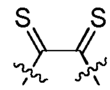, 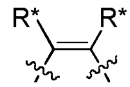,

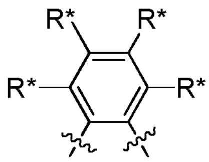,

wobei nicht zwei Einheiten VG3 gleichzeitig gleich

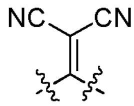, 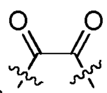,

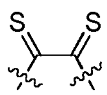, 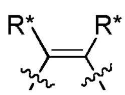, 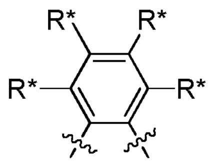

sind;
X ist bei jedem Auftreten unabhängig voneinander C; oder ist CR**, N wenn q = 0;
Y ist C; oder ist CR**, $CR^{**}_2$, N, NR**, O, S wenn r = 0;
K ist Y; oder ist R* wenn r = 0 und gleichzeitig s = 0;
Z ist bei jedem Auftreten unabhängig voneinander CR** oder N;
und wobei maximal 4 der Einheiten VG3, K, X, Y, Z gleichzeitig gleich N sind;
und wobei mindestens 1 der Einheiten VG3, K, X, Y, Z gleich einer von C-H verschiedenen Gruppe ist, die mindestens 1 Stickstoffatom oder ein Sauerstoffatom oder ein Schwefelatom enthält;
und wobei im Falle von r = 0 auch zwei benachbarte Gruppen Z miteinander oder eine Gruppe VG3 mit der ihr am nächsten gelegenen Gruppe Z oder X oder Y über die folgenden Einheiten verbrückt sein können:

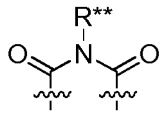 , 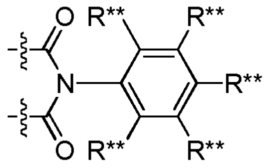 ;

wobei maximal drei dieser Einheiten in der Struktur gemäß Unterformel 1 enthalten sind;

und wobei AF2 eine Struktur der Unterformel 2 aufweist

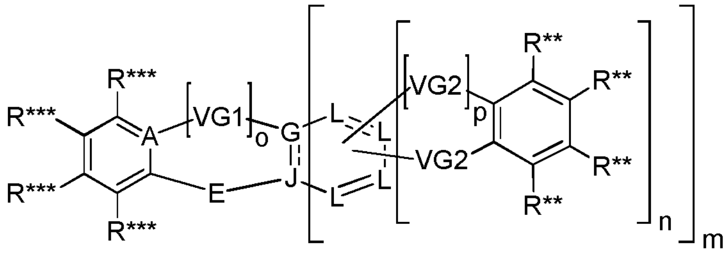

**Unterformel 2**

wobei gilt:

m ist 0 oder 1;
n ist 0 oder 1, wobei bei m = 0 immer auch n = 0 ist;
o ist 0 oder 1;
p ist 0 oder 1;
A ist CR*** wenn o = 0, ansonsten C;
VG1 = verbrückende Gruppe, ist ausgewählt aus der Gruppe bestehend aus

- NR**, $CR^{**}_2$, O, S und einer C-C-Einfachbindung; oder
- NR**, $CR^{**}_2$, O, S, einer C-C-Einfachbindung, BR**, AsR**, $SiR^{**}_2$, $GeR^{**}_2$,

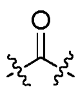 , 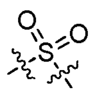 , 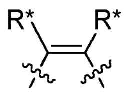 , 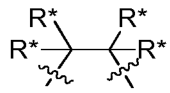 , 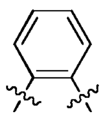 , 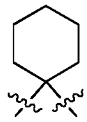 , 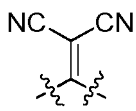 , 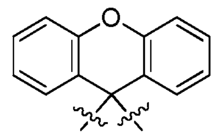 ,

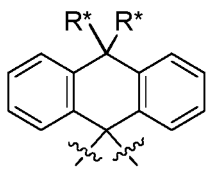 , 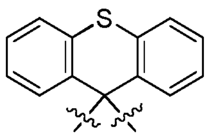 , und 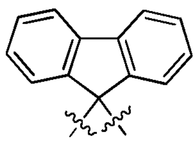 ,

wenn m = 1 und gleichzeitig n = 0;

VG2 = verbrückende Gruppe ist bei jedem Auftreten unabhängig voneinander ausgewählt aus der Gruppe bestehend aus $CR^{**}_2$, NR**, O, S und einer C-C-Einfachbindung, wobei nicht zwei Einheiten VG2 gleichzeitig gleich einer C-C-Einfachbindung sind;
E ist ausgewählt aus der der Gruppe bestehend aus NR**,

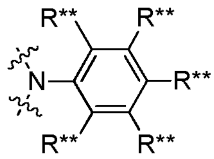

,

O und S;

G ist C wenn o = 1 und gleichzeitig m = 1; ist CR** wenn o = 0 und gleichzeitig m = 1; ist CR**, $CR^{**}_2$, wenn o = 1 und gleichzeitig m = 0; ist R* wenn o = 0 und gleichzeitig m = 0; ist CR**, $CR^{**}_2$, N, NR* wenn m = 0 und gleichzeitig VG1 eine C-C-Einfachbindung ist;

J ist C wenn m = 1; ist CR**, $CR^{**}_2$, NR** wenn m = 0;

L ist CR*** wenn n = 0; ist CR**, C (bei kovalenter Bindung zu VG2) wenn n = 1;

R*** ist R** oder ist ausgewählt aus den folgenden Einheiten, wobei maximal zwei der Reste R*** gleichzeitig gleich einer der folgenden Einheiten sind:

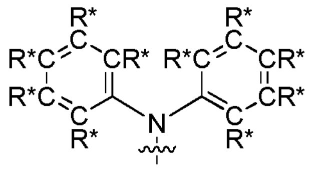

,

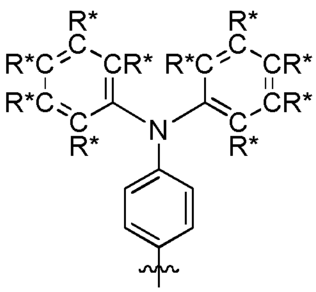

,

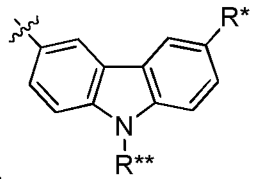

,

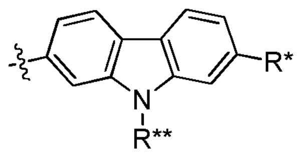

,

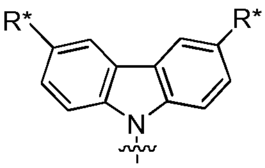

,

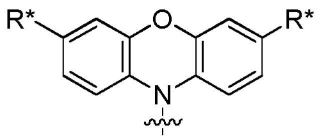

,

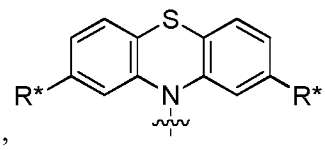

,

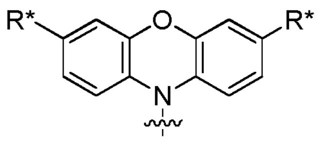

,

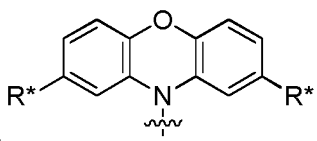

;

und wobei gilt

R** ist bei jedem Auftreten unabhängig voneinander ein Rest R* und/oder markiert eine Anknüpfungsstelle an einen Separator S, wobei genau ein R** eine Anknüpfungsstelle an einen Separator S ist;

wobei der Separator der Teil des organischen Moleküls gemäß Formel 1 ohne chemische Einheiten AF ist.

**2.** Organisches Molekül nach Anspruch 1, wobei das organische Molekül eine Struktur der Formel 2a oder 2b aufweist:

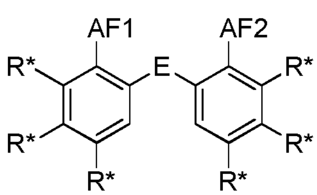

**Formel 2a**

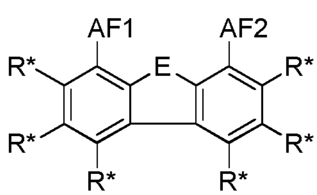

**Formel 2b**

wobei die in Anspruch 1 angegebenen Definitionen gelten.

**3.** Organisches Molekül nach Anspruch 1 oder 2, wobei das Molekül eine der nachfolgenden Strukturen aufweist und die in Anspruch 1 angegebenen Definitionen gelten:

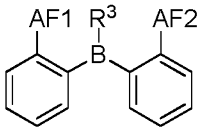
2-1

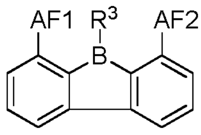
2-2

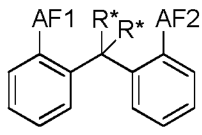
2-3

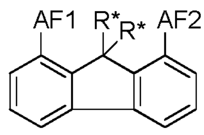
2-4

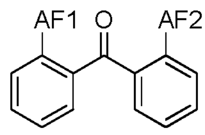
2-5

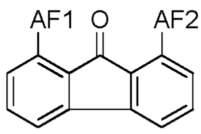
2-6

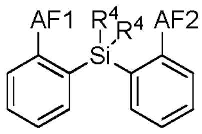
2-7

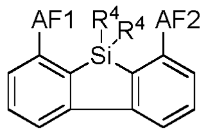
2-8

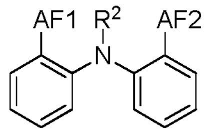
2-9

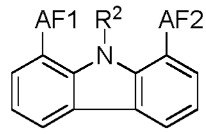
2-10

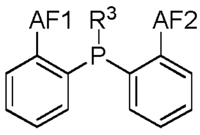
2-11

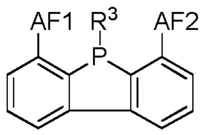
2-12

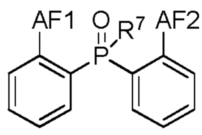
2-13

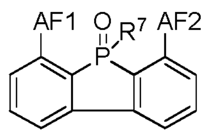
2-14

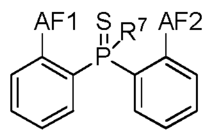
2-15

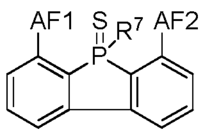
2-16

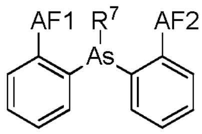
2-17

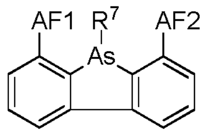
2-18

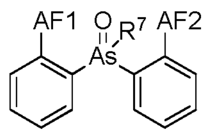
2-19

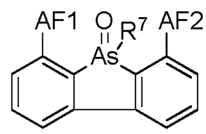
2-20

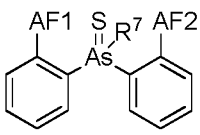
2-21

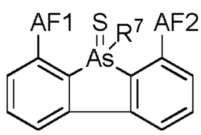
2-22

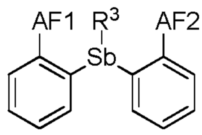
2-23

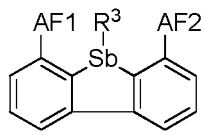
2-24

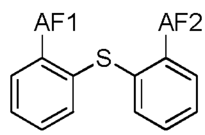
2-25

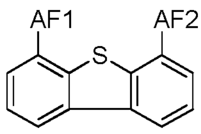
2-26

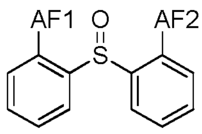
2-27

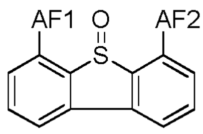
2-28

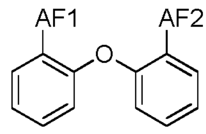
2-31

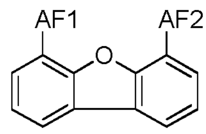
2-32

**4.** Organisches Molekül nach Anspruch 1 bis 3, wobei das organische Molekül eine Struktur der Formel 5a oder 5b aufweist:

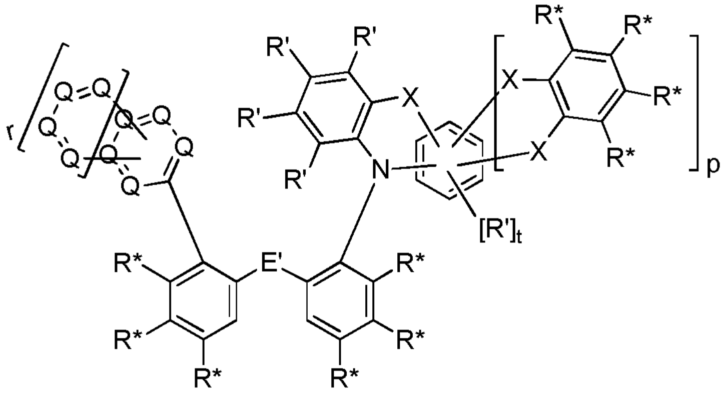

**Formel 5a**

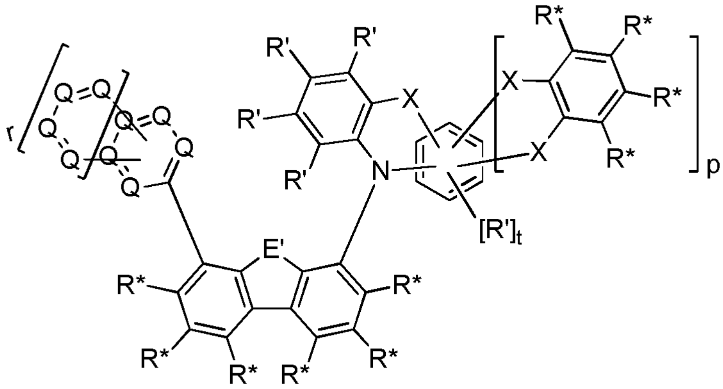

**Formel 5b**

wobei in Formel 5a/5b bedeutet:

p ist 0 oder 1;
t = 4 - 2p;
X ist $CR^*_2$, $NR^*$, Sauerstoff, Schwefel, eine direkte Bindung, wobei maximal zwei Platzhalter X gleichzeitig eine direkte Bindung sind, wobei diese nicht Bestandteil desselben Rings sind;
Q ist N, CR* oder für r = 1 die entsprechenden Anellierungspositionen C, wobei nicht zwei direkt benachbarte Einheiten Q gleichzeitig gleich N sind;
r = 0 oder 1,
E' ist S, O, C(=O), $CR^*_2$, $Si(R^4)_2$, $Ge(R^4)_2$, $NR^2$, $BR^3$;
R' ist R* oder ist ausgewählt aus den folgenden Einheiten, wobei maximal zwei der Reste R' gleichzeitig gleich einer der folgenden Einheiten sind:

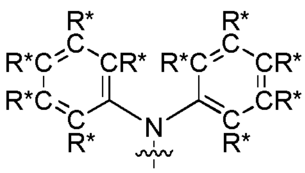 , 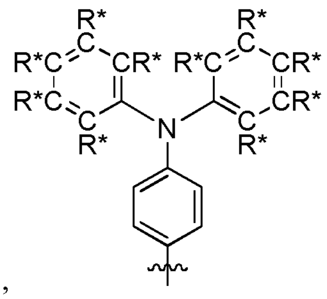 , 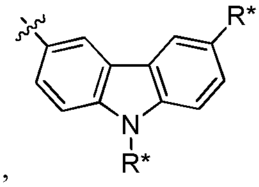 , 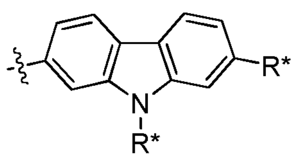 ,

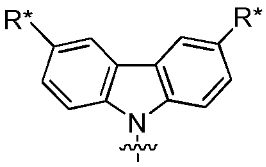 , 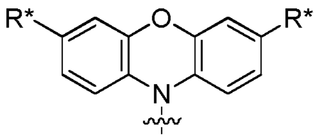 , 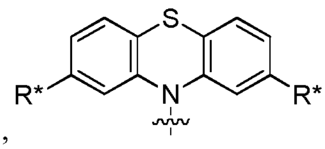 ,

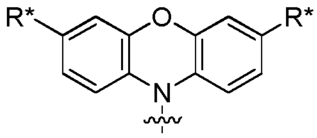 , 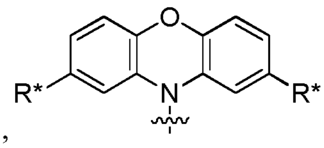 ;

und R* wie bei Anspruch 1 definiert ist.

**5.** Organisches Molekül nach Anspruch 1 bis 3, wobei das organische Molekül eine Struktur der Formel 6a oder 6b aufweist:

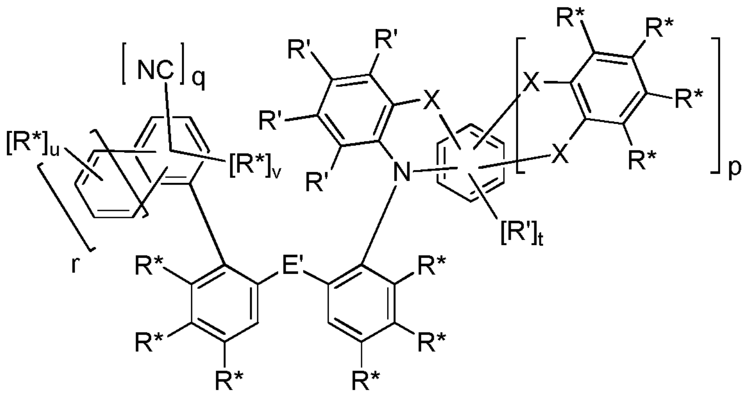

**Formel 6a**

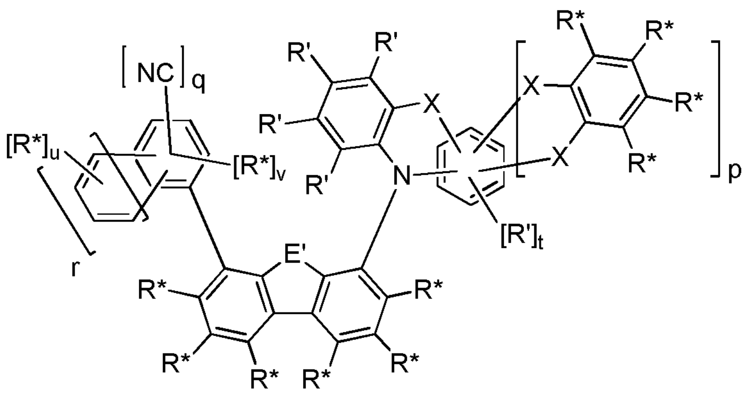

**Formel 6b**

wobei in Formel 6a/6b bedeutet:

p ist 0 oder 1;
t = 4 - 2p;
X ist $CR^*_2$, NR*, Sauerstoff, Schwefel, eine direkte Bindung, wobei maximal zwei Platzhalter X gleichzeitig eine direkte Bindung sind, wobei diese nicht Bestandteil desselben Rings sind;
r = 0 oder 1,
q = 1 bis 5, für r = 1 ist q = 1 bis 3,

u ist 1, 2, 3 oder 4

v ist 1, 2 oder 3; wobei gilt: u + v = 3 + 4r - q

E' ist S, O, C(=O), $CR^*_2$, $Si(R^4)_2$, $Ge(R^4)_2$, $NR^2$, $BR^3$;

R' ist R* oder ist ausgewählt aus den folgenden Einheiten, wobei maximal zwei der Reste R' gleichzeitig gleich einer der folgenden Einheiten sind:

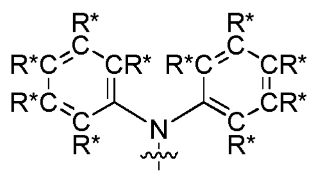
,
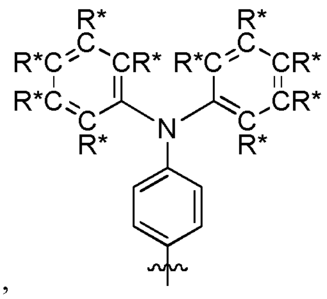
,
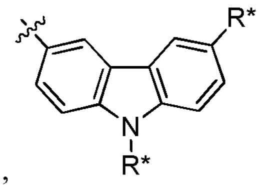
,
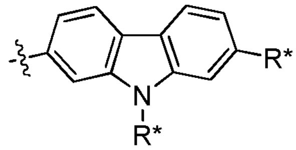
,

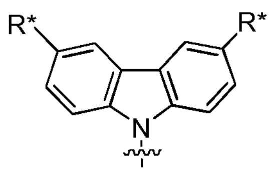
,
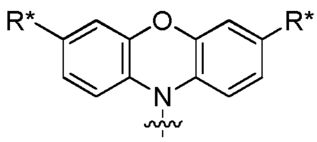
,
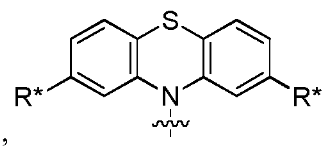
,

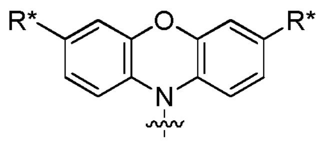
,
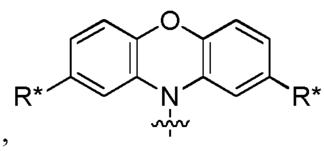
;

und R* wie bei Anspruch 1 definiert ist.

6. Organisches Molekül nach Anspruch 1 bis 3, wobei das organische Molekül eine Struktur der Formel 7a oder 7b aufweist:

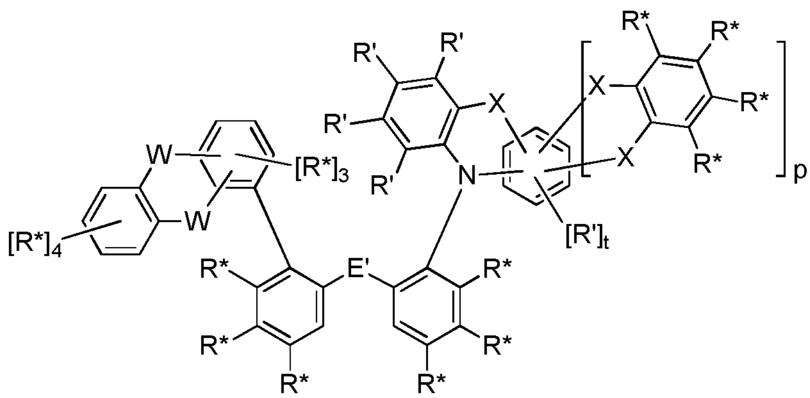

**Formel 7a**

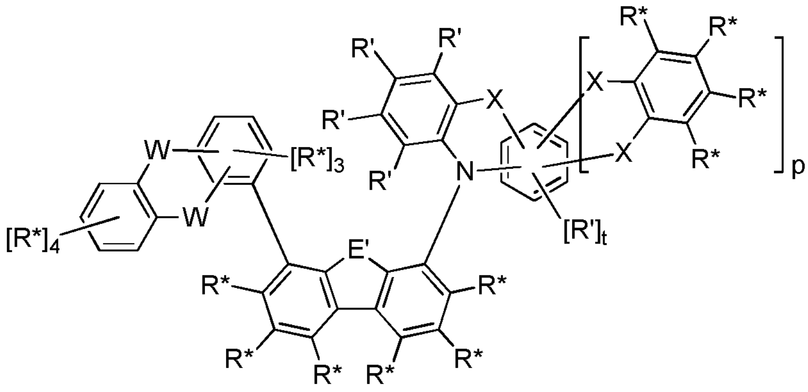

**Formel 7b**

wobei in Formel 7a/7b bedeutet:

p ist 0 oder 1;
t = 4 - 2p;
X ist $CR^*_2$, NR*, Sauerstoff, Schwefel, eine direkte Bindung, wobei maximal zwei Platzhalter X gleichzeitig eine direkte Bindung sind, wobei diese nicht Bestandteil desselben Rings sind;
W ist ausgewählt aus der Gruppe bestehend aus

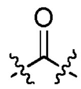, 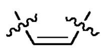, 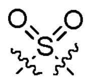,

eine Element-Element-Einfachbindung, wobei nicht zwei Einheiten W gleichzeitig eine Element-Element-Einfachbindung sind, NR*, wobei nicht zwei Einheiten W gleichzeitig gleich NR* sind;
E' ist ausgewählt aus der Gruppe bestehend aus S, O, C(=O), $CR^*_2$, $Si(R^4)_2$, $Ge(R^4)_2$, $NR^2$ und $BR^3$;
R' ist R* oder ist ausgewählt aus der Gruppe der folgenden Einheiten, wobei maximal zwei der Reste R' gleichzeitig gleich einer der folgenden Einheiten sind:

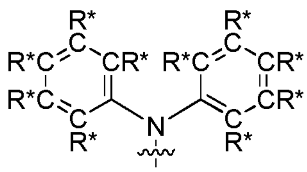, 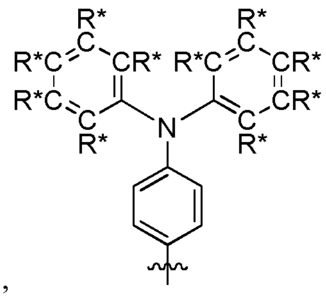, 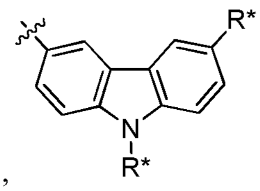, 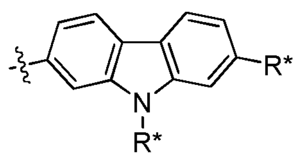,

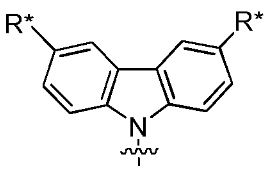, 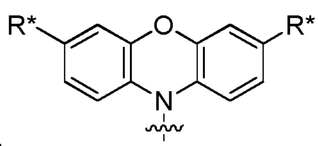, 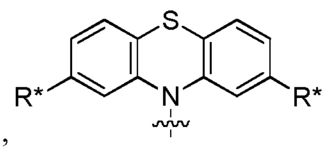,

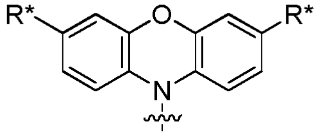, 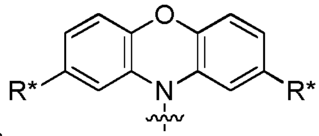;

und R* wie bei Anspruch 1 definiert ist.

**7.** Organisches Molekül nach einem oder mehreren der Ansprüche 1 bis 6, wobei das organische Molekül an mindestens einer Position mindestens einen weiteren Rest R aufweist, wobei
R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN,-$CF_3$, -$NO_2$, -OH, C(=O)OH, C(=O)$OR^3$, C(=O)$N(R^3)_2$, C(=O)$SR^3$, C(=S)$SR^3$, $Si(R^4)_3$, $B(OR^5)_2$, $B(N(R^6)_2)_2$, C(=O)$R^3$, P(=O)$(R^7)_2$, As(=O)$(R^7)_2$, P(=S)$(R^7)_2$, As(=S)$(R^7)_2$, S(=O)$R^3$, S=$NR^3$, S(=O)$NR^3$, $S(=O)_2NR^3$, $S(=O)_2R^3$, O-$S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C$=$CR^9$-, -C=C-, oder eine benachbarte $CH_2$-Gruppe durch -$Si(R^4)_2$-, -$Ge(R^4)_2$-, -$Sn(R^4)_2$, -C(=O)-, -C(=S)-, -C(=Se)-, -C=N-, - C(=O)O-, -C(=O)$N(R^3)$-, -P(=O)$(R^7)$-, -As(=O)$(R^7)$-, -P(=S)$(R^7)$-, -As(=S)$(R^7)$-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein Ringsystem mit insgesamt fünf oder sechs Ringgliedern bilden; wobei $R^2$ bis $R^9$ definiert sind wie in Anspruch 1;
und wobei das organische Molekül durch polymerisierbare Reste, die polymerisierbare funktionelle Einheiten tragen, die mit sich selbst homopolymerisiert oder mit anderen Monomeren copolymiersiert werden können, als Polymer mit Wiederholungseinheiten nach Formel 8 und/oder Formel 9 erhalten wird

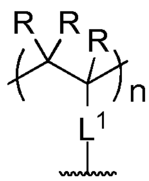

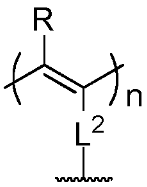

**Formel 8** **Formel 9**

mit
gewellte Linie = Position, über die die Linkergruppe $L^1$ oder $L^2$ an das organische Molekül gebunden ist;
$L^1$ und $L^2$ = gleiche oder verschiedene Linkergruppen, aufweisend zwischen 0 bis 20 Kohlenstoffatome; oder aufweisend eine Form -C(=O)O;
und wobei insbesondere $L^1$ und $L^2$ = -X-$L^3$ mit X = O oder S;
$L^3$ = eine weitere Linkergruppe, ausgewählt aus der Gruppe aus einersubstituierten und unsubstituierten Alkylengruppe (linear, verzweigt oder cyclisch) und einer substituierten und unsubstituierten Arylengruppe, wobei auch Kombinationen möglich sind.

**8.** Verwendung eines organischen Moleküls nach einem oder mehreren der Ansprüche 1 bis 7 als lumineszierender Emitter und/oder als Hostmaterial und/oder als Elektronentransportmaterial und/oder als Lochinjektionsmaterial und/oder als Lochblockiermaterial in einem optoelektronischen Bauelement, wobei das optoelektronische Bauelement insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

9. Verwendung nach Anspruch 8, wobei die Konzentration des organischen Moleküls als Emitter in optischen Licht emittierenden Bauelementen, insbesondere in OLEDs, zwischen 5 % und 80 % beträgt.

10. Optoelektronisches Bauelement, aufweisend ein organisches Molekül nach einem oder mehreren der Ansprüche 1 bis 7, insbesondere ausgeformt als ein Bauelement ausgewählt aus der Gruppe bestehend aus organische lichtemittierende Diode (OLED), lichtemittierende elektrochemische Zelle, OLED-Sensor, insbesondere nicht hermetisch nach außen abgeschirmten Gas- und Dampf-Sensoren, organische Diode, organische Solarzelle, organischer Transistor, organischer Feldeffekttransistor, organischer Laser und Down-Konversion-Element.

11. Optoelektronisches Bauelement nach Anspruch 10, aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein organisches Molekül nach einem oder mehreren der Ansprüche 1 bis 7 enthält.

12. Optoelektronisches Bauelement nach Anspruch 10 oder 11, in dem die lichtemittierende Schicht mindestens ein Hostmaterial aufweist, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls nach einem oder mehreren der Ansprüche 1 bis 7.

13. Optoelektronisches Bauelement nach Anspruch 10 bis 12, wobei mindestens ein Hostmaterial aus einem organischen Molekül gemäß einem oder mehreren der Ansprüche 1 bis 7 besteht.

14. Optoelektronisches Bauelement nach Anspruch 10 bis 13, wobei die lichtemittierende Schicht ein fluoreszentes oder phosphoreszentes Material aufweist und wobei das Material der lichtemittierenden Schicht insbesondere ausgewählt ist aus organischen Molekülen gemäß einem oder mehreren der Ansprüche 1 bis 7.

15. Optoelektronisches Bauelement nach Anspruch 14, in dem ein organisches Molekül in Form eines organischen Moleküls gemäß einem oder mehreren der Ansprüche 1 bis 7 und ein funktionelles Material einen Exciplex bilden.

16. Optoelektronisches Bauelement nach Anspruch 10 bis 15, in dem ein organisches Molekül nach einem oder mehreren der Ansprüche 1 bis 7 als Ladungstransportschicht verwendet wird.

17. Verfahren zur Herstellung eines optoelektronischen Bauelements, wobei ein organisches Molekül nach einem oder mehreren der Ansprüche 1 bis 7 verwendet wird, wobei die Verarbeitung des organischen Moleküls insbesondere mittels eines Vakuumverdampfungsverfahrens oder aus einer Lösung erfolgt.

18. Verwendung eines Separators in Form einer neutralen chemischen Einheit zur Bereitstellung eins organischen Moleküls nach einem oder mehreren der Ansprüche 1 bis 7, wobei mindestens eine erste chemische Einheit AF1, aufweisend ein konjugiertes System und mindestens eine zweite chemische Einheit AF2, die nicht mit AF1 identisch ist, aufweisend ein konjugiertes System, kovalent über den Separator verknüpft wird.

## Claims

1. Organic molecule comprising a structure of formula 1

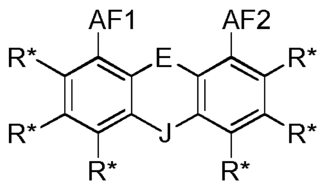

**Formula 1**

wherein

E = is selected from the group consisting of S, S(=O), O, C(=O), $CR^*_2$ $Si(R^4)_2$, $Ge(R^4)_2$, $NR^2$, $PR^3$, $P(=O)R^7$,

$P(=S)R^7$, $AsR^3$, $As(=O)R^7$, $As(=S)R^7$, $SbR^3$ and $BR^3$;

J = not a bond, so that at the two phenyl rings at this position is in each case a radical R*, or a single bond, which connects the both shown phenyl rings;

R* independently at each instance is selected from the group consisting of H, deuterium, phenyl, naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by $-R^9C=CR^9-$, -C=C-, or one adjacent $CH_2$ group may be replaced by $-Si(R^4)_2-$,$-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or-S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^2$ radicals, or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^9$ radicals, or a combination of these systems; at the same time two or more of these R* substituents together may also form a ring system with five or six ring segments;

$R^2$ independently at each instance is selected from the group consisting of H, deuterium, phenyl, naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by $-R^9C=CR^9-$, -C=C-, or one adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$,-O-, or -S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ radicals or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^9$ radicals, or a combination of these systems; at the same time two or more of these $R^2$ substituents together may also form a mono- or polycyclic, aliphatic, aromatic and/or benzofused ring system;

$R^3$ independently at each instance is selected from the group consisting of H, deuterium, phenyl, naphthyl, $CF_3$ or an aliphatic, aromatic and/or heteroaromatic hydrocarbyl radical which has 1 to 20 carbon atoms and in which one or more hydrogen atoms may also be replaced by F or $CF_3$; at the same time, two or more $R^3$ substituents together may also form a mono- or polycyclic aliphatic ring system;

$R^4$ independently at each instance is selected from the group consisting of H, deuterium, phenyl, naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by $-R^9C=CR^9-$, -C=C-, or one adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S- and where one or more hydrogen atoms may be replaced by deuterium, F, Cl, Br, I, CN, $CF_3$ or $NO_2$ or an aromatic or heteroaromatic ring system which has 5 to 60 aromatic ring atoms and may be substituted in each case by one or more $R^8$ radicals, or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^9$ radicals, or a combination of these systems; at the same time, two or more of these $R^4$ substituents together may also form a mono- or polycyclic, aliphatic, aromatic and/or benzofused ring system;

$R^5$ independently at each instance is selected from the group consisting of phenyl, naphthyl, $CF_3$, $C(=O)R^3$, $P(=O)(R^7)_2$, $As(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by $-R^9C=CR^9-$, -C=C-, or one adjacent $CH_2$ group

may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ radicals, or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^9$ radicals, or a combination of these systems; at the same time, two or more of these $R^5$ substituents together may also form a mono- or polycyclic, aliphatic, aromatic and/or benzofused ring system;

$R^6$ independently at each instance is selected from the group consisting of phenyl, naphthyl, $CF_3$, $Si(R^4)_3$, $C(=O)R^3$, $P(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by $-R^9C=CR^9-$, -C=C-, or one adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ radicals, or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^9$ radicals, or a combination of these systems; at the same time, two or more of these $R^6$ substituents together may also form a mono- or polycyclic, aliphatic, aromatic and/or benzofused ring system;

$R^7$ independently at each instance is selected from the group consisting of phenyl, naphthyl, $N(R^2)_2$, CN, $CF_3$, $C(=O)OR^3$, $C(=O)N(R^3)_2$, $Si(R^4)_3$, $C(=O)R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^9$ radicals, where one or more adjacent $CH_2$ groups may be replaced by-$R^9C=CR^9-$, -C=C-, or one adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, or -S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^9$ radicals, or an aryloxy or heteroaryloxy group which has 5 to 60 aromatic ring atoms and may be substituted by one or more $R^9$ radicals, or a diarylamino group, diheteroarylamino group or arylheteroarylamino group which has 10 to 40 aromatic ring atoms and may be substituted by one or more $R^3$ radicals, or a combination of these systems; at the same time, two or more of these $R^7$ substituents together may also form a mono- or polycyclic, aliphatic, aromatic and/or benzofused ring system;

$R^8$ independently at each instance is selected from the group consisting of H, deuterium, phenyl, naphthyl, F, $CF_3$ or an aliphatic, aromatic and/or heteroaromatic hydrocarbyl radical which has 1 to 20 carbon atoms and in which one or more hydrogen atoms may also be replaced by F or $CF_3$; at the same time, two or more of these $R^8$ substituents together may also form a mono- or polycyclic aliphatic ring system;

$R^9$ independently at each instance is selected from the group consisting of H, deuterium, phenyl, naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, each of which may be substituted by one or more $R^8$ radicals, where one or more adjacent $CH_2$ groups may be replaced by $-R^3C=CR^3-$, -C=C-, or one adjacent $CH_2$ group may be replaced by $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, or -S- and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may be substituted in each case by one or more $R^8$ radicals, or an aryloxy or heteroaryloxy group which has 5 to 60 aromatic ring atoms and may be substituted by one or more $R^3$ radicals, or a diarylamino group, diheteroarylamino group or arylheteroarylamino group which has 10 to 40 aromatic ring atoms and may be substituted by one or more $R^8$ radicals, or a combination of these systems; at the same time two or more of these $R^9$ substituents together may also form a mono- or polycyclic, aliphatic, aromatic and/or benzofused ring system;

AF1 and AF2 are organic chemical units, wherein AF1 and AF2 are different from each other and wherein AF1 comprises a structure of sub-formula 1

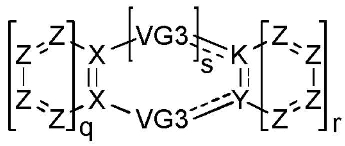

**Sub-Formula 1**

wherein:

q is 0, 1; r is 0, 1; s is 0, 1;
VG3 = bridging group, is at each instance independently selected from the groups consisting of N, O, S, CR**, C, an element-element-single bond between X and Y or between X and K, wherein 2 VG3 units are not at the same time an element-element-single bond between X and Y and between X and K; * □ BR**, NR**, $GeR^{**}_2$, $AsR^{**}_2$, $SiR^{**}_2$, wherein two VG3 units are not at the same time BR**, NR**, $GeR^{**}_2$, $AsR^{**}_2$, $SiR^{**}_2$;

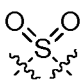, 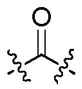, 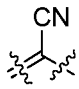, 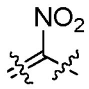, 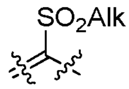, 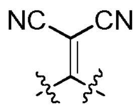, 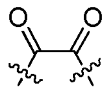, 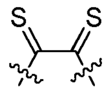, 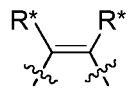,

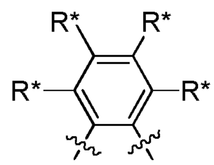,

wherein two VG3 units are not at the same time

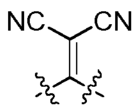, 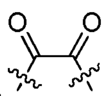, 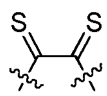

, 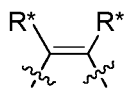, 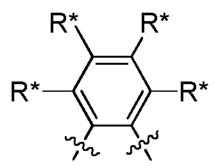;

X is at each instance independently C; or is CR**, N if q = 0;
Y is C; or is CR**, $CR^{**}_2$, N, NR**, O, S if r = 0;
K is Y; or is R* if r = 0 and at the same time s = 0;
Z is at each instance independently CR** or N;
and where maximally 4 of the VG3 units, K, X, Y, Z are at the same time N; und where at least 1 of the VG3 units, K, X, Y, Z is a group which is different from a C-H group, and comprises at least 1 nitrogen atom or an oxygen atom or a sulfur atom.
and where for the case r = 0 two adjacent Z groups may form with each other or a VG3 group may form with the nearest group Z or X or Y a bridge via the following units:

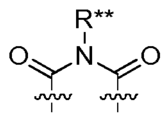, 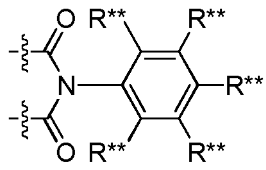 ;

where maximally three of these units are part of the structure according to sub-formula 1;

and where AF2 comprises a structure of sub-formula 2

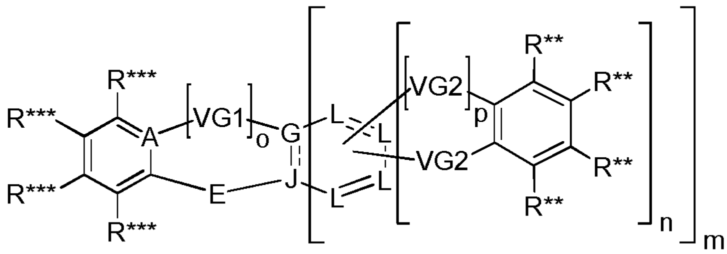

**Sub-Formula 2**

wherein:

m is 0 or 1;
n is 0 or 1, wherein if m = 0 always also n = 0;
o is 0 or 1;
p is 0 or 1;
A is CR*** if o = 0, otherwise C;
VG1 = bridging group, is selected from the group consisting of

- NR**, CR**$_2$, O, S and a C-C-single bond; or
- NR**, CR**$_2$, O, S, a C-C-single bond, BR**, AsR**, SiR**$_2$, GeR**$_2$,

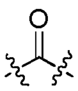, 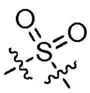, 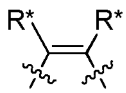, 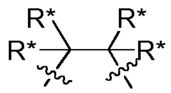, 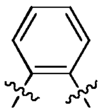, 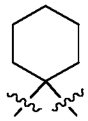, 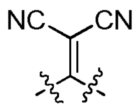, 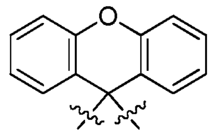,

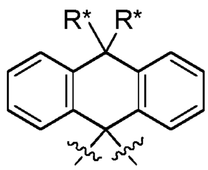, 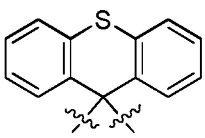, and 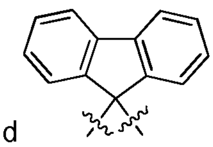,

when m = 1 and simultaneously n = 0;

VG2 = bridging group is at each instance independently selected from the group consisting of CR**$_2$, NR**, O, S and a C-C single bond, wherein two VG2 units are not at the same time a C-C single bond;
E is selected from the group consistent of NR**,

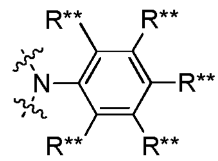

,

O and S;

G is C if o = 1 and at the same time m = 1; G is CR** if o = 0 and at the same time m = 1; is CR**, $CR**_2$, if o = 1 and at the same time m = 0; is R* if o = 0 and at the same time m = 0; is CR**, $CR**_2$, N, NR* if m = 0 and at the same time VG1 is C-C-single bond;

J is C if m = 1; is CR**, $CR**_2$, NR** if m = 0;

L is CR*** if n = 0; is CR** or C (in the case of covalent bonding to VG2) if n = 1;

R*** is R** or is selected from the following units, wherein maximally two of the radicals R*** at the same time are equal to one of the following entities:

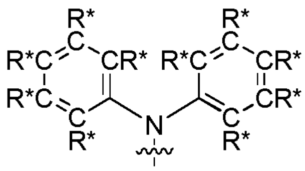
,
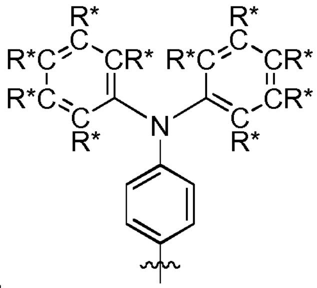
,
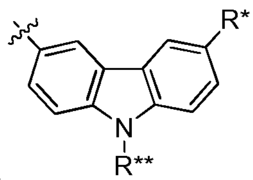
,
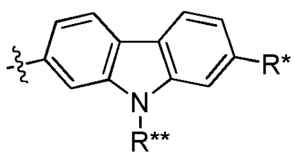
,

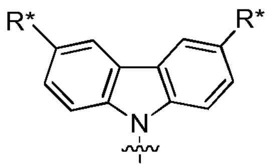
,
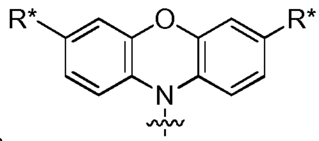
,
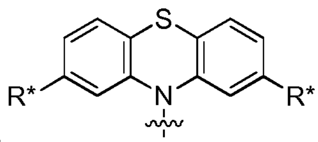
,

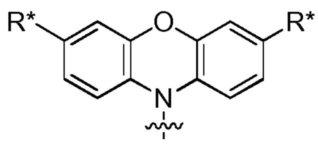
,
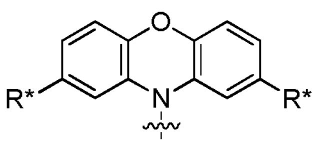
;

and wherein

R** is at each instance independently a radical R* and/or indicates an attachment point on a separator S, wherein exactly one R** is an attachment point to a separator S;

wherein the separator is the part of the organic molecule according to formula 1 without chemical units AF.

**2.** The organic molecule according to claim 1, where the organic molecule comprises of a structure according to formula 2a or 2b:

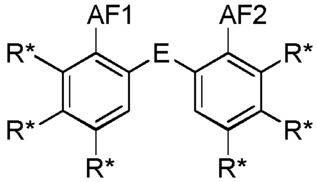

**Formula 2a**

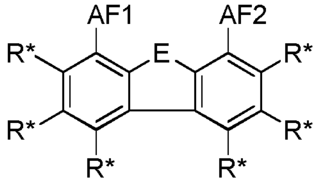

**Formula 2b**

wherein the definitions from claim 1 apply.

**3.** The organic molecule according to claim 1 or 2, wherein the molecule comprises of one of the following structures and wherein the definitions from claim 1 apply:

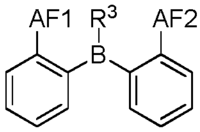
2-1

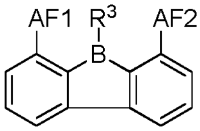
2-2

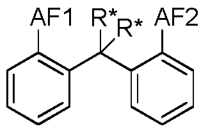
2-3

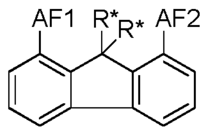
2-4

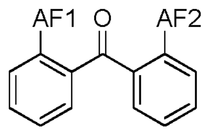
2-5

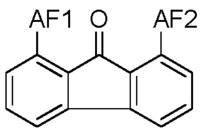
2-6

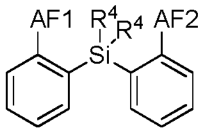
2-7

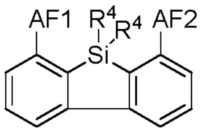
2-8

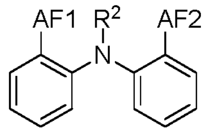
2-9

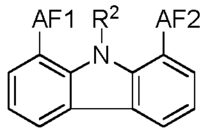
2-10

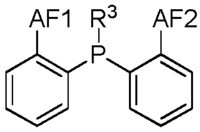
2-11

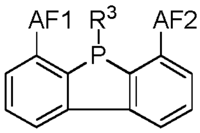
2-12

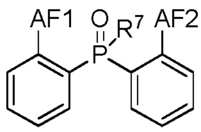
2-13

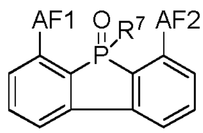
2-14

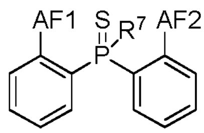
2-15

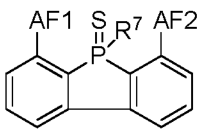
2-16

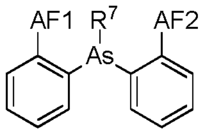
2-17

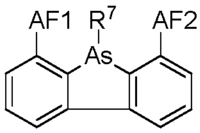
2-18

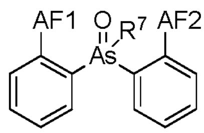
2-19

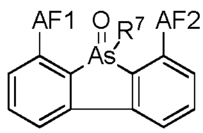
2-20

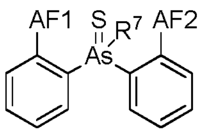
2-21

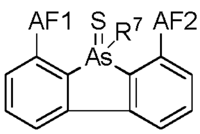
2-22

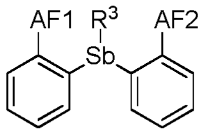
2-23

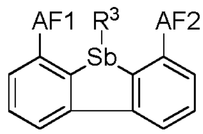
2-24

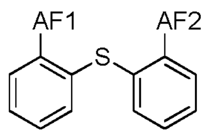
2-25

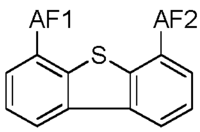
2-26

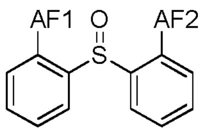
2-27

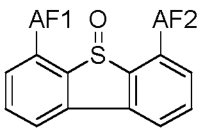
2-28

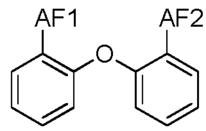
2-31

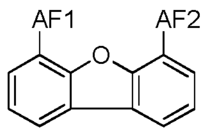
2-32

**4.** The organic molecule according to claim 1 to 3, wherein the organic molecule comprises of a structure of formula 5a or 5b:

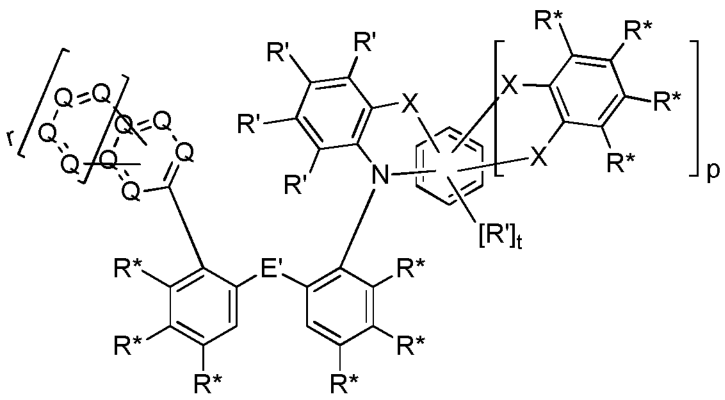

**Formula 5a**

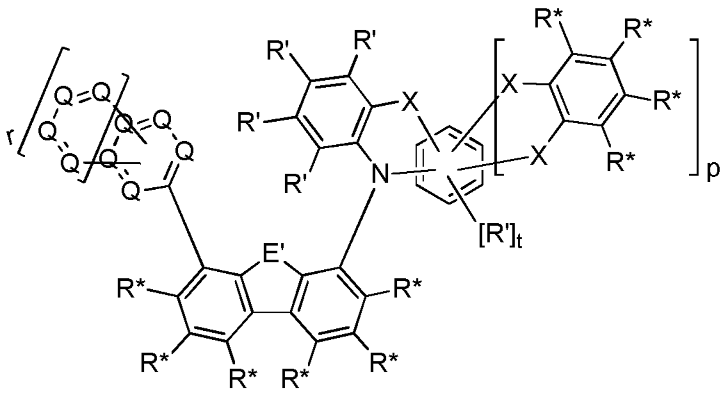

**Formula 5b**

where in formula 5a/5b:

p is 0 or 1;
t = 4 - 2p;
X is $CR^*_2$, NR*, oxygen, sulfur, a direct bond, wherein maximally two place holders X are at the same time a direct bond, wherein the maximally two place holders X are not part of the same ring;
Q is N, CR* or for r = 1 Q is the corresponding annulation position C, wherein two adjacent Q units are not at the same time N;
r = 0 or 1,
E' is S, O, C(=O), $CR^*_2$, $Si(R^4)_2$, $Ge(R^4)_2$, $NR^2$, $BR^3$;
R' is R* or R' is selected from the following units, wherein maximally two of the radicals R' are at the same time one of the following units:

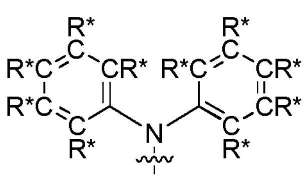 , 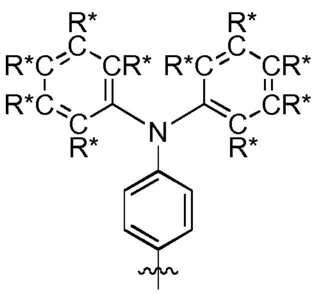 , 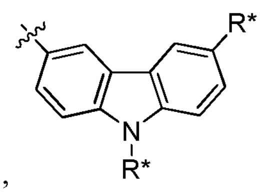 , 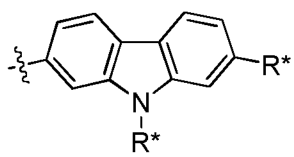 ,

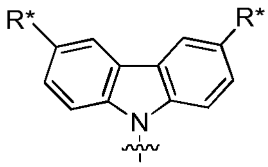 , 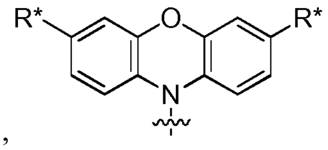 , 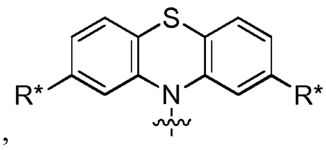 ,

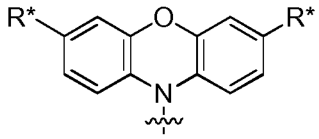 , 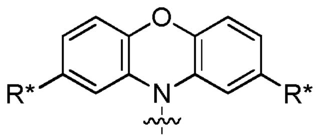 ;

and R* is defined as in claim 1.

**5.** The organic molecule according to claims 1 to 3, wherein the organic molecule comprises a structure of the formula 6a or 6b:

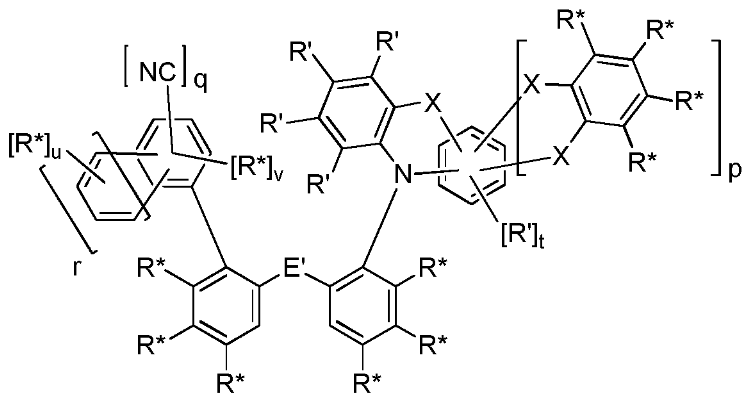

**Formula 6a**

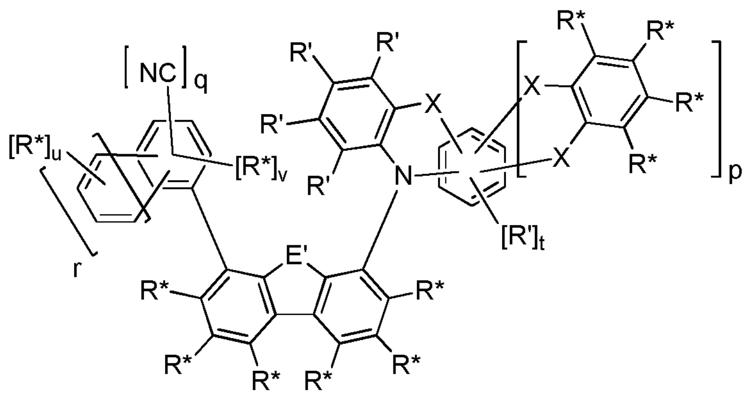

**Formula 6b**

where in formula 6a/6b:

p is 0 or 1;
t = 4 - 2p;
X is $CR^*_2$, NR*, Oxygen, Sulfur, a direct bond, wherein maximally two place holders X are at the same time a direct bond, wherein the maximally two place holders X are not part of the same ring;
r = 0 or 1,
q = 1 to 5, for r = 1 is q = 1 to 3,

u is 1, 2, 3 or 4

v is 1, 2 or 3; wherein: u + v = 3 + 4r - q

E' is S, O, C(=O), $CR^*_2$, $Si(R^4)_2$, $Ge(R^4)_2$, $NR^2$, $BR^3$;

R' is R* or R' is selected from the following units, wherein maximally two of the radicals R' are at the same time one of the following units:

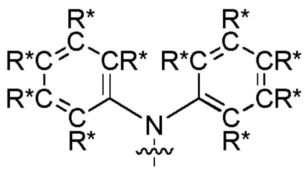
,
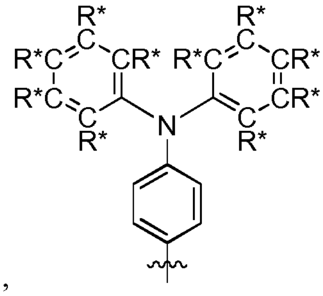
,
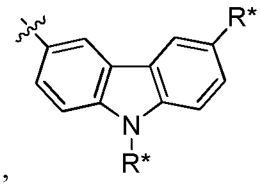
,
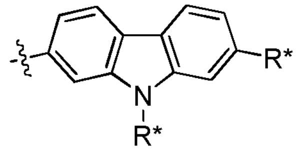
,

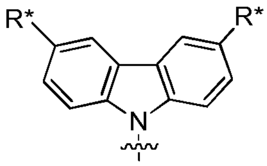
,
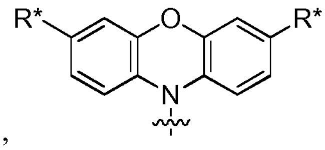
,
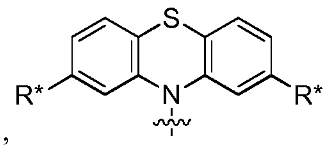
,

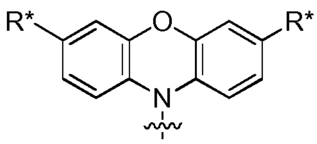
,
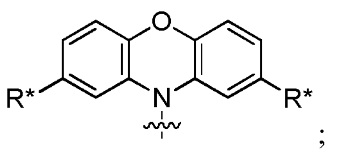
;

and R* is defined as in claim 1.

**6.** The organic Molecule according to claims 1 to 3, wherein the organic molecule comprises a structure of formula 7a or 7b:

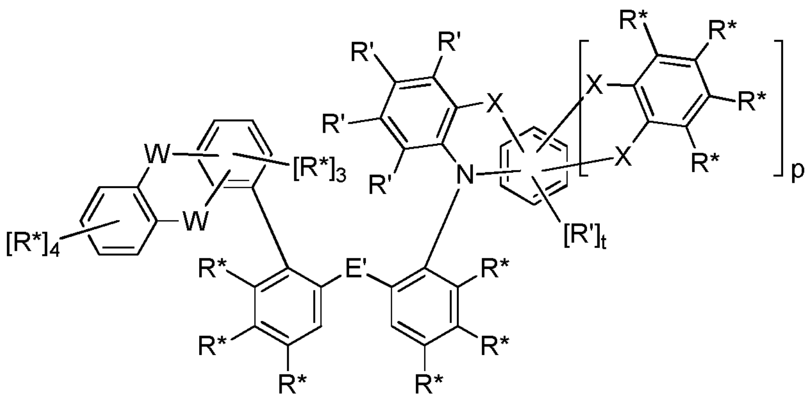

**Formula 7a**

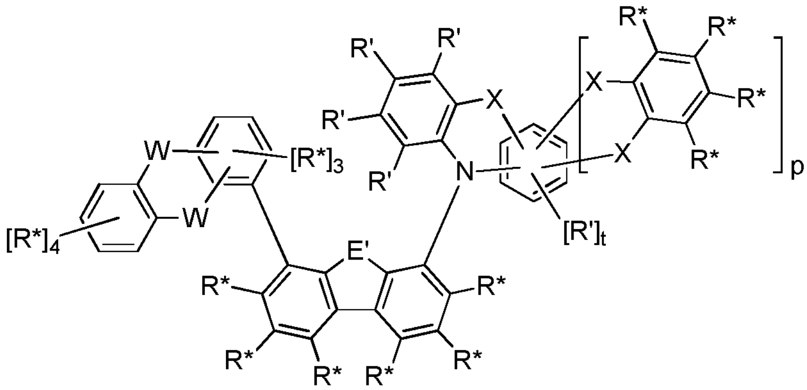

**Formula 7b**

where in formula 7a/7b:

p is 0 or 1;
t = 4 - 2p;
X is $CR^*_2$, NR*, oxygen, sulfur, a direct bond, wherein maximally two place holders X are at the same time a direct bond, wherein the maximally two place holders X are not part of the same ring;
W is selected from the group consisting of

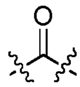, 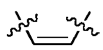, 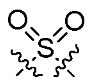,

an element-element single bond, where two units W are not at the same time an element-element single bond, NR*, where two units W are not at the same time NR*;
E' is selected from the group consisting of S, O, C(=O), $CR^*_2$, $Si(R^4)_2$, $Ge(R^4)_2$, $NR^2$ and $BR^3$;
R' is R* or is selected from the group of the following units, wherein maximally two of the radicals R' are at the same time one of the following units:

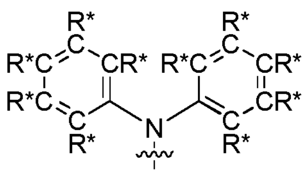, 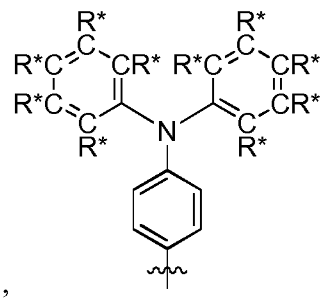, 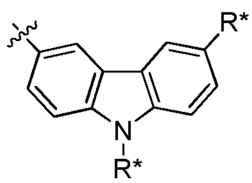, 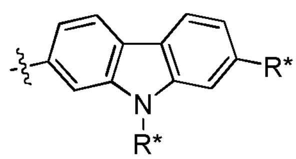,

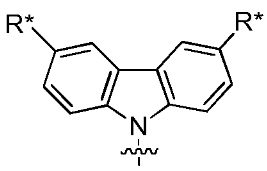, 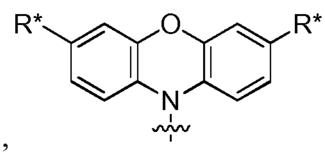, 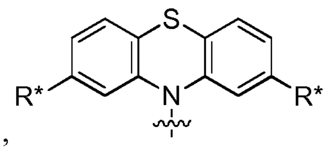,

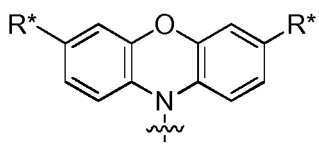, 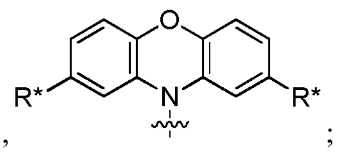;

and R* is defined as in claim 1.

**7.** The organic molecule according to one or more of claims 1 to 6, wherein the organic molecule at at least one position comprises at least one further radical R, wherein R is at each instance independently selected from the group consisting of H, deuterium, phenyl, naphthyl, F, Cl, Br, I, $N(R^2)_2$, -CN, -NC, -SCN, $-CF_3$, $-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$ a linear alkyl, alkoxy or thioalkoxy group with 1 to 40 C atoms or a linear alkenyl or alkynyl group with 2 to 40 C atoms or a branched or cyclic alkyl, alkenyl, alkynyl, alkoxy or thioalkoxy group with 3 to 40 C atoms, which in each case may be substituted with one or more radicals $R^9$, wherein one or more adjacent $CH_2$ groups may be replaced by $-R^9C=CR^9-$, -C=C-, or one adjacent $CH_2$-group may be replaced by $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$,-S(=O)-, $-S(=O)_2-$, $-NR^{2}-$, -O-, or-S- and wherein one or more H atoms may be replaced by deuterium, F, Cl, Br, I, CN, $CF_3$ or $NO_2$, or an aromatic or heteroaromatic ring system with 5 to 60 aromatic ring atoms, which in each case may be substituted with one or more radicals $R^2$ or an aryloxy or heteroaryloxy group with 5 to 60 aromatic ring atoms, which may be substituted with one or more radicals $R^9$, or a diarylamino group, diheteroarylamino group or arylheteroarylamino group with 10 to 40 aromatic ring atoms, which may be substituted with one or more radicals $R^9$, or a combination of these systems; two or more of these substituents R may also form a mono- or polycyclic aliphatic, aromatic and/or benzoanellated ring system; wherein $R^2$ to $R^9$ are defined as in claim 1.
and wherein due to polymerizable radicals bearing polymerizable functional units, which can homopolymerize with themselves or copolymerize with other monomers, the organic molecule is obtained as a polymer with repeat units according to formulas 8 and/or 9

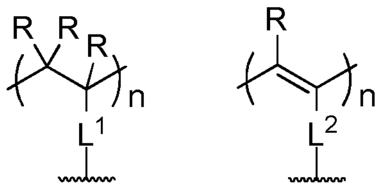

**Formula 8** **Formula 9**

with
curved line = position through which the linker group $L^1$ or $L^2$ is bound to the organic molecule; $L^1$ and $L^2$ = identical or different linker groups, comprising between 0 to 20 carbon atoms, or comprising a form -C(=O)O;
and wherein in particular $L^1$ and $L^2$ =$-X-L^3-$ with X = O or S;
$L^3$ = a linker group selected from the group of a substituted und unsubstituted alkylene group (linear, branched or cyclic) and a substituted and unsubstituted arylene group, wherein combinations are also possible.

**8.** Use of an organic molecule according to one or more of claims 1 to 7 as luminescent emitter and/or as host material and/or as electron transport material and/or as hole injection material and/or as hole blocking material in an optoelectronic component, wherein the optoelectronic component is in particular selected from the group consisting of:

- Organic light-emitting diodes (OLEDs),
- Light-emitting electrochemical cells,
- OLED sensors, especially in gas and vapor sensors not hermetically sealed to the outside,
- Organic diodes,
- Organic solar cells,
- Organic transistors,
- Organic field effect transistors,
- Organic lasers, and
- Down-conversion elements.

**9.** The use according to claim 8, wherein the concentration of the organic molecule as emitter in optical light emitting components, in particular in OLEDs, is between 5 % and 80 %.

**10.** Optoelectronic component, comprising an organic molecule according to one or more of claims 1 to 7, in particular

formed as a component selected from the group consisting of organic light-emitting diode (OLED), light-emitting electrochemical cell, OLED sensor, in particular gas and vapor sensors not hermetically sealed to the outside, organic diode, organic solar cell, organic transistor, organic field effect transistor, organic laser and down-conversion element.

**11.** The optoelectronic component according to claim 10, comprising a substrate, an anode and a cathode, wherein the anode and the cathode are applied to the substrate, and at least one light-emitting layer applied between the anode and the cathode and comprising an organic molecule according to one or more of claims 1 to 7.

**12.** The optoelectronic component according to claim 10 or 11, in which the light-emitting layer comprises at least one host material, wherein in particular the excited singlet state ($S_1$) and/or the excited triplet state ($T_1$) of the at least one host material is higher than that of the excited singlet state ($S_1$) and/or the excited triplet state ($T_1$) of the organic molecule of one or more of claims 1 to 7.

**13.** The optoelectronic component according to claims 10 to 12, comprising at least one host material consisting of an organic molecule according to one or more of claims 1 to 7

**14.** The optoelectronic component according to claims 10 to 13, wherein die light-emitting layer comprises fluorescent or phosphorescent materials and wherein the materials of the light-emitting layer are in particular selected from organic molecules according to one or more of claims 1 to 7.

**15.** The optoelectronic component according to claim 14, in which an organic molecule in form of an organic molecule according to one or more of the claim 1 to 7 and a functional material are forming an exciplex.

**16.** The optoelectronic component according to claims 10 to 15, in which an organic molecule according to one or more of claims 1 to 7 is used as a charge transport layer.

**17.** Method for producing an optoelectronic component, wherein an organic molecule according to one or more of claims 1 to 7 is used, wherein the processing of the organic molecule is performed using a vacuum evaporation method or from a solution.

**18.** Use of a separator in form of a neutral chemical unit for the provision of an organic molecule according to one or more of the claims 1 to 7, wherein at least a first chemical unit AF1, comprising a conjugated system, and at least a second chemical unit AF2, which is not identical with AF1, comprising a conjugated system, are linked to each other via the separator.

**Revendications**

**1.** Molécule organique, comportant une structure de formule 1

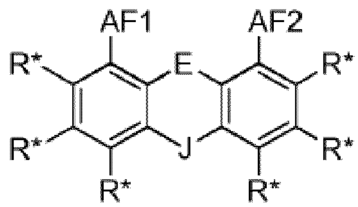

**Formule 1**

dans laquelle

E = est choisi dans le groupe constitué par S, S(=O), O, C(=O), $CR^*_2$ $Si(R^4)_2$, $Ge(R^4)_2$, $NR^2$, $PR^3$, $P(=O)R^7$, $P(=S)R^7$, $AsR^3$, $As(=0)R^7$, $As(=S)R^7$, $SbR^3$ et $BR^3$;
J = aucune liaison, de sorte qu'un radical R* est présent en cette position sur chacun des deux cycles phényle, ou une liaison simple qui relie l'un à l'autre les deux cycles phényle indiqués ;
R* est choisi indépendamment à chaque occurrence dans le groupe constitué par H, le deutérium, les groupes

phényle, naphtyle, F, Cl, Br, I, $N(R^2)_2$, -CN, -NC, -SCN, $-CF_3$, $-NO_2$, OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par-$R^9C=CR^9$-, -C°C-, ou un groupe $CH_2$ voisin pouvant être remplacé par $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^2$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants R* peuvent en outre également former ensemble un système cyclique ayant au total cinq ou six chaînons formant le cycle ;

$R^2$ est choisi indépendamment à chaque occurrence dans le groupe constitué par H, le deutérium, les groupes phényle, naphtyle, $-CF_3$, $C(=O)OR^3$, $C(=O)N(R^3)_2$, $Si(R^4)_3$, $C(=O)R^3$ $P(=O)(R^7)_2$, $As(=O)(R^7)_2$, $P(=S)(R^7)_2$, $As(=S)(R^7)_2$, $S(=O)R^3$, $S(=O)_2R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par -$R^9C=CR^9$-, -C°C-, ou un groupe $CH_2$ voisin pouvant être remplacé par $-Si(R^4)_2-$, $-Ge(R^4)_2-$, $-Sn(R^4)_2-$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^2$ peuvent en outre également former ensemble un système cyclique mono- ou polycyclique, aliphatique, aromatique et/ou soudé à un noyau benzénique ;

$R^3$ est choisi indépendamment à chaque occurrence dans le groupe constitué par H, le deutérium, les groupes phényle, naphtyle, $CF_3$ ou un radical hydrocarboné aliphatique, aromatique et/ou hétéroaromatique ayant de 1 à 20 atomes de carbone, dans lequel un ou plusieurs atomes d'hydrogène peuvent également être remplacés par F ou $CF_3$; deux ou plus de deux de ces substituants $R^3$ peuvent en outre également former ensemble un système cyclique mono- ou polycyclique, aliphatique ;

$R^4$ est choisi indépendamment à chaque occurrence dans le groupe constitué par H, le deutérium, les groupes phényle, naphtyle, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par -$R^9C=CR^9$-, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^8$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^4$ peuvent en outre également former ensemble un système cyclique mono- ou polycyclique, aliphatique, aromatique et/ou soudé à un noyau benzénique ;

$R^5$ est choisi indépendamment à chaque occurrence dans le groupe constitué par les groupes phényle, naphtyle, $CF_3$, $C(=O)R^3$, $P(=O)(R^7)_2$, $As(=O)(R^7)_2$, un groupe alkyle, alcoxy ou thioalcoxy linéaire ayant de 1 à 40 atomes

de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^5$ peuvent en outre également former ensemble un système cyclique mono- ou polycyclique, aliphatique, aromatique et/ou soudé à un noyau benzénique ;

$R^6$ est choisi indépendamment à chaque occurrence dans le groupe constitué par les groupes phényle, naphtyle, $CF_3$, $Si(R^4)_3$, $C(=O)R^3$, $P(=O)(R^7)_2$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^9$, ou une combinaison de ces systèmes ; deux ou plus de deux de ces substituants $R^6$ peuvent en outre également former ensemble un système cyclique mono- ou polycyclique, aliphatique, aromatique et/ou soudé à un noyau benzénique ;

$R^7$ est choisi indépendamment à chaque occurrence dans le groupe constitué par les groupes phényle, naphtyle, $N(R^2)_2$, CN, $CF_3$, $C(=O)OR^3$, $C(=O)N(R^3)_2$, $Si(R^4)_3$, $C(=O)R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^9$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^3$, ou une combinaison de ces systèmes ; deux ou plus de deux de ces substituants $R^7$ peuvent en outre également former ensemble un système cyclique mono- ou polycyclique, aliphatique, aromatique et/ou soudé à un noyau benzénique ;

$R^8$ est choisi indépendamment à chaque occurrence dans le groupe constitué par H, le deutérium, les groupes phényle, naphtyle, F, $CF_3$, ou un radical hydrocarboné aliphatique, aromatique et/ou hétéroaromatique ayant de 1 à 20 atomes de carbone, dans lequel un ou plusieurs atomes d'hydrogène peuvent également être remplacés par F ou $CF_3$; deux ou plus de deux de ces substituants $R^8$ peuvent en outre également former ensemble un système cyclique mono- ou polycyclique, aliphatique ;

$R^9$ est choisi indépendamment à chaque occurrence dans le groupe constitué par H, le deutérium, les groupes phényle, naphtyle, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $AS(=O)(R^7)_2$, $P(=S)(R^7)_2$, $S(=O)R^3$, $S(=O)_2R^3$, $OSO_2R^3$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^8$, un ou plusieurs groupes $CH_2$ non contigus pouvant être remplacés par $-R^3C=CR^3-$, -C°C-, ou un groupe $CH_2$ voisin

pouvant être remplacé par -Si($R^4$)$_2$-, -Ge($R^4$)$_2$-, - Sn($R^4$)$_2$, -C(=O)-, -C(=S)-, -C(=Se)-, -C=N-, -C(=O)O-, -C(=O)N($R^3$)-, -P(=O)($R^7$)-, - As(=O)($R^7$)-, -P(=S)($R^7$)-, -As(=S)($R^7$)-, -S(=O)-, -S(=O)$_2$-, -$NR^2$-, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^8$, ou un groupe aryloxy ou hétéroaryloxy ayant de 5 à 60 atomes formant le cycle aromatique, qui peut être substitué par un ou plusieurs radicaux $R^3$, ou un groupe diarylamino, un groupe dihétéroarylamino ou un groupe arylhétéroarylamino ayant de 10 à 40 atomes formant le cycle aromatique, qui peut être substitué par un ou plusieurs radicaux $R^8$, ou une combinaison de ces systèmes ; deux ou plus de deux de ces substituants $R^9$ peuvent en outre également former ensemble un système cyclique mono- ou polycyclique, aliphatique, aromatique et/ou soudé à un noyau benzénique ;
AF1 et AF2 sont des unités chimiques organiques, AF1 et AF2 étant différente l'une de l'autre et AF1 présentant une structure de sous-formule 1

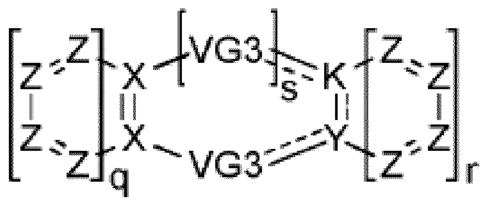

**Sous-formule 1**

dans laquelle :

q est 0 ou 1 ; r est 0 ou 1 ; s est 0 ou 1 ;
VG3 = est un groupe pontant choisi indépendamment à chaque occurrence dans les groupes constitués par N, O, S, CR**, une simple liaison élément-élément entre X et Y ou entre X et K, 2 unités VG3 ne représentant pas simultanément une simple liaison élément-élément entre X et Y et entre X et K ; BR**, NR**, GeR**$_2$, AsR**$_2$, SiR**$_2$, deux unités VG3 ne représentant pas simultanément BR**, NR**, GeR**$_2$, AsR**$_2$, SiR**$_2$;

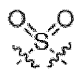, 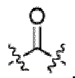, 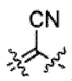, 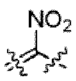, 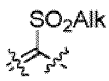, 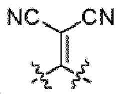, 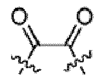, 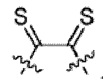, 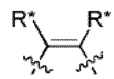,

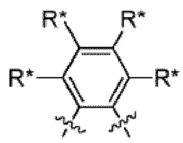,

deux unités VG3 ne représentant pas simultanément

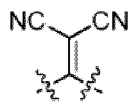, 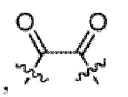, 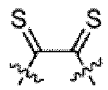, 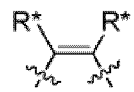, 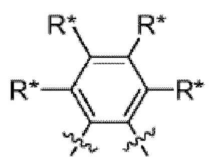 ;

X est indépendamment à chaque occurrence C ; ou X est CR**, N lorsque q = 0 ;
Y est C ; ou Y est CR**, CR**$_2$, N, NR**, O ou S lorsque r = 0 ;
K est Y ; ou K est R* lorsque r = 0 et en même temps s = 0 ;
Z est indépendamment à chaque occurrence CR** ou N ;
et où au maximum 4 des unités VG3, K, X, Y, Y, Z représentent simultanément N ; et où au moins 1 des unités VG3, K, X, Y, Z représente un groupe différent de C-H, qui contient au moins 1 atome d'azote ou un atome d'oxygène ou un atome de soufre ;

et où dans le cas où r = 0, deux groupes Z voisins ensemble ou un groupe VG3 avec le groupe Z ou X ou Y se trouvant le plus proche de lui peuvent également être pontés par les unités suivantes :

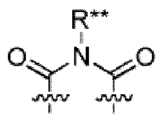, 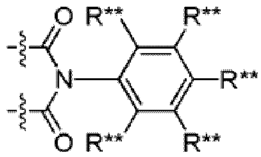 ;

au maximum trois de ces unités étant contenues dans la structure selon la sous-formule 1 ;
et AF2 présentant une structure de sous-formule 2

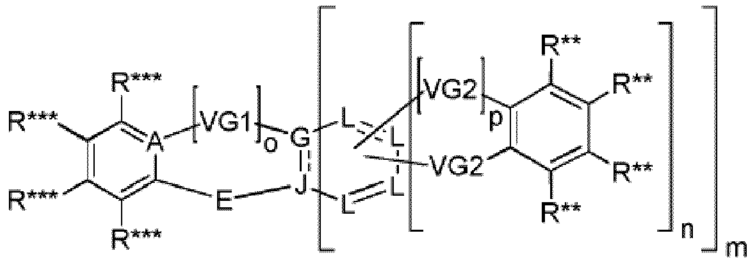

**Sous-formule 2**

dans laquelle :

m est 0 ou 1 ;
n est 0 ou 1, lorsque m = 0 n valant également toujours 0 ;
o est 0 ou 1 ;
p est 0 ou 1 ;
A est CR*** lorsque o = 0, sinon C ;

VG1 = est un groupe pontant choisi dans le groupe constitué par

- NR**, $CR^{**}_2$, O, S et une simple liaison C-C ; ou
- NR**, $CR^{**}_2$, O, S, une simple liaison C-C, BR**, AsR**, $SiR^{**}_2$, $GeR^{**}_2$,

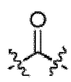, 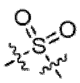, 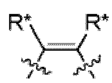, 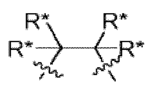, 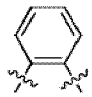, 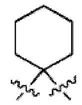, 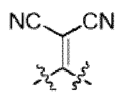, 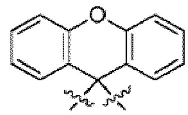,

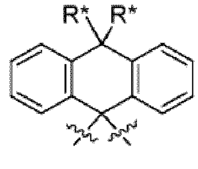, 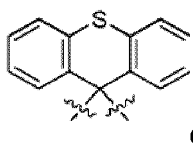 et 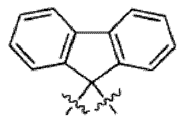,

lorsque m = 1 et en même temps n = 0 ;

VG2 = est un groupe pontant choisi indépendamment à chaque occurrence dans le groupe constitué par $CR^{**}_2$, NR**, O, S et une simple liaison C-C, deux unités VG2 ne représentant pas simultanément une simple liaison C-C ;
E est choisi dans le groupe constitué par NR**,

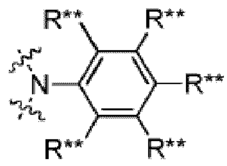

,

O et S;

G est C lorsque o = 1 et en même temps m = 1 ; est CR** lorsque o = 0 et en même temps m = 1 ; est CR** ou $CR^{**}_2$ lorsque o = 1 et en même temps m = 0 ; est R* lorsque o = 0 et en même temps m = 0 ; est CR**, $CR^{**}_2$, N ou NR* lorsque m = 0 et en même temps VG1 est une simple liaison C-C ;

J est C lorsque m = 1 ; est CR**, $CR^{**}_2$ ou NR** lorsque m = 0 ;

L est CR*** lorsque n = 0 ; est CR** ou C (avec liaison covalente à VG2) lorsque n = 1 ;

R*** est R** ou est choisi parmi les unités suivantes, au maximum deux des radicaux R*** représentant simultanément l'une des unités suivantes :

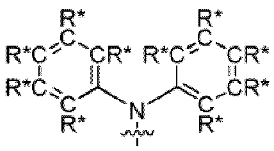 , 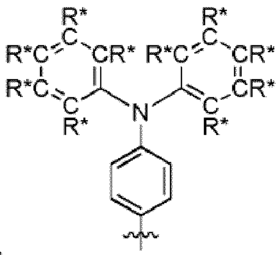 , 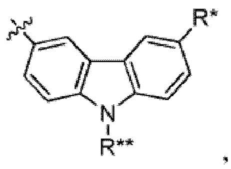 , 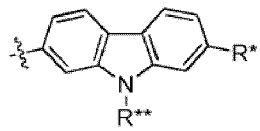 ,

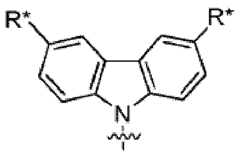 , 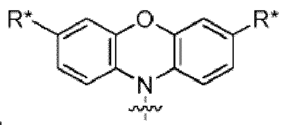 , 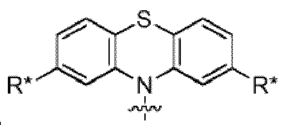 ,

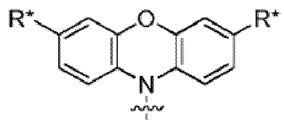 , 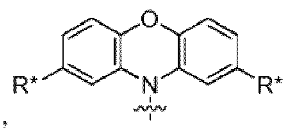 ;

et où

R** est indépendamment à chaque occurrence un radical R* et/ou marque une position d'attachement à un séparateur S, un radical R** étant précisément une position d'attachement à un séparateur S ;

le séparateur étant la partie de la molécule organique selon la formule 1 sans unités chimiques AF.

**2.** Molécule organique selon la revendication 1, où la molécule organique présente une structure de formule 2a ou 2b :

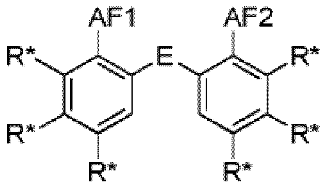

**Formule 2a**

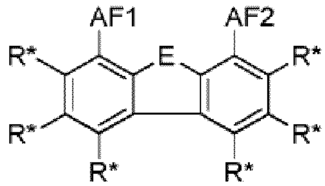

**Formule 2b**

où s'appliquent les définitions indiquées dans la revendication 1.

**3.** Molécule organique selon la revendication 1 ou 2, où la molécule présente l'une des structures suivantes et où

s'appliquent les définitions indiquées dans la revendication 1 :

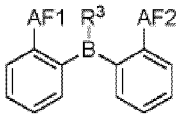
2-1

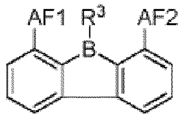
2-2

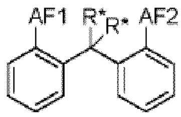
2-3

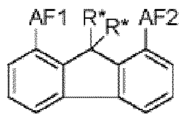
2-4

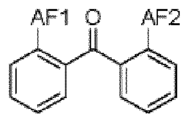
2-5

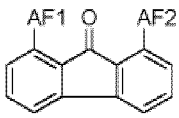
2-6

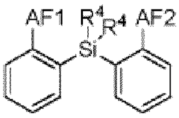
2-7

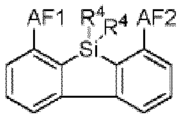
2-8

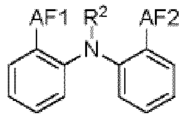
2-9

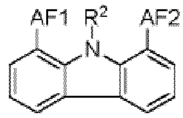
2-10

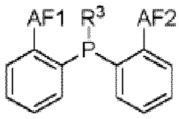
2-11

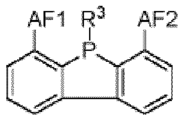
2-12

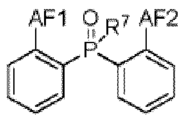
2-13

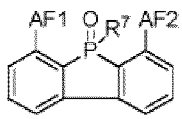
2-14

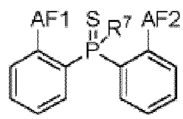
2-15

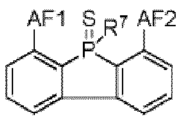
2-16

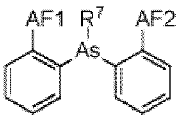
2-17

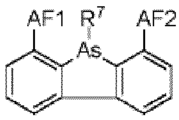
2-18

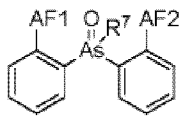
2-19

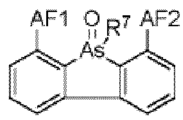
2-20

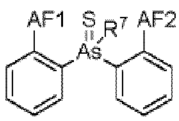
2-21

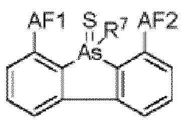
2-22

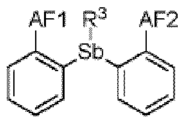
2-23

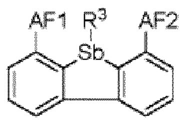
2-24

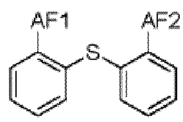
2-25

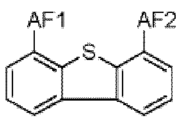
2-26

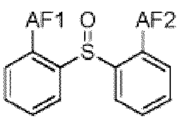
2-27

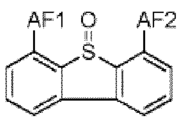
2-28

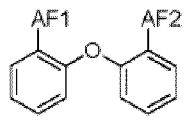
2-31

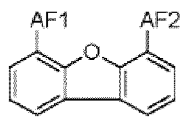
2-32

**4.** Molécule organique selon l'une quelconque des revendications 1 à 3, où la molécule organique présente l'une des structures de formule 5a ou 5b :

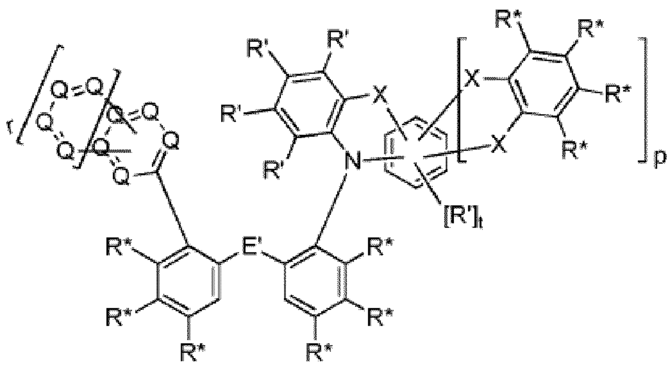

**Formule 5a**

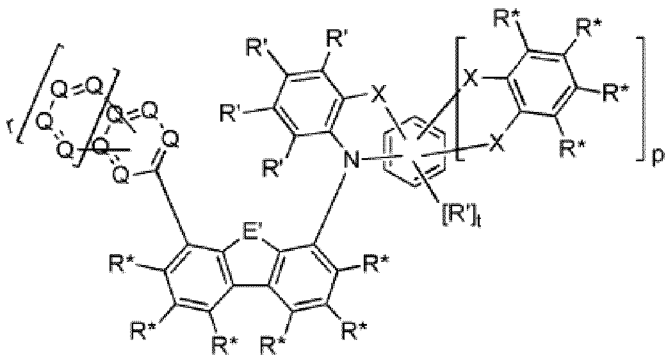

**Formule 5b**

où dans la formule 5a/5b

p est 0 ou 1 ;
t = 4-2p ;
X est $CR^*_2$, NR*, un atome d'oxygène, un atome de soufre, une liaison directe, au maximum deux symboles X représentant simultanément une liaison directe, et ceux-ci ne faisant pas partie du même cycle ;
Q est N, CR* ou, pour r = 1 les positions de condensation correspondantes C, deux unités directement voisines Q ne représentant pas simultanément N ;
r = 0 ou 1 ;
E' est S, O, C(=O), $CR^*_2$, $Si(R^4)_2$, $Ge(R^4)_2$, $NR^2$, $BR^3$;
R' est R* ou est choisi parmi les unités suivantes, au maximum deux des radicaux R' représentant simultanément l'une des unités suivantes :

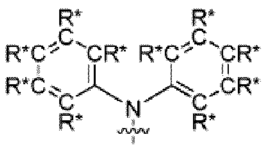 , 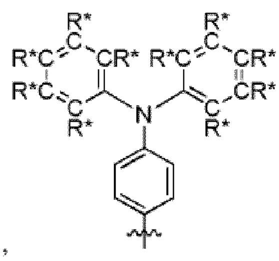 , 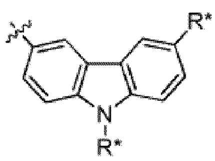 , 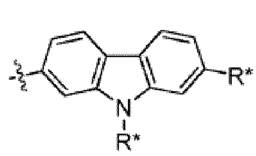 ,

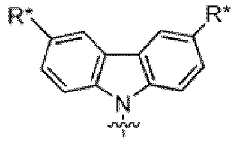 , 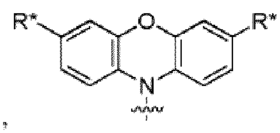 , 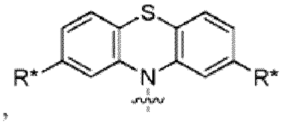 ,

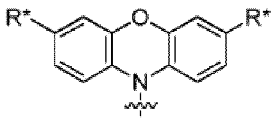
,
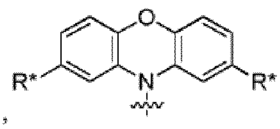
;

et R* étant tel que défini dans la revendication 1.

**5.** Molécule organique selon l'une quelconque des revendications 1 à 3, où la molécule organique présente une structure de formule 6a ou 6b :

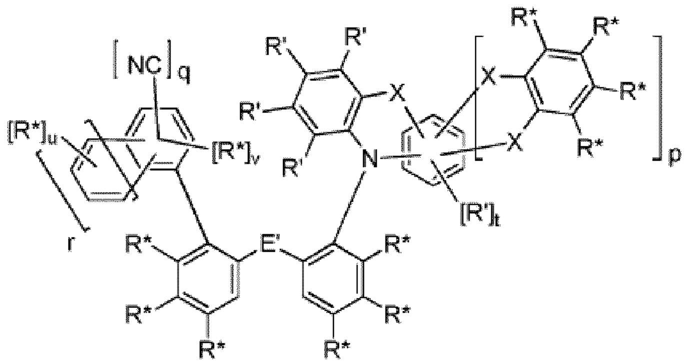

**Formule 6a**

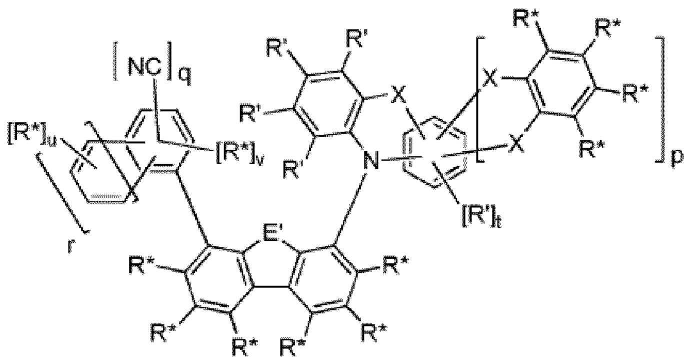

**Formule 6b**

où dans la formule 6a/6b

p est 0 ou 1 ;
t = 4-2p ;
X est $CR^*_2$, $NR^*$, un atome d'oxygène, un atome de soufre, une liaison directe, au maximum deux symboles X représentant simultanément une liaison directe, et ceux-ci ne faisant pas partie du même cycle ;
r = 0 ou 1,
q = 1 à 5, pour r = 1 q = 1 à 3,
u est 1, 2, 3 ou 4
v est 1, 2 ou 3 ; où il s'applique que :
u + v = 3 + 4r - q
E' est S, O, C(=O), $CR^*_2$, $Si(R^4)_2$, $Ge(R^4)_2$, $NR^2$, $BR^3$;
R' est R* ou est choisi parmi les unités suivantes, au maximum deux des radicaux R' représentant simultanément l'une des unités suivantes :

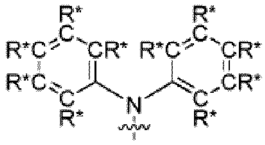, 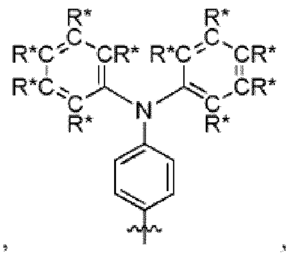, 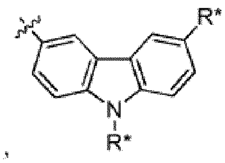, 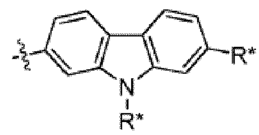,

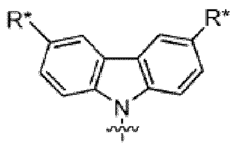, 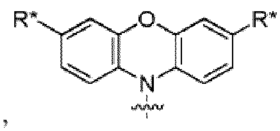, 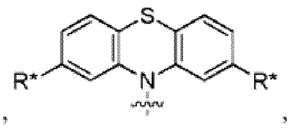,

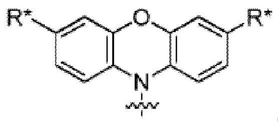, 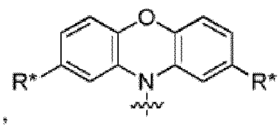;

et R* étant tel que défini dans la revendication 1.

6. Molécule organique selon l'une quelconque des revendications 1 à 3, où la molécule organique présente une structure de formule 7a ou 7b :

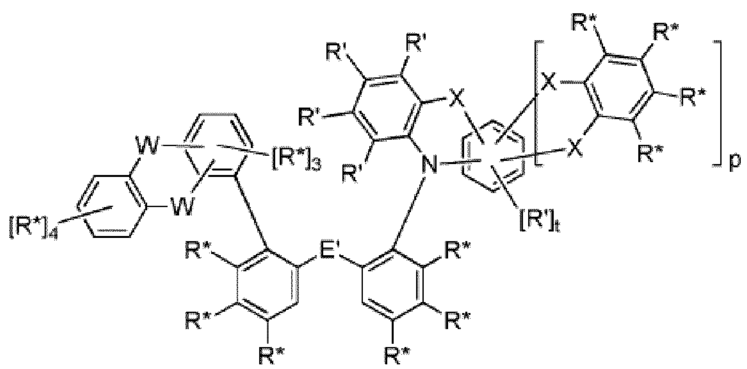

**Formule 7a**

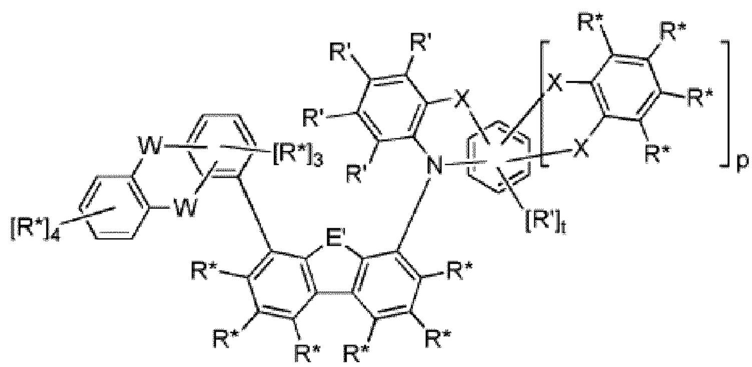

**Formule 7b**

où dans la formule 7a/7b

p est 0 ou 1 ;
t = 4-2p ;
X est $CR^*_2$, NR*, un atome d'oxygène, un atome de soufre, une liaison directe, au maximum deux symboles X représentant simultanément une liaison directe, et ceux-ci ne faisant pas partie du même cycle ;
W est choisi dans le groupe constitué par

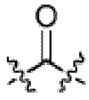, 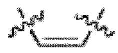, 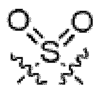,

une simple liaison élément-élément, deux unités W n'étant pas simultanément une simple liaison élément-élément, NR*, deux unités W ne représentant pas simultanément NR* ;
E' est choisi dans le groupe constitué par S, O, C(=O), $CR^*_2$, $Si(R^4)_2$, $Ge(R^4)_2$, $NR^2$ et $BR^3$ ;
R' est R* ou est choisi dans l'ensemble constitué par le groupe des unités suivantes, au maximum deux des radicaux R' représentant simultanément l'une des unités suivantes :

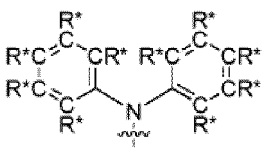, 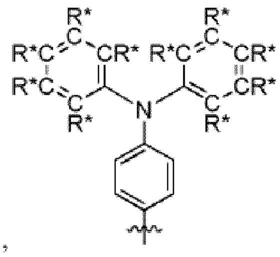, 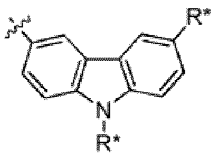, 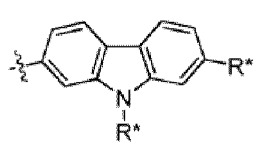,

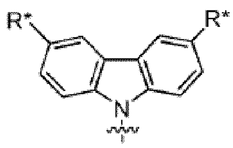, 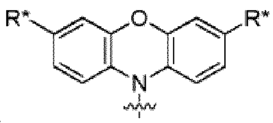, 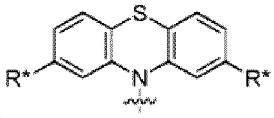,

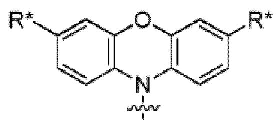, 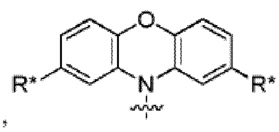;

et R* étant tel que défini dans la revendication 1.

**7.** Molécule organique selon une ou plusieurs des revendications 1 à 6, où la molécule organique comporte en au moins une position au moins un autre radical R, où
R est choisi indépendamment à chaque occurrence dans le groupe constitué par H, le deutérium, les groupes phényle, naphtyle, F, Cl, Br, I, $N(R^2)_2$, -CN, -NC, -SCN, $-CF_3$, - $NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, un groupe alkyle, alcoxy ou thioalcoxy linéaire ayant de 1 à 40 atomes de carbone ou un groupe alcényle ou alcynyle linéaire ayant de 2 à 40 atomes de carbone ou un groupe alkyle, alcényle, alcynyle, alcoxy ou thioalcoxy ramifié ou cyclique ayant de 3 à 40 atomes de carbone, qui peut dans chaque cas être substitué par un ou plusieurs radicaux $R^9$, un ou plusieurs groupes $CH_2$ contigus pouvant être remplacés par $-R^9C=CR^9-$, -C°C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O-, ou -S- et un ou plusieurs atomes d'hydrogène pouvant être remplacés par le deutérium, F, Cl, Br, I, CN, $CF_3$ ou $NO_2$, ou un système cyclique aromatique ou hétéroaromatique ayant de 5 à 60 atomes formant le cycle aromatique, qui peut dans chaque cas être substitué par un ou plusieurs radicaux $R^2$, ou un groupe aryloxy ou hétéroaryloxy ayant de 5 à 60 atomes formant le cycle aromatique, qui peut être substitué par un ou plusieurs radicaux $R^9$, ou un groupe diarylamino, un groupe dihétéroarylamino ou un groupe arylhétéroarylamino ayant de 10 à 40 atomes formant le cycle aromatique, qui peut être substitué par

un ou plusieurs radicaux $R^9$, ou une combinaison de ces systèmes ; deux ou plus de deux de ces substituants R peuvent en outre également former ensemble un système cyclique ayant au total cinq ou six chaînons formant le cycle ; $R^2$ à $R^9$ étant tels que définis dans la revendication 1 ;
et où au moyen de radicaux aptes à la polymérisation, qui portent des unités fonctionnelles aptes à la polymérisation, qui peuvent être homopolymérisés avec eux-mêmes ou copolymérisés avec d'autres monomères, on obtient la molécule organique sous forme de polymère à motifs répétitifs selon la formule 8 et/ou la formule 9

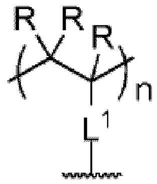

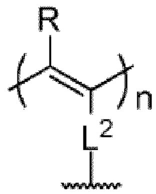

**Formule 8 Formule 9**

où

trait ondulé = position par laquelle le groupe de liaison $L^1$ ou $L^2$ est attaché à la molécule organique ;
$L^1$ et $L^2$ = des groupes de liaison identiques ou différents, comportant entre 0 et 20 atomes de carbone ; ou présentant une forme -C(C=O)O ;
et où en particulier $L^1$ et $L^2$ = -X-$L^3$ où X = O ou S ;
$L^3$ = un autre groupe de liaison choisi dans le groupe constitué par un groupe alkylène substitué et un groupe alkylène non substitué (linéaires, ramifiés ou cycliques) et un groupe arylène substitué et un groupe arylène non substitué, des associations étant également possibles.

**8.** Utilisation d'une molécule organique selon une ou plusieurs des revendications 1 à 7 en tant qu'émetteur luminescent et/ou en tant que matériau hôte et/ou en tant que matériau transporteur d'électrons et/ou en tant que matériau d'injection de trous et/ou en tant que matériau de blocage de trous dans un composant optoélectronique, le composant optoélectronique étant choisi en particulier dans le groupe constitué par :

- des diodes électroluminescentes organiques (OLED),
- des cellules électrochimiques photoémettrices,
- des capteurs OLED, en particulier dans des capteurs de gaz et de vapeur non isolés hermétiquement de l'extérieur,
- des diodes organiques,
- des cellules solaires organiques,
- des transistors organiques,
- des transistors à effet de champ organiques,
- des lasers organiques et
- des éléments d'abaissement de fréquence.

**9.** Utilisation selon la revendication 8, dans laquelle la concentration de la molécule organique en tant qu'émetteur dans des composants luminescents optiques, en particulier dans des OLED, est comprise entre 5 % et 80 %.

**10.** Composant optoélectronique, comportant une molécule organique selon une ou plusieurs des revendications 1 à 7, en particulier configuré sous forme d'un composant choisi dans le groupe constitué par des diodes électroluminescentes organiques (OLED), une cellule électrochimique photoémettrice, un capteur OLED, en particulier des capteurs de gaz et de vapeur non isolés hermétiquement de l'extérieur, des diodes organiques, une cellule solaire organique, un transistor organique, un transistor à effet de champ organique, un laser organique et un élément d'abaissement de fréquence.

**11.** Composant optoélectronique selon la revendication 10, comportant un substrat, une anode et une cathode, l'anode et la cathode étant appliquées sur le substrat, et au moins une couche photoémettrice qui est disposée entre l'anode et la cathode et qui contient une molécule organique selon une ou plusieurs des revendications 1 à 7.

**12.** Composant optoélectronique selon la revendication 10 ou 11, dans lequel la couche photoémettrice comporte au

moins un matériau hôte, où en particulier l'état singulet excité ($S_1$) et/ou l'état triplet excité ($T_1$) dudit au moins un matériau hôte est plus élevé que l'état singulet excité ($S_1$) et/ou que l'état triplet excité ($T_1$) de la molécule organique selon une ou plusieurs des revendications 1 à 7.

**13.** Composant optoélectronique selon l'une quelconque des revendications 10 à 12, dans lequel au moins un matériau hôte consiste en une molécule organique selon une ou plusieurs des revendications 1 à 7.

**14.** Composant optoélectronique selon l'une quelconque des revendications 10 à 13, dans lequel la couche photoémettrice comporte un matériau fluorescent ou phosphorescent et dans lequel le matériau de la couche photoémettrice est choisi en particulier parmi des molécules organiques selon une ou plusieurs des revendications 1 à 7.

**15.** Composant optoélectronique selon la revendication 14, dans lequel une molécule organique sous forme d'une molécule organique selon une ou plusieurs des revendications 1 à 7 et un matériau fonctionnel forment un exciplexe.

**16.** Composant optoélectronique selon l'une quelconque des revendications 10 à 15, dans lequel une molécule organique selon une ou plusieurs des revendications 1 à 7 est utilisée en tant que couche de transport de charge.

**17.** Procédé pour la production d'un composant optoélectronique, dans lequel on utilise une molécule organique selon une ou plusieurs des revendications 1 à 7, en effectuant la mise en oeuvre de la molécule organique en particulier par un procédé de dépôt sous vide en phase vapeur ou à partir d'une solution.

**18.** Utilisation d'un séparateur sous forme d'une unité chimique neutre pour la préparation d'une molécule organique selon une ou plusieurs des revendications 1 à 7, dans laquelle on relie par liaison covalente par l'intermédiaire du séparateur au moins une première unité chimique AF1, comportant un système conjugué, et au moins une deuxième unité chimique AF2, qui n'est pas identique à AF1, comportant un système conjugué.

Figur 1

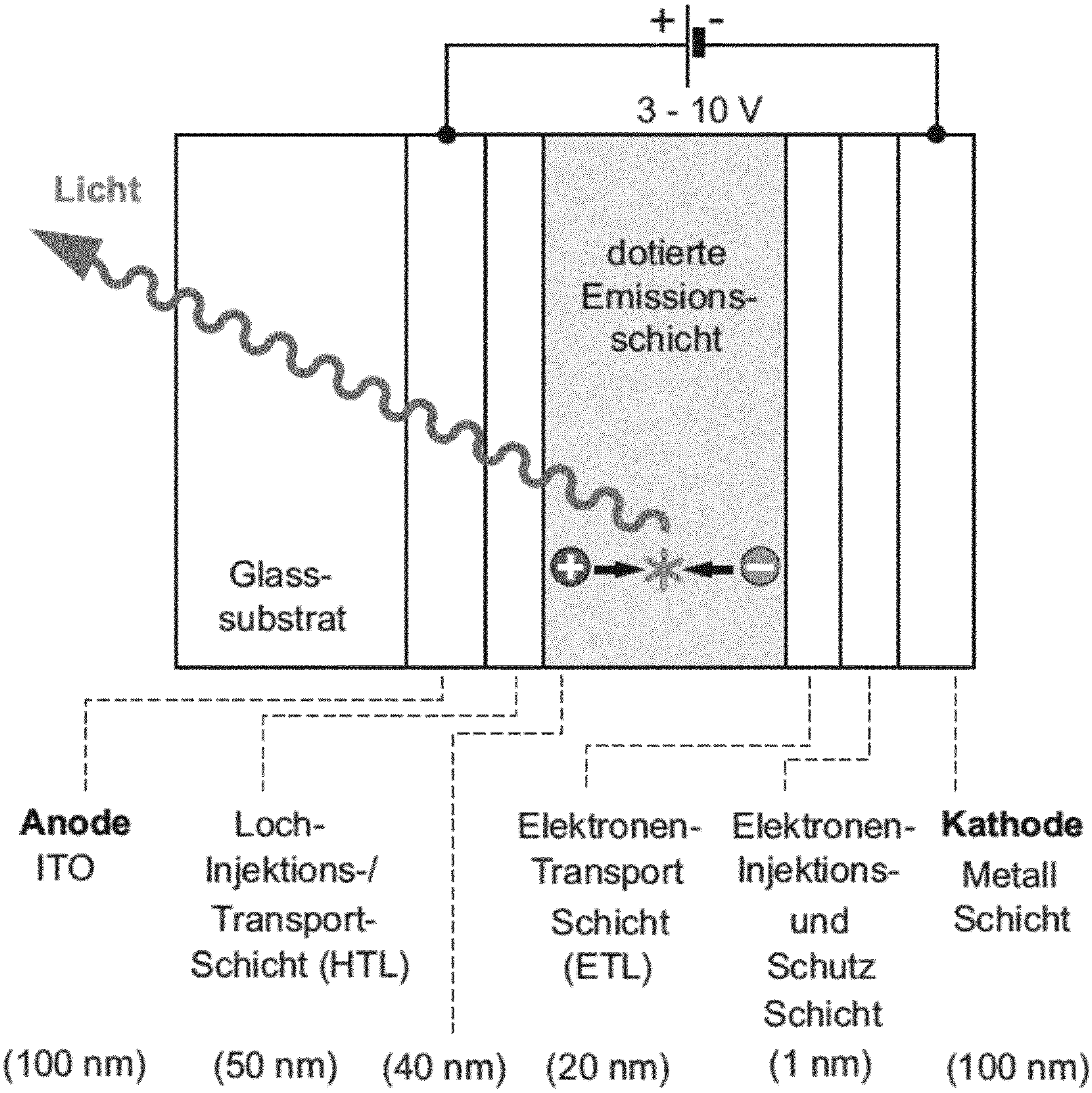

+
-
3 - 10 V
Licht
dotierte Emissions-schicht
Glass-substrat
Anode ITO (100 nm)
Loch-Injektions-/Transport-Schicht (HTL) (50 nm)
(40 nm)
Elektronen-Transport Schicht (ETL) (20 nm)
Elektronen-Injektions- und Schutz Schicht (1 nm)
Kathode Metall Schicht (100 nm)

Figur 2

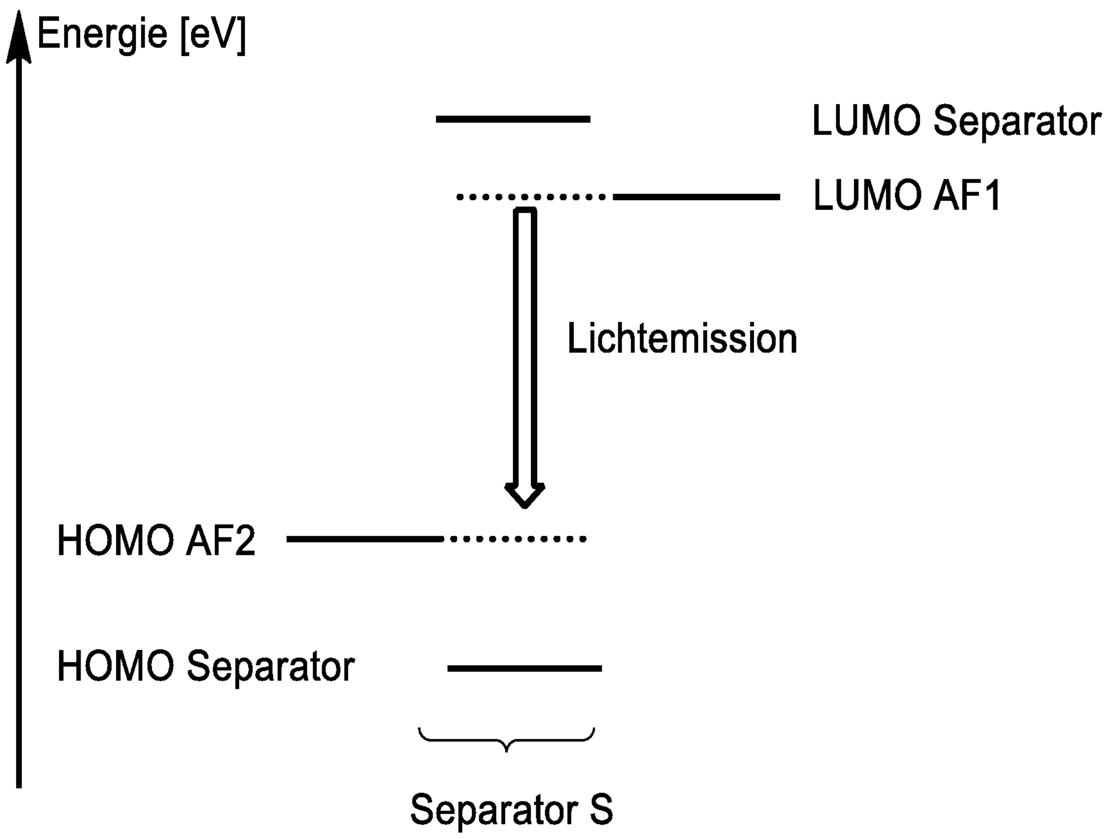
Energie [eV]
LUMO Separator
LUMO AF1
Lichtemission
HOMO AF2
HOMO Separator
Separator S

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2013161437 A
- US 2012075171 A1
- WO 002011057706 A2


### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **M. A. BALDO ; D. F. O'BRIEN ; M. E. THOMPSON ; S. R. FORREST.** *Phys. Rev. B,* 1999, vol. 60, 14422
- **BECKE, A. D.** *Phys. Rev. A,* 1988, vol. 38, 3098-3100
- **PERDEW.** *J. P. Phys. Rev. B,* 1986, vol. 33, 8822-8827
- **PERDEW, J. P.** *Phys. Rev. B,* 1986, vol. 33, 8822-8827
- **WANG.** *Adv. Mater.,* 2014, vol. 26, 5198-5204
- **SCHWARTZ.** *J. Am. Chem. Soc.,* 1992, vol. 114 (27), 10775-10784
- **PERDEW, J. P.** *Phys. Rev. B,* 1986, vol. 33, 8822-8827
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11
- **HÄSER, M. ; AHLRICHS, R.** *J. Comput. Chem.,* 1989, vol. 10, 104-111
- **WEIGEND, F. ; HÄSER, M.** *Theor. Chem. Acc.,* 1997, vol. 97, 331-340
- **SIERKA, M. ; HOGEKAMP, A. ; AHLRICHS, R.** *J. Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J.** *J.Phys.Chem.,* 1994, vol. 98, 11623-11627